# EUROPEAN PATENT APPLICATION

(11) **EP 4 706 737 A2**
(43) Date of publication of application: **11.03.2026**
(21) Application number: 26150387.4
(22) Date of filing: 03.09.2019
(51) Int. Cl.: A61M 39/10

(54) **BREAKAWAY INTRA-MEDICAL TUBING CONNECTOR ASSEMBLY**

(62) Divisional of application: 19944227.8
(71) Applicant: Elcam Medical A.C.A.L., 1386000 Merom Hagalil (IL)
(72) Inventor: MERMELSHTEIN, Gilad, 1215500 Upper Galilee (IL); SEGEV, Micha, 1529500 Lower Galilee (IL); MERMELSHTEIN, Gilad, 1386000 Merom Hagalil (IL); SADE, Dror, 2284900 West Galilee (IL); YESHAYAHU, Hillel, 1386000 Merom Hagalil (IL)
(74) Representative: Evens, Paul Jonathan

(57) **Abstract**

An automatically bidirectionally-sealable breakaway intra-medical tubing connector assembly comprising: a male luer actuated valve assembly (1102) and a female luer-actuated valve assembly (1100), which are configured for connection to opposite intermediate ends of a medical tube; and a connector body (1000) arranged for tensile force responsive disconnectable snap fit connection with at least one of said male luer actuated valve assembly (1102) and said female luer-actuated valve assembly (1100), which connection provides for opening of both said male luer actuated valve assembly (1102) and said female luer-actuated valve assembly (1100), said connector body (1000) is configured such that disconnection of at least one of said male luer actuated valve assembly (1102) and said female luer-actuated valve assembly (1100) from said connector body (1000) permits automatic closing of both said male luer actuated valve assembly (1102) and said female luer-actuated valve assembly (1100).

## Description

### REFERENCE TO RELATED APPLICATIONS

Reference is hereby made to US Provisional Patent application Serial No. 62/638,326, filed March 5, 2018 and entitled "BREAK AWAY CONNECTOR", and to US Provisional Patent application Serial No. 62/672,601, filed May 17, 2018 and entitled "Break-away Connector", the disclosures of which are hereby incorporated by reference. Reference is additionally made to PCT Patent application No. PCT/IL2019/050233, filed March 4, 2019 and entitled "BREAKAWAY INTRA-MEDICAL TUBING CONNECTOR ASSEMBLY", the disclosure of which is hereby incorporated by reference.

### FIELD OF THE INVENTION

The present invention relates to medical access devices and more particularly to break-away connectors for medical tubing.

### BACKGROUND OF THE INVENTION

Various medical tubes are used in treatment of patients, such as intravenous catheters, feeding tubes, dialysis tubes, enteral feeding, foley catheters. It is appreciated that these medical tubes are generally flexible, thus allowing the patient a range of movement.

For instance, medicaments may be administered to a patient through an IV line, which is connected to a needle, which is in turn fixed to an injection site. Additional example is a catheter which is inserted into a desired treatment location within the body of the patient.

During movement of the patient undergoing treatment, the medical tubes are subject to certain tensile forces that may cause dislocation of the catheter or of the needle. Additionally, certain tensile forces, greater than a pre-determined threshold, may cause spillage of the medicaments being administered to the patient or the patient's body fluids and thus expose the patient to a risk of infection.

### SUMMARY OF THE INVENTION

The present invention seeks to provide an improved break-away connector.

There is thus provided in accordance with an embodiment of the present invention an automatically bidirectionally-sealable breakaway intra-medical tubing connector assembly including first and second luer-actuated valve assemblies configured for connection to opposite intermediate ends of a medical tube; a connector body, having first and second ends and arranged for tensile force responsive disconnectable snap fit connection with at least one of the first and second luer-actuated valve assemblies, which connection provides opening of the first and second luer actuated valve assemblies; a connector element, having first and second ends and defining a fluid flow path therethrough, slidably located within the connector body and being configured to permit automatic closing of both the first and second luer actuated valves upon disconnection of at least one of the first and second luer actuated valve assemblies from the connector body.

Preferably, the automatically bidirectionally-sealable breakaway intra-medical tubing connector assembly also includes at least one snap-fit fitting adapted to be connected to the at least one of first and second luer-actuated valve assemblies and being arranged for tensile force responsive disconnectable snap fit connection with one of first or second ends of the connector body.

Further preferably, the automatically bidirectionally-sealable breakaway intra-medical tubing connector assembly also includes two snap-fit fittings, each being adapted to be connected to the at least one of first and second luer-actuated valve assemblies and being arranged for tensile force responsive disconnectable snap fit connection with one of first or second ends of the connector body.

Still further preferably, the connector element is configured to have first and second operative orientations with respect to the first and second luer-actuated valve assemblies: the first operative orientation in which the first and second ends of the connector element both openingly engage the respective first and second luer-actuated valve assemblies, the first operative orientation existing when both of the first and second luer-actuated valve assemblies are in snap fit connection with the connector body; the second operative orientation in which neither of the first and second ends of the connector element openingly engage the respective first and second luer-actuated valve assemblies, the second operative orientation existing when at least one of the first and second luer-actuated valve assemblies is disconnected from the connector body.

Alternatively, the connector element is configured to have first and second operative orientations with respect to the first and second luer-actuated valve assemblies: the first operative orientation in which the first and second ends of the connector element both openingly engage the respective first and second luer-actuated valve assemblies, the first operative orientation existing when both of the first and second snap-fit fittings are in snap fit connection with the connector body; the second operative orientation in which neither of the first and second ends of the connector element openingly engage the respective first and second luer-actuated valve assemblies, the second operative orientation existing when at least one of said first and second snap-fit fittings is disconnected from the connector body.

In accordance with an embodiment of the present invention, in the second operative orientation, both of the first and second luer-actuated valve assemblies are in a closed and sealed operative orientation.

Preferably, the connector element is a double-sided male connector, having first and second conical connector ends. Further preferably, the medical tube is selected from the group consisting of a catheter, an IV line and a drain. Still further preferably, a radially inwardly extending protrusion is formed on an inner surface of the connector body and a radially outwardly extending protrusion is formed on an outer surface of the connector element, and the connector element is freely slidable within the connector body up to engagement of the radially outwardly extending protrusion with the radially inwardly extending protrusion.

Yet further preferably, a first snap connection is formed on an outer surface of the connector body, adjacent the first end thereof and a second snap connection is formed on the outer surface of the connector body, adjacent the second end thereof.

Preferably, the snap-fit fitting is adapted to be connected to the first or second snap connection and arranged for tensile force responsive disconnection from the first or second snap connection. Further preferably, the connector element is axially slidable with respect to both the connector body and the at least one snap-fit fitting. Still further preferably, at least one of the first or second luer-actuated valve assembly is configured for threadable connection to the at least one snap-fit fitting. Yet further preferably, the inner diameter of a fluid flow passage provided between the first and second luer-actuated valve assemblies is substantially the same as the diameter of a thoroughgoing bore formed within the connector element.

In accordance with an embodiment of the present invention, each of the first and second luer-actuated valve assemblies includes a compressible sealing element having a first open end and a second selectably openable end, including a selectively openable slit and wherein the sealing element is biased to its normally closed position when no compression force is exerted thereon.

Preferably, upon engagement of the first and second luer-actuated valve assemblies with the breakaway intra-medical tubing connector assembly, the first end of the connector element compresses the sealing element of the first luer-actuated valve assembly and the second end of the connector element compresses the sealing element of the second luer-actuated valve assembly, thereby opening the respective selectively openable slits and establishing fluid flow passage between the first and second luer-actuated valve assemblies.

Further preferably, the at least one snap-fit fitting is freely rotatable about the connector body. Still further preferably, tensile force in the range of 0.5kgf - 3kgf is required in order to disconnect the luer-actuated valve assembly from the connector body. Alternatively, tensile force in the range of 0.5kgf - 3kgf is required in order to disconnect the at least one snap-fit fitting from the connector body.

In accordance with an embodiment of the present invention, the magnitude of the required tensile force to disconnect the first luer-actuated valve assembly from the connector body is greater than the tensile force required to disconnect the second luer-actuated valve assembly from the connector body. Alternatively, the magnitude of the required tensile force to disconnect the first snap-fit fitting from the connector body is greater than the tensile force required to disconnect the second snap-fit fitting from the connector body.

Preferably, in the second operative orientation the first and second luer-actuated valve assemblies are swabbable. Further preferably, in the second operative orientation the first and second luer-actuated valve assemblies are swabbable once the first and second snap-fit fittings are detached therefrom.

Yet further preferably, the medical tube remains static upon disconnection of at least one of the first and second luer actuated valve assemblies from the connector body. Alternatively, the medical tube remains static upon disconnection of at least one of the snap-fit fittings from the connector body.

In accordance with an embodiment of the present invention, at least one of the first and second luer-actuated valve assemblies is integrally formed with or fixedly connected to the connector body.

Preferably, each of the first and second ends of the connector body includes a plurality of radially arranged spaced apart arms and a plurality of cut-outs formed therebetween, wherein at least one of the plurality of arms includes a snap portion extending radially inwardly and configured for engagement with at least one of the first and second luer-actuated valve assemblies. Further preferably, the connector body is composed of two parts attachable to each other.

In accordance with another embodiment of the present invention, an automatically bidirectionally-sealable breakaway intra-medical tubing connector assembly configured for use with first and second luer-actuated valves connected to opposite intermediate ends of a medical tube, the connector assembly including a connector body, having first and second ends and arranged for tensile force responsive disconnectable snap fit connection with at least one of the first and second luer actuated valve assemblies, which connection provides opening of the first and second luer actuated valve assemblies; and a connector element, having first and second ends and defining a fluid flow path therethrough, slidably located within the connector body and being configured to permit automatic closing of both the first and second luer actuated valve assemblies upon disconnection of at least one of the first and second luer actuated valve assemblies from the connector body.

In accordance with still another embodiment of the present invention, an automatically bidirectionally-sealable breakaway intra-medical tubing connector assembly includes a male luer actuated valve assembly and a female luer-actuated valve assembly, which are configured for connection to opposite intermediate ends of a medical tube; a connector body arranged for tensile force responsive disconnectable snap fit connection with at least one of the male luer actuated valve assembly and the female luer-actuated valve assembly, which connection provides for opening of both the male luer actuated valve assembly and the female luer-actuated valve assembly, the connector body is configured such that disconnection of at least one of the male luer actuated valve assembly and the female luer-actuated valve assembly from the connector body permits automatic closing of both the male luer actuated valve assembly and the female luer-actuated valve assembly.

Preferably, the connection provides a fluid flow path between the male luer actuated valve assembly and the female luer-actuated valve assembly. Further preferably, the male luer-actuated valve assembly and the female luer-actuated valve assembly assume a closed and sealed operative orientation upon disconnection thereof from the connector body. Still further preferably, the medical tube is selected from the group consisting of a catheter, an IV line and a drain. Yet further preferably, a first snap connection is formed on an inner surface of the connector body, adjacent the first end thereof and a second snap connection is formed on the inner surface of the connector body, adjacent the second end thereof, the first and second snap portions are configured for attachment with the male luer actuated valve assembly and the female luer-actuated valve assembly respectively.

In accordance with an embodiment of the present invention, each of the male luer actuated valve assembly and the female luer-actuated valve assembly includes a compressible sealing element having a first open end and a second selectably openable end, including a selectively openable slit and wherein the sealing element is biased to its normally closed position when no compression force is exerted thereon.

Preferably, upon engagement of the male luer actuated valve assembly and the female luer-actuated valve assembly with the connector body, the female luer-actuated valve assembly actuates the male luer-actuated valve assembly , both sealing elements of the male luer-actuated valve assembly and of the female luer-actuated valve assembly are compressed, thereby opening the respective selectively openable slits and establishing fluid flow passage between the male luer-actuated valve assembly and the female luer-actuated valve assembly.

Further preferably, the tensile force in the range of 0.5kgf - 3kgf is required in order to disconnect one of the male luer actuated valve assembly and the female luer-actuated valve assembly from the connector body. Still further preferably, the magnitude of the required tensile force to disconnect one of the male luer-actuated valve assembly and the female luer-actuated valve assembly from the connector body is greater than the tensile force required to disconnect the other of the male luer-actuated valve assembly and the female luer-actuated valve assembly from the connector body. Yet further preferably, upon disconnection of the male luer-actuated valve assembly and the female luer-actuated valve assembly from the connector body, both the male luer-actuated valve assembly and the female luer-actuated valve assembly are swabbable. Further preferably, the medical tube remains static upon disconnection of the male luer-actuated valve assembly and the female luer-actuated valve assembly from the connector body.

In accordance with an embodiment of the present invention, an automatically bidirectionally-sealable breakaway intra-medical tubing connector body configured for use with a male luer-actuated valve assembly and a female luer-actuated valve assembly connected to opposite intermediate ends of a medical tube, including: the connector body arranged for tensile force responsive disconnectable snap fit connection with at least one of the male luer actuated valve assembly and the female luer-actuated valve assembly, which connection provides for opening of both the male luer actuated valve assembly and the female luer-actuated valve assembly, the connector body is configured such that disconnection of at least one of the male luer actuated valve assembly and the female luer-actuated valve assembly from the connector body permits automatic closing of both the male luer actuated valve assembly and the female luer-actuated valve assembly.

In accordance with an embodiment of the present invention, an automatically bidirectionally-sealable breakaway intra-medical tubing connector assembly configured for use with first and second luer-actuated valve assemblies connected to opposite intermediate ends of a medical tube, the connector assembly comprising: a first snap-fit fitting adapted to be connected to the first luer-actuated valve assembly and a second snap-fit fitting adapted to be connected to the second luer-actuated valve assembly, the first and second snap-fit fittings being arranged for tensile force responsive disconnectable snap fit connection with each other; and a connector element, having first and second ends and defining a fluid flow path therethrough, the connector element being at least partially located within the first and second snap-fit fittings, the first and second snap-fit fittings and the connector element being configured upon connection of the first and second luer-actuated valve assemblies thereto for opening of the first and second luer-actuated valve assemblies and upon disconnection of the first and second snap-fit fittings from each other for automatic closing of both the first and second luer actuated valve assemblies.

Preferably, the connector element is configured to have first and second operative orientations with respect to the first and second luer-actuated valve assemblies: the first operative orientation in which the first and second ends of the connector element both openingly engage the respective first and second luer-actuated valve assemblies, the first operative orientation existing when both of the snap-fit fittings are in snap fit connection with each other; the second operative orientation in which neither of the first and second ends of the connector element openingly engage the respective first and second luer-actuated valve assemblies, the second operative orientation existing when the snap-fit fittings are disconnected from each other and the first and second ends of the connector element are axially pulled out of both first and second luer-actuated valve assemblies.

Further preferably, in the second operative orientation, both of the first and second luer-actuated valve assemblies are in a closed and sealed operative orientation. Still further preferably, the connector element is a double-sided male connector, having first and second conical connector ends. Yet further preferably, the medical tube is selected from the group consisting of a catheter, an IV line and a drain.

In accordance with an embodiment of the present invention, a radially inwardly extending protrusion is formed on an inner surface of each of the first snap-fit fitting and the second snap-fit fitting and at least one radially outwardly extending protrusion is formed on an outer surface of the connector element, and the connector element is slidable with respect to at least one of the first and second snap-fit fittings up to engagement of the at least one radially outwardly extending protrusion with one of the radially inwardly extending protrusions.

Preferably, a first snap connection is formed on the first snap-fit fitting and a second snap connection is formed on the second snap-fit fitting. Further preferably, the first snap connection is adapted to be connected with the second snap connection and arranged for tensile force responsive disconnection from each other. Still further preferably, a first radially inwardly extending protrusion is formed on an inner surface of the first snap-fit fitting and a second radially inwardly extending protrusion is formed on an inner surface of the second snap-fit fitting and a first radially outwardly extending protrusion is formed on an outer surface of the first end of the connector element and a second radially outwardly extending protrusion is formed on an outer surface of the second end of the connector element, and wherein during disconnection of the breakaway intra-medical tubing connector assembly, the connector element is slidable with respect to the first snap-fit fitting up to engagement of the first radially outwardly extending protrusion with the first radially inwardly extending protrusion and the connector element is slidable with respect to the second snap-fit fitting up to engagement of the second radially outwardly extending protrusion with the second radially inwardly extending protrusion.

In accordance with an embodiment of the present invention, the connector element is longitudinally fixed with respect to both snap-fit fittings. Preferably, the connector element has a radially outwardly extending intermediate protrusion formed on the outer surface of the connector element, the intermediate protrusion is formed between the first radially outwardly extending protrusion and the second radially outwardly extending protrusion. Further preferably, the first luer-actuated valve assembly is configured for threadable connection to the first snap-fit fitting and the second luer-actuated valve assembly is configured for threadable connection to the second snap-fit fitting. Still further preferably, the inner diameter of a fluid flow passage provided between the first and second luer-actuated valve assemblies is substantially the same as the diameter of a thoroughgoing bore formed within the connector element. Yet further preferably, each of the first and second luer-actuated valve assemblies includes a compressible sealing element seated in a female luer portion of the first and second luer-actuated valve assemblies and configured to prevent fluid flow through the first and second luer-actuated valve assemblies when the sealing element is disposed in a closed operative orientation.

Preferably, the sealing element having a first open end and a second selectably openable end, including a selectively openable slit and wherein the sealing element is biased to its normally closed position when no compression force is exerted thereon. Further preferably, the sealing element is configured to permit flow around it when the sealing element is disposed in an open operative orientation. Alternatively, the sealing element is configured to permit flow therethrough when the sealing element is disposed in an open operative orientation.

In accordance with an embodiment of the present invention, upon engagement of the first and second luer-actuated valve assemblies with the breakaway intra-medical tubing connector assembly, the first end of the connector element compresses the sealing element of the first luer-actuated valve assembly and the second end of the connector element compresses the sealing element of the second luer-actuated valve assembly, thereby positioning both the first and the second luer-actuated valve assemblies in the open operative orientation.

Preferably, in the open operative orientation, the selectively openable slits of the first and second luer-actuated valve assemblies are open, thereby providing for establishing fluid flow passage between the first and second luer-actuated valve assemblies. Further preferably, tensile force in the range of 0.2 - 5kgf is required in order to disconnect the first and second snap-fit fittings from each other. Still further preferably, in the second operative orientation, the first and second luer-actuated valve assemblies are swabbable once their respective first and second snap-fit fittings are detached therefrom. Yet further preferably, the medical tube remains substantially fixed in its initial position upon disconnection of the first and second snap-fit fitting from each other.

In accordance with an embodiment of the present invention, the first and second snap-fit fittings being locked to each other during connection of the luer actuated valve assemblies to the breakaway intra-medical tubing connector assembly. Preferably, the first and second snap-fit fittings are locked to each other by means of a bayonet mechanism. Further preferably, the first radially inwardly extending protrusion extends radially inwardly to a different extent than the second radially inwardly extending protrusion. Still further preferably, the first radially inwardly extending protrusion radially overlaps with the first radially outwardly extending protrusion and the second radially inwardly extending protrusion radially overlaps with the second radially outwardly extending protrusion, thereby restricting axial displacement of the connector element relative to at least one of the first and second snap-fit fittings upon engagement of at least one of the first radially outwardly extending protrusion with the first radially inwardly extending protrusion and the second radially outwardly extending protrusion with the second radially inwardly extending protrusion until a pre-determined tensile force threshold is applied on one of the first and second snap-fit fittings. Yet further preferably, the first and second snap-fit fittings being unlocked with respect to each other by means of relative rotation with respect to each other.

### BRIEF DESCRIPTION OF THE DRAWINGS

The present invention will be understood and appreciated more fully from the following detailed description, taken in conjunction with the drawings in which:
Fig. 1 is a simplified pictorial illustration of an assembled breakaway intra-medical tubing connector assembly, constructed and operative in accordance with an embodiment of the present invention;
Figs. 2A and 2B are respectively a simplified exploded illustration and a sectional exploded illustration of the breakaway intra-medical tubing connector assembly of Fig. 1, section being taken along lines B - B in Fig. 2A;
Figs. 3A - 3D are simplified respective perspective, side view, top view and a sectional illustration taken along lines D - D in Fig. 3B of a connector body, forming part of the breakaway intra-medical tubing connector assembly of Figs. 1 - 2B;
Figs. 4A - 4D are simplified respective perspective, side view, top view and a sectional illustration taken along lines D - D in Fig. 4B of a connector element, forming part of the breakaway intra-medical tubing connector assembly of Figs. 1 - 2B;
Figs. 5A - 5E are simplified respective perspective, side view, top view, bottom view and a sectional illustration taken along lines E - E in Fig. 5B of a snap-fit fitting, forming part of the breakaway intra-medical tubing connector assembly of Figs. 1 - 2B;
Figs. 6A - 6C are simplified respective side view, top view and a sectional illustration taken along lines C - C in Fig. 6A of the assembled breakaway intra-medical tubing connector assembly of Figs. 1 - 2B;
Figs. 7A - 7C are simplified respective perspective view, side view and a sectional illustration taken along lines C - C in Fig. 7B of the breakaway intra-medical tubing connector assembly of Figs. 6A - 6C, shown in a first operative orientation, just prior to connection thereof with a first and a second luer-actuated valve assemblies constructed and operative in accordance with an embodiment of the present invention;
Figs. 8A - 8C are simplified respective perspective view, side view and a sectional illustration taken along lines C - C in Fig. 8B of the breakaway intra-medical tubing connector assembly of Figs. 6A - 6C, shown in a second operative orientation, when the first and the second luer-actuated valve assemblies of Figs. 7A - 7C are connected to the breakaway intra-medical tubing connector assembly;
Figs. 9A - 9C are simplified respective perspective view, side view and a sectional illustration taken along lines C - C in Fig. 9B of the breakaway intra-medical tubing connector assembly of Figs. 6A - 6C, shown in a third operative orientation, when one of the luer-actuated valve assemblies of Figs. 7A - 7C is disconnected from the breakaway intra-medical tubing connector assembly;
Figs. 10A - 10C are simplified respective perspective view, side view and a sectional illustration taken along lines C - C in Fig. 10B of the breakaway intra-medical tubing connector assembly of Figs. 6A - 6C, shown in a first operative orientation, just prior to connection thereof with a first and a second luer-actuated valve assemblies constructed and operative in accordance with another embodiment of the present invention;
Figs. 11A - 11C are simplified respective perspective view, side view and a sectional illustration taken along lines C - C in Fig. 11B of the breakaway intra-medical tubing connector assembly of Figs. 6A - 6C, shown in a second operative orientation, when the first and the second luer-actuated valve assemblies of Figs. 10A - 10C are connected to the breakaway intra-medical tubing connector assembly;
Figs. 12A - 12C are simplified respective perspective view, side view and a sectional illustration taken along lines C - C in Fig. 12B of the breakaway intra-medical tubing connector assembly of Figs. 6A - 6C, shown in a third operative orientation, when one of the luer-actuated valve assemblies of Figs. 10A - 10C is disconnected from the breakaway intra-medical tubing connector assembly;
Fig. 13 is a simplified pictorial illustration of an assembled breakaway intra-medical tubing connector assembly, constructed and operative in accordance with another embodiment of the present invention;
Figs. 14A and 14B are respectively a simplified exploded illustration and a sectional exploded illustration of the breakaway intra-medical tubing connector assembly of Fig. 13, section being taken along lines B - B in Fig. 14A;
Figs. 15A - 15D are simplified respective side view, top view, bottom view and a sectional illustration taken along lines D - D in Fig. 15A of a male luer portion of a first luer-actuated valve assembly, forming part of the breakaway intra-medical tubing connector assembly of Figs. 13 - 14B;
Figs. 16A - 16C are simplified respective side view, top view and a sectional illustration taken along lines C - C in Fig. 16A of a cover element of the first luer-actuated valve assembly, forming part of the breakaway intra-medical tubing connector assembly of Figs. 13 -14B;
Figs. 17A - 17C are simplified respective side view, top view and a sectional illustration taken along lines C - C in Fig. 17A of a connector body, forming part of the breakaway intra-medical tubing connector assembly of Figs. 13 - 14B;
Figs. 18A - 18C are simplified respective side view, top view and a sectional illustration taken along lines C - C in Fig. 18A of the assembled breakaway intra-medical tubing connector assembly of Figs. 13 - 14B;
Figs. 19A - 19C are simplified respective perspective view, side view and a sectional illustration taken along lines C - C in Fig. 19B of the breakaway intra-medical tubing connector assembly of Figs. 18A - 18C, shown when a second luer-actuated valve assembly is connected to the breakaway intra-medical tubing connector assembly;
Figs. 20A - 20C are simplified respective perspective view, side view and a sectional illustration taken along lines C - C in Fig. 20B of the breakaway intra-medical tubing connector assembly of Figs. 18A - 18C, shown when the second luer-actuated valve assembly is disconnected from the breakaway intra-medical tubing connector assembly;
Fig. 21 is a simplified pictorial illustration of an assembled breakaway intra-medical tubing connector assembly, constructed and operative in accordance with still another embodiment of the present invention;
Figs. 22A and 22B are respectively a simplified exploded illustration and a sectional exploded illustration of the breakaway intra-medical tubing connector assembly of Fig. 21, section being taken along lines B - B in Fig. 22A;
Figs. 23A - 23D are simplified respective perspective, side view, top view and a sectional illustration taken along lines D - D in Fig. 23C of a connector body, forming part of the breakaway intra-medical tubing connector assembly of Figs. 21 - 22B;
Figs. 24A - 24D are simplified respective perspective, side view, top view and a sectional illustration taken along lines D - D in Fig. 24C of a snap-fir fitting, forming part of the breakaway intra-medical tubing connector assembly of Figs. 21 - 22B;
Figs. 25A and 25B are simplified respective side view and a sectional illustration taken along lines B - B in Fig. 25A of the assembled breakaway intra-medical tubing connector assembly of Figs. 21 - 22B;
Figs. 26A - 26C are simplified respective perspective view, side view and a sectional illustration taken along lines C - C in Fig. 26B of the breakaway intra-medical tubing connector assembly of Figs. 25A and 25B, shown in a first operative orientation, just prior to connection thereof with a first and a second luer-actuated valve assemblies constructed and operative in accordance with an embodiment of the present invention;
Figs. 27A - 27C are simplified respective perspective view, side view and a sectional illustration taken along lines C - C in Fig. 27B of the breakaway intra-medical tubing connector assembly of Figs. 25A and 25B, shown in a second operative orientation, when the first and the second luer-actuated valve assemblies of Figs. 26A - 26C are connected to the breakaway intra-medical tubing connector assembly;
Figs. 28A - 28C are simplified respective perspective view, side view and a sectional illustration taken along lines C - C in Fig. 28B of the breakaway intra-medical tubing connector assembly of Figs. 25A and 25B, shown in a third operative orientation, when one of the luer-actuated valve assemblies of Figs. 26A - 26C is disconnected from the breakaway intra-medical tubing connector assembly;
Figs. 29A - 29C simplified respective perspective view, side view, and top view of a connector body constructed and operative in accordance with yet another embodiment of the present invention;
Fig. 29D and 29E are simplified orthogonal sectional illustrations of the connector body of Figs. 29A - 29C, sections being taken along lines D - D and E - E in Fig. 29C;
Figs. 30A and 30B are simplified orthogonal side views of the connector body of Figs. 29A - 29E, shown in a first operative orientation, just prior to connection thereof with a first and a second luer-actuated valve assemblies constructed and operative in accordance with an embodiment of the present invention;
Figs. 31A and 31B are respective simplified orthogonal sectional views of Figs. 30A and 30B, sections taken along lines A - A in Fig. 30A and lines B - B in Fig. 30B;
Figs. 32A and 32B are simplified orthogonal side views of the connector body of Figs. 29A - 29E, shown in a second operative orientation, when the first and the second luer-actuated valve assemblies of Figs. 30A and 30B are connected to the connector body of Figs. 29A - 29E;
Figs. 33A and 33B are respective simplified orthogonal sectional views of Figs. 31A and 31B, sections taken along lines A - A in Fig. 31A and lines B - B in Fig. 31B;
Figs. 34A and 34B are simplified orthogonal side views of the connector body of Figs. 29A - 29E, shown in a third operative orientation, when one of the luer-actuated valve assemblies of Figs. 30A and 30B is disconnected from the connector body of Figs. 29A - 29E;
Figs. 35A and 35B are respective simplified orthogonal sectional views of Figs. 34A and 34B, sections taken along lines A - A in Fig. 34A and lines B - B in Fig. 34B;
Fig. 36 is a simplified pictorial illustration of an assembled breakaway intra-medical tubing connector assembly, constructed and operative in accordance with still another embodiment of the present invention;
Figs. 37A and 37B are respectively a simplified exploded illustration and a sectional exploded illustration of the breakaway intra-medical tubing connector assembly of Fig. 36, section being taken along lines B - B in Fig. 37A;
Figs. 38A - 38C are simplified respective side view, top view and a sectional illustration taken along lines C - C in Fig. 38A of a connector body, forming part of the breakaway intra-medical tubing connector assembly of Figs. 36 - 37B;
Figs. 39A - 39C are simplified respective side view, top view and a sectional illustration taken along lines C - C in Fig. 39A of a connector element, forming part of the breakaway intra-medical tubing connector assembly of Figs. 36 - 37B;
Figs. 40A and 40B are simplified respective side view and a sectional illustration taken along lines B - B in Fig. 40A of the assembled breakaway intra-medical tubing connector assembly of Figs. 36 - 37B;
Figs. 41A - 41C are simplified respective perspective view, side view and a sectional illustration taken along lines C - C in Fig. 41B of the breakaway intra-medical tubing connector assembly of Figs. 40A and 40B, shown in a first operative orientation, just prior to connection thereof with a first and a second luer-actuated valve assemblies constructed and operative in accordance with an embodiment of the present invention;
Figs. 42A - 42C are simplified respective perspective view, side view and a sectional illustration taken along lines C - C in Fig. 42B of the breakaway intra-medical tubing connector assembly of Figs. 40A and 40B, shown in a second operative orientation, when the first and the second luer-actuated valve assemblies of Figs. 41A - 41C are connected to the breakaway intra-medical tubing connector assembly;
Figs. 43A - 43C are simplified respective perspective view, side view and a sectional illustration taken along lines C - C in Fig. 43B of the breakaway intra-medical tubing connector assembly of Figs. 40A and 40B, shown in a third operative orientation, which is a transition operative orientation when one of the luer-actuated valve assemblies of Figs. 41A -41C is in the process of disconnection from the breakaway intra-medical tubing connector assembly;
Figs. 44A - 44C are simplified respective perspective view, side view and a sectional illustration taken along lines C - C in Fig. 44B of the breakaway intra-medical tubing connector assembly of Figs. 40A and 40B, shown in a fourth operative orientation, when one of the luer-actuated valve assemblies of Figs. 41A - 41C is disconnected from the breakaway intra-medical tubing connector assembly;
Fig. 45 is a simplified pictorial illustration of an assembled breakaway intra-medical tubing connector assembly, constructed and operative in accordance with yet another embodiment of the present invention;
Figs. 46A and 46B are respectively a simplified exploded illustration and a sectional exploded illustration of the breakaway intra-medical tubing connector assembly of Fig. 45, section being taken along lines B - B in Fig. 46A;
Figs. 47A - 47E are simplified respective perspective view, side view, top view, bottom view and a sectional illustration taken along lines E - E in Fig. 47B of a connector body portion, forming part of the breakaway intra-medical tubing connector assembly of Figs. 45 -46B;
Figs. 48A - 48D are simplified respective perspective view, side view, top view and a sectional illustration taken along lines D - D in Fig. 48B of a connector element, forming part of the breakaway intra-medical tubing connector assembly of Figs. 45 - 46B;
Figs. 49A and 49B are simplified respective side view and a sectional illustration taken along lines B - B in Fig. 49A of the assembled breakaway intra-medical tubing connector assembly of Figs. 45 - 46B;
Fig. 50 is a simplified pictorial illustration of an assembled breakaway intra-medical tubing connector assembly, constructed and operative in accordance with still another embodiment of the present invention;
Figs. 51A and 51B are respectively a simplified exploded illustration and a sectional exploded illustration of the breakaway intra-medical tubing connector assembly of Fig. 50, section being taken along lines B - B in Fig. 51A;
Figs. 52A - 52D are simplified respective perspective, side view, top view and a sectional illustration taken along lines D - D in Fig. 52B of a first snap-fit fitting, forming part of the breakaway intra-medical tubing connector assembly of Figs. 50 - 51B;
Figs. 53A - 53D are simplified respective perspective, side view, top view and a sectional illustration taken along lines D - D in Fig. 4B of a connector element, forming part of the breakaway intra-medical tubing connector assembly of Figs. 50 - 51B;
Figs. 54A - 54D are simplified respective perspective, side view, top view and a sectional illustration taken along lines D - D in Fig. 54B of a second snap-fit fitting, forming part of the breakaway intra-medical tubing connector assembly of Figs. 50 - 51B;
Figs. 55A - 55C are simplified respective side view, top view and a sectional illustration taken along lines C - C in Fig. 55A of the assembled breakaway intra-medical tubing connector assembly of Figs. 50 - 52B, shown in a locked operative position;
Figs. 56A - 56C are simplified respective perspective view, side view and a sectional illustration taken along lines C - C in Fig. 56B of the breakaway intra-medical tubing connector assembly of Figs. 55A - 55C, shown in a first operative orientation, just prior to connection thereof with a first and a second luer-actuated valve assemblies constructed and operative in accordance with an embodiment of the present invention, the breakaway intra-medical tubing connector assembly of Figs. 50 - 52B is shown in a locked operative position;
Figs. 57A - 57C are simplified respective perspective view, side view and a sectional illustration taken along lines C - C in Fig. 57B of the breakaway intra-medical tubing connector assembly of Figs. 55A - 55C, shown in a second operative orientation, when the first and the second luer-actuated valve assemblies of Figs. 56A - 56C are connected to the breakaway intra-medical tubing connector assembly, which is shown in a locked operative position;
Figs. 58A and 58B are simplified respective perspective view and side view of the breakaway intra-medical tubing connector assembly of Figs. 55A - 55C, shown in a third operative orientation, when the first and the second luer-actuated valve assemblies of Figs. 56A - 56C are connected to the breakaway intra-medical tubing connector assembly, which is shown in an unlocked operative position;
Figs. 59A and 59B are simplified respective perspective view and side view of the breakaway intra-medical tubing connector assembly of Figs. 55A - 55C, shown in a fourth operative orientation, when the first and the second luer-actuated valve assemblies of Figs. 56A - 56C are connected to the breakaway intra-medical tubing connector assembly, which is shown in an intermediate stage of disconnection;
Figs. 59C and 59D are simplified sectional illustrations of the breakaway intra-medical tubing connector assembly of Figs. 59A and 59B, sections taken along respective lines C - C and D - D in Fig. 59B, when the breakaway intra-medical tubing connector assembly is shown in a first intermediate stage of disconnection;
Figs. 59E and 59F are simplified sectional illustrations of the breakaway intra-medical tubing connector assembly of Figs. 59A and 59B, sections taken along respective lines E - E and F - F in Fig. 59B, when the breakaway intra-medical tubing connector assembly is shown in a second intermediate stage of disconnection;
Figs. 60A and 60B are simplified respective perspective view and side view of the breakaway intra-medical tubing connector assembly of Figs. 55A - 55C, shown in a fifth operative orientation, when the first and the second luer-actuated valve assemblies of Figs. 56A - 56C are connected to the breakaway intra-medical tubing connector assembly, which is shown in an intermediate stage just prior to disconnection;
Fig. 60C is a simplified sectional illustration of the breakaway intra-medical tubing connector assembly of Figs. 60A and 60B, section taken along lines C - C in Fig. 60B, when the breakaway intra-medical tubing connector assembly is shown in an operative orientation just prior to disconnection;
Figs. 61A - 61C are simplified respective perspective view, side view and a sectional illustration taken along lines C - C in Fig. 61B of the breakaway intra-medical tubing connector assembly of Figs. 55A - 55C, shown in a sixth operative orientation, when the breakaway intra-medical tubing connector assembly is shown in a disconnected orientation.

### DESCRIPTION OF EMBODIMENTS OF THE INVENTION

An automatically bidirectionally-sealable breakaway intra-medical tubing connector assembly is provided in accordance with an embodiment of the present invention. The breakaway intra-medical tubing connector assembly is preferably configured for use with a first and a second luer-actuated valves connected to opposite intermediate ends of a medical tube, the connector assembly preferably includes a connector body arranged for tensile force responsive disconnectable snap fit connection with at least one of the first and second luer actuated valves, which connection provides opening of the first and second luer actuated valve assemblies. The connector assembly further preferably includes a connector element, having first and second ends and defining a fluid flow path therethrough, slidably located within the connector body and being configured to permit automatic closing of both the first and second luer actuated valves upon disconnection of at least one of the first and second luer actuated valves from the connector body.

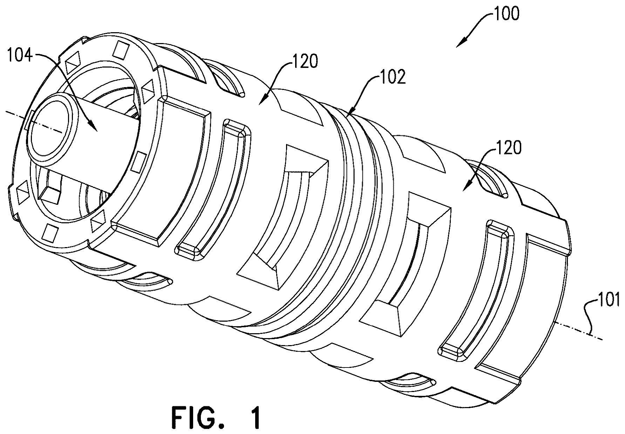

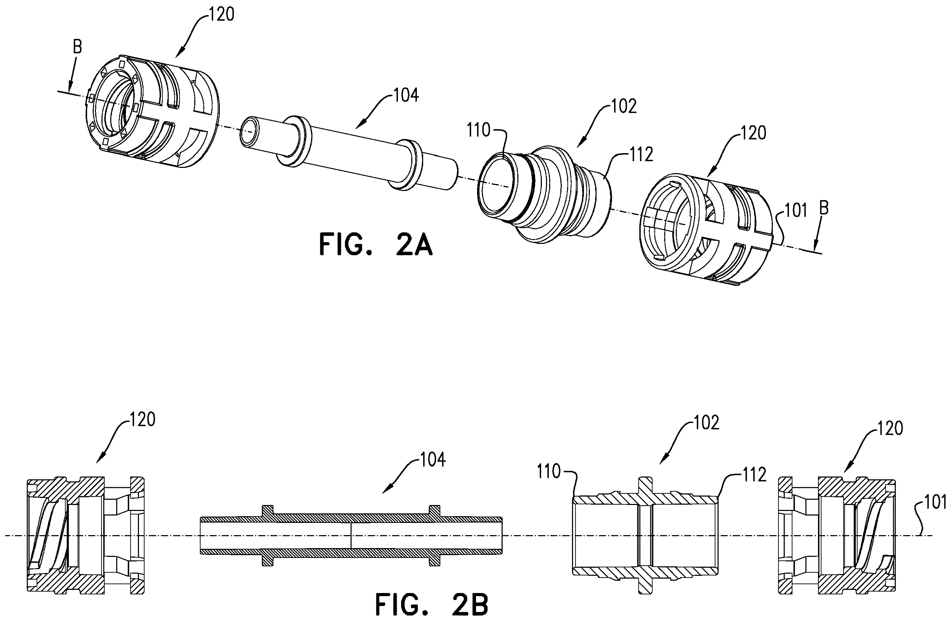

Reference is now made to Fig. 1, which is a simplified pictorial illustration of an assembled breakaway intra-medical tubing connector assembly, constructed and operative in accordance with an embodiment of the present invention. Reference is additionally made to Figs. 2A and 2B, which are respectively a simplified exploded illustration and a sectional exploded illustration of the breakaway intra-medical tubing connector assembly of Fig. 1, section being taken along lines B - B in Fig. 2A.

A breakaway intra-medical tubing connector assembly 100 arranged along a longitudinal axis 101 is seen in Figs. 1 - 2B. The breakaway intra-medical tubing connector assembly 100 preferably includes a generally cylindrical hollow connector body 102 and a generally cylindrical hollow connector element 104, which is adapted to be partially inserted through the inner volume of the connector body 102. The connector body 102 and the connector element 104 are mutually arranged along the longitudinal axis 101. The connector element 104 is adapted to be freely axially slidable within the connector body 102 along the longitudinal axis 101.

It is a particular feature of an embodiment of the present invention, as seen in Figs. 2A & 2B, that the connector body 102 has a first end 110 and a second end 112. Two preferably identical snap-fit fittings 120 are provided as part of the breakaway intra-medical tubing connector assembly 100. One snap-fit fitting 120 is adapted to be connected to the first end 110 of the connector body 102 and another to the second end 112 of the connector body 102 in a tensile force responsive disconnectable snap fit connection manner.

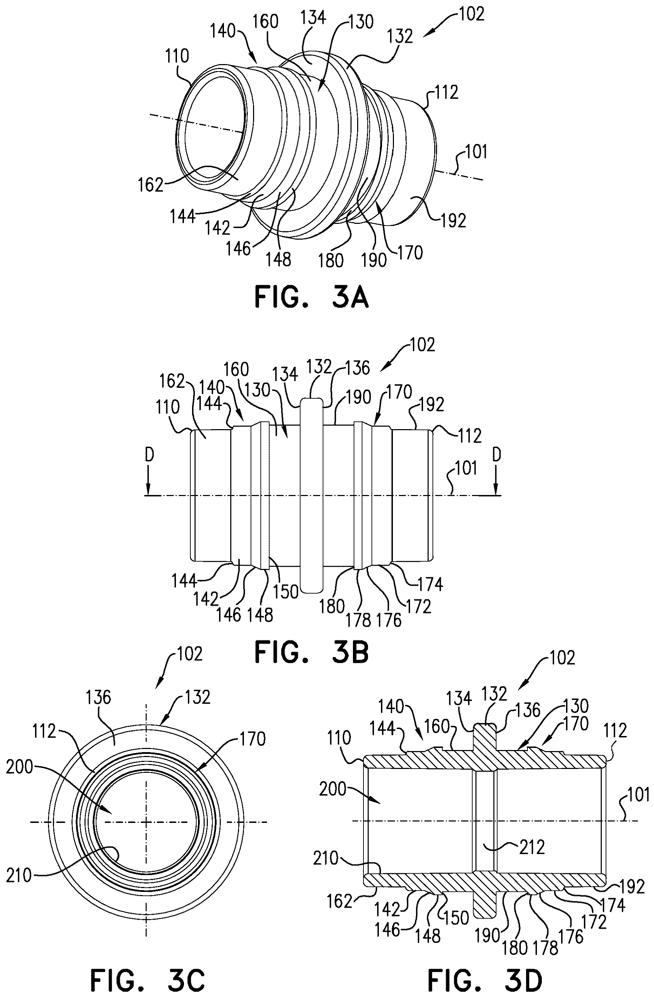

Reference is now made to Figs. 3A - 3D, which are simplified respective perspective, side view, top view and a sectional illustration taken along lines D - D in Fig. 3B of the connector body 102, forming part of the breakaway intra-medical tubing connector assembly 100 of Figs. 1 - 2B.

The connector body 102 is an integrally made cylindrical hollow element arranged along longitudinal axis 101, and preferably made of plastic, such as Polypropylene, Polycarbonate or Polyethylene. The connector body has first open end 110 and second open end 112, as mentioned hereinabove. The connector body has an outer surface 130 and a central flange 132 that is disposed at an intermediate location between the two ends 110 and 112 and extends radially outwardly therefrom. The central flange 132 defines a first annular surface 134 facing the first end 110 and a second annular surface 136 facing the second end 112.

A first snap connection 140 is disposed generally at a central location between the central flange 132 and the first end 110. The first snap connection 140 includes a first annular protrusion 142 extending generally radially outwardly from the outer surface 130 of the connector body 102 and having a first diameter. The first annular protrusion 142 defines a forwardly facing shoulder 144 at one side thereof, which faces the first end 110 and lies in a plane that is generally perpendicular to the longitudinal axis 101. At another side, the first annular protrusion 142 continues to a rearwardly tapered annular protrusion 146, which in turn continues to a second annular protrusion 148, which extends generally radially outwardly from the outer surface 130 of the connector body 102 and having a second diameter, generally greater than the first diameter. The second annular protrusion 148 defines a rearwardly facing shoulder 150, which faces central flange 132 and lies in a plane that is generally perpendicular to the longitudinal axis 101.

A first annular portion 160 is formed between the first snap connection 140 and the central flange 132, and a second annular portion 162 is formed between the first snap connection 140 and the first end 110 of the connector body 102. Both the first annular portion 160 and the second annular portion 162 extend generally axially along longitudinal axis 101.

A second snap connection 170 is disposed generally at a central location between the central flange 132 and the second end 112. The second snap connection 170 includes a first annular protrusion 172 extending generally radially outwardly from the outer surface 130 of the connector body 102 and having a first diameter. The first annular protrusion 172 defines a rearwardly facing shoulder 174 at one side thereof, which faces the second end 112 and lies in a plane that is generally perpendicular to the longitudinal axis 101. At another side, the first annular protrusion 172 continues to a forwardly tapered annular protrusion 176, which in turn continues to a second annular protrusion 178, which extends generally radially outwardly from the outer surface 130 of the connector body 102 and having a second diameter, generally greater than the first diameter. The second annular protrusion 178 defines a forwardly facing shoulder 180, which faces central flange 132 and lies in a plane that is generally perpendicular to the longitudinal axis 101.

A first annular portion 190 is formed between the second snap connection 170 and the central flange 132, and a second annular portion 192 is formed between the second snap connection 170 and the second end 112 of the connector body 102. Both the first annular portion 190 and the second annular portion 192 extend generally axially along longitudinal axis 101.

A through bore 200 extends axially longitudinally along axis 101, through connector body 102, defining an inner surface 210 of the connector body 102. An annular protrusion 212 is generally disposed at a central location between the first and second ends 110 and 112 and generally extends slightly radially inwardly.

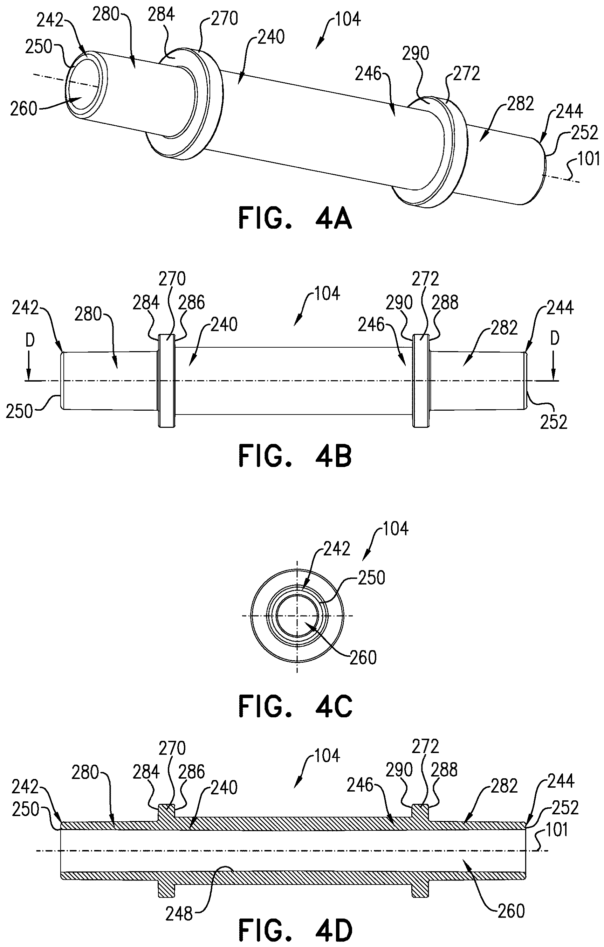

Reference is now made to Figs. 4A - 4D, which are simplified respective perspective, side view, top view and a sectional illustration taken along lines D - D in Fig. 4B of the connector element 104, forming part of the breakaway intra-medical tubing connector assembly 100 of Figs. 1 - 2B.

The connector element 104 is an integrally made element arranged along longitudinal axis 101, and preferably made of plastic, such as Polypropylene, Polycarbonate, Polyethylene. The connector element 104 includes a generally cylindrical hollow portion 240, defining a first end 242 and a second end 244. The cylindrical hollow portion 240 also defines an outer surface 246 and an inner surface 248. A generally rounded edge 250 is formed at the first end 242 and a generally rounded edge 252 is formed at the second end 244. A thoroughgoing bore 260 extends axially longitudinally along the connector element 104.

A first annular protrusion 270 and a second annular protrusion 272 are formed on the cylindrical hollow portion 240. Both annular protrusions 270 and 272 extend generally radially outwardly from the cylindrical hollow portion 240. The first annular protrusion 270 is formed in a relative proximity to the first end 242 and forming a first tubular portion 280 therebetween. The second annular protrusion 272 is formed in a relative proximity to the second end 244 and forming a second tubular portion 282 therebetween. The first tubular portion 280 and the second tubular portion 282 are generally conical. Alternatively, the first tubular portion 280 and the second tubular portion 282 may also be cylindrical.

The first annular protrusion 270 defines a first forwardly facing shoulder 284, which faces the first end 242 and a second rearwardly facing shoulder 286 which faces the second annular protrusion 272. The second annular protrusion 272 defines a first rearwardly facing shoulder 288, which faces the second end 244 and a second forwardly facing shoulder 290 which faces the first annular protrusion 270.

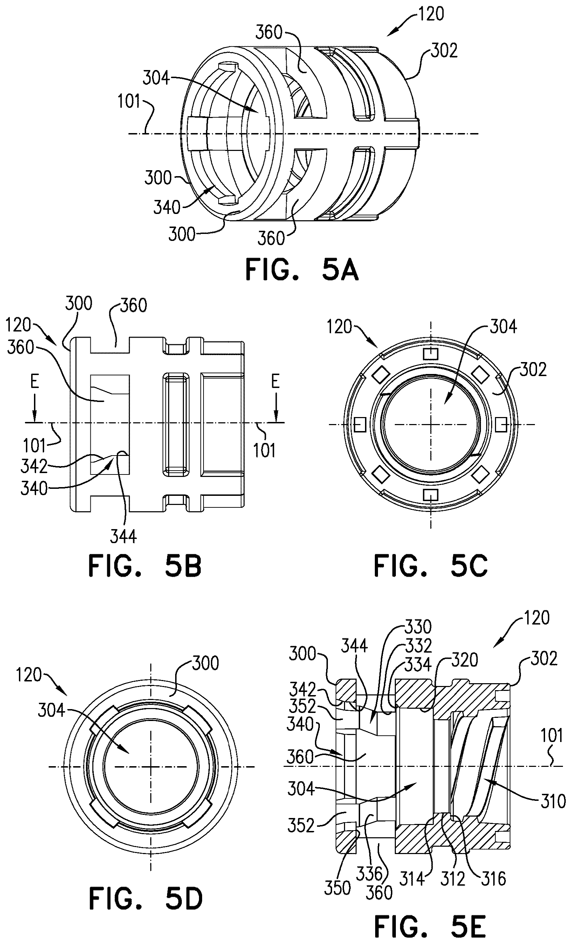

Reference is now made to Figs. 5A - 5E, which are simplified respective perspective, side view, top view, bottom view and a sectional illustration taken along lines E - E in Fig. 5B of the snap-fit fitting 120, forming part of the breakaway intra-medical tubing connector assembly 100 of Figs. 1 - 2B.

The snap-fit fitting 120 is an integrally made element arranged along longitudinal axis 101, and preferably made of plastic, such as Polypropylene, Polycarbonate, Polyethylene. The snap-fit fitting 120 is a generally cylindrical hollow element having a first open end 300 and a second open end 302 and a thoroughgoing bore 304 extending along longitudinal axis 101.

An internally threaded portion 310 forms part of the thoroughgoing bore 304 of the snap-fit fitting 120 and is disposed adjacent second open end 302. The internally threaded portion 310 extends longitudinally along axis 101 and terminates at an annular protrusion 312, which extends slightly radially inwardly with respect to the internally threaded portion 310. The annular protrusion 312 defines an annular shoulder 314 facing the first open end 300 and an annular shoulder 316 facing the second open end 302, both of which lie in a plane, which is generally perpendicular to axis 101.

An annular bore portion 320 extends longitudinally from the annular shoulder 314 of the annular protrusion 312 and typically slightly extends towards the first open end 300.

A snap-fit bore portion 330 extends longitudinally from the annular bore portion 320 up to the first open end 300. The snap-fit bore portion 330 includes an annular portion 332 having a first inner diameter and defining an annular shoulder 334 between the annular portion 332 and the annular bore portion 320. A tapered portion 336 extends forwardly from the annular portion 332 along longitudinal axis 101. A generally circumferential snap connection 340 is disposed between the tapered portion 336 and between the first open end 300. The snap connection 340 has a tapered generally circumferential protrusion 342 extending rearwardly from the first open end 300 and inwardly into thoroughgoing bore 304. The tapered generally circumferential protrusion 342 continues with a generally annular protrusion 344 extending further away from the first open end 300 along axis 101 and defining a shoulder 350, which lies generally in a plane perpendicular to the longitudinal axis 101. Several typically radially spaced apart longitudinal grooves 352 are formed in snap-fit bore portion 330 and extend longitudinally along axis 101, from the first open end 300 up to shoulder 334. Grooves 352 are typically provided for increasing the resilience of the snap-fit fitting 120 adjacent to the first open end 300 thereof.

It is further seen in Figs. 5A & 5B that several radially spaced apart openings 360 are formed through the snap-fit fitting 120 adjacent the second open end 302 thereof, typically provided for the same purpose of increasing the resilience of the snap-fit fitting 120 adjacent to the first open end 300 thereof.

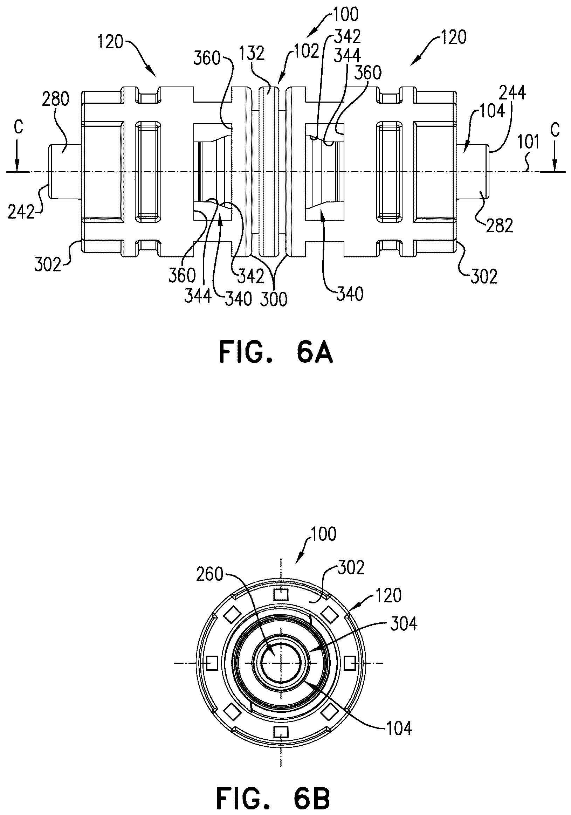

Reference is now made to Figs. 6A - 6C, which are simplified respective side view, top view and a sectional illustration taken along lines C - C in Fig. 6A of the assembled breakaway intra-medical tubing connector assembly100 of Figs. 1 - 2B.

It is seen in Figs. 6A - 6C that the connector element 104 is at least partially inserted into the through bore 200 of the connector body 102, such that the connector element 104 and the connector body 102 extend along mutual longitudinal axis 101.

It is a particular feature of an embodiment of the present invention that the connector element 104 is freely slidable with respect to the connector body 102 along longitudinal axis 101 up to engagement of either the first or the second annular protrusion 270 or 272 of the connector element 104 with the annular protrusion 212 of the connector body 102, specifically up to engagement of either rearwardly facing shoulder 286 with annular protrusion 212 or forwardly facing shoulder 290 with annular protrusion 212.

The first snap-fit fitting 120 is mounted onto one end of the connector body 102, such that the first open end 300 of the first snap-fit fitting 120 is disposed adjacent to and faces the first annular surface 134 of the central flange 132 of the connector body 102. The second snap-fit fitting 120 is mounted onto another end of the connector body 102, such that the first open end 300 of the second snap-fit fitting 120 is disposed adjacent to and faces the second annular surface 136 of the central flange 132 of the connector body 102.

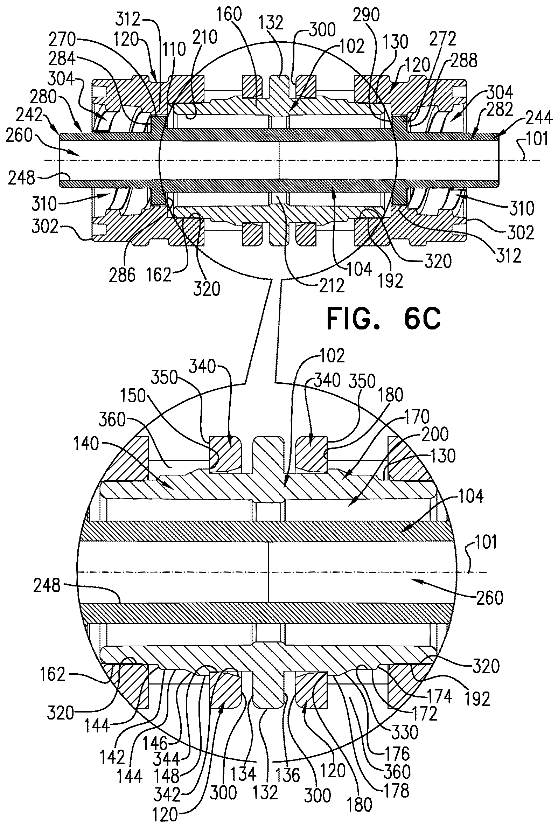

It is a further particular feature of an embodiment of the present invention and is particularly seen in Fig. 6C that one snap-fit fitting 120 is disconnectably snap-fittingly connected to the first snap connection 140 of the connector body 102 and another snap-fit fitting 120 is disconnectably snap-fittingly connected to the second snap connection 170 of the connector body 102 and it is noted that each of the snap-fit fittings 120 can be disconnected from the connector body 102 in response to tensile force exerted on one of the snap-fit fittings 120 along longitudinal axis 101. Specifically, it is seen in Fig. 6C that snap connection 340 of the first snap-fit fitting 120 is disconnectably connected with the first snap connection 140 of the connector body 102, such that shoulder 350 of the snap connection 340 engages the rearwardly facing shoulder 150 of the first snap connection 140. Further seen in Fig. 6C that snap connection 340 of the second snap-fit fitting 120 is disconnectably connected with the second snap connection 170 of the connector body 102, such that shoulder 350 of the snap connection 340 engages rearwardly facing shoulder 180 of the second snap connection 170.

It is seen in Figs. 6A & 6C that the central flange 132 of the connector body 102 is disposed between two respective first open ends 300 of the two snap-fit fittings 120. First open end 110 of the connector body 102 is disposed adjacent the annular protrusion 312 of the first snap-fit fitting 120 and the second open end 112 of the connector body 102 is disposed adjacent the annular protrusion 312 of the second snap-fit fitting 120.

It is noted that the outer surface 130 of the connector body 102 generally corresponds to the geometry of the annular bore portion 320 and to the snap-fit bore portion 330 of the thoroughgoing bore 304 of each of the snap-fit fittings 120. Second annular portions 162 and 192 of connector body 102 are disposed in a sealing engagement with the respective annular bore portions 320 of the snap-fit fittings 120.

It is noted that in this assembled operative orientation of the breakaway intra-medical tubing connector assembly 100, the connector body 102 is static with respect to both snap-fit fittings 120. The connector element 104 is inserted through the through bore 200 of the connector body 102 and at least partially through the thoroughgoing bore 304 of each of the snap-fit fittings 120, preferably such that the first annular protrusion 270 of the connector element 104 is aligned with the annular protrusion 312 of the first snap-fit fitting 120 and the second annular protrusion 272 of the connector element 104 is aligned with the annular protrusion 312 of the second snap-fit fitting 120.

It is particularly seen in Figs. 6A - 6C that the first tubular portion 280 of the connector element 104 is preferably partially surrounded by internally threaded portion 310 of the first snap-fit fitting 120 and partially protrudes outwardly from the second open end 302 of the first snap-fit fitting 120. Additionally, the second tubular portion 282 of the connector element 104 is partially surrounded by internally threaded portion 310 of the second snap-fit fitting 120 and partially protrudes outwardly from the second open end 302 of the second snap-fit fitting 120.

It is a particular feature of an embodiment of the present invention that the connector element 104 is axially slidable with respect to both the connector body 102 and the first and second snap-fit fittings 120.

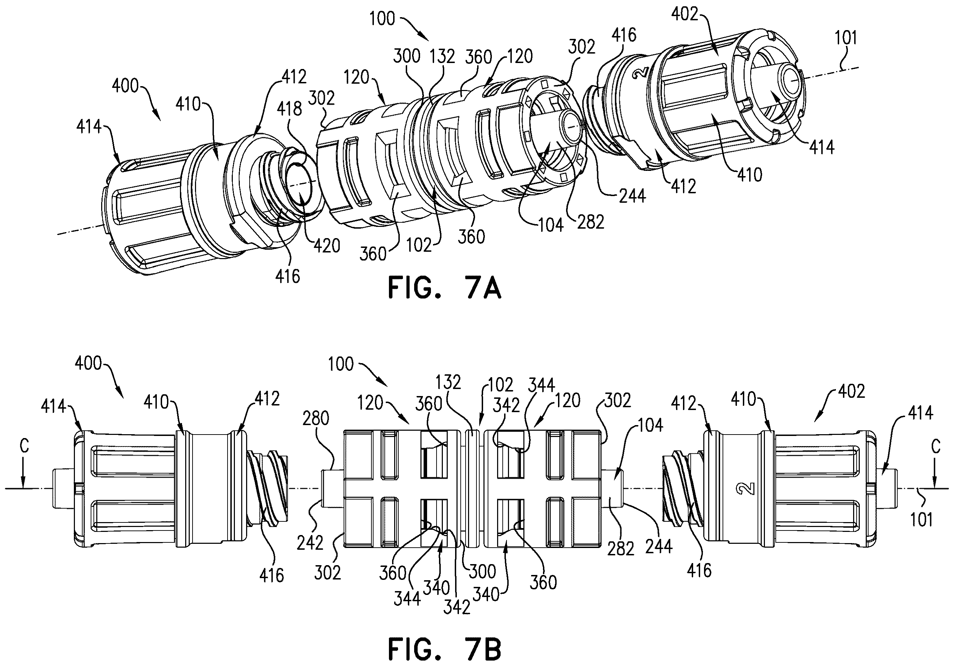

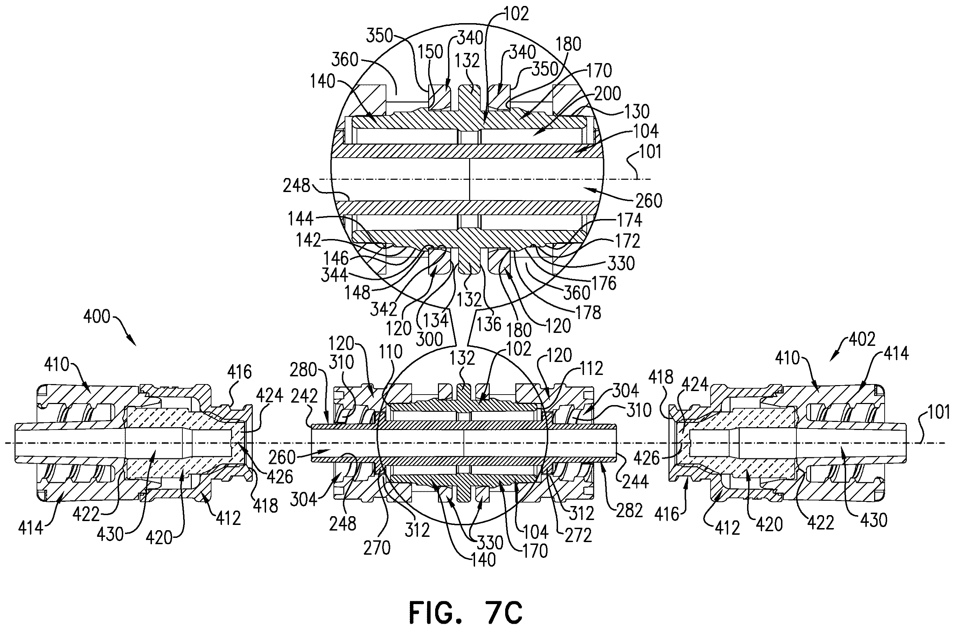

Reference is now made to Figs. 7A - 7C, which are simplified respective perspective view, side view and a sectional illustration taken along lines C - C in Fig. 7B of the breakaway intra-medical tubing connector assembly 100 of Figs. 6A - 6C, shown in a first operative orientation, just prior to connection thereof with a first and a second luer-actuated valve assemblies constructed and operative in accordance with an embodiment of the present invention.

It is seen in Figs. 7A - 7C that a first luer-actuated valve assembly 400 and a second luer-actuated valve assembly 402 are about to be threadably connected to the breakaway intra-medical tubing connector assembly 100. The first luer-actuated valve assembly 400 is adapted to be threadably connected to the first snap-fit fitting 120 and the second luer-actuated valve assembly 402 is adapted to be threadably connected to the second snap-fit fitting 120 of the breakaway intra-medical tubing connector assembly 100.

It is appreciated that the first and second luer-actuated valve assemblies 400 and 402 are commercially available from various manufacturers, such as Haemopharm, Halkey-Roberts, Paolo Gobbi Frattini S.r.l. It is noted that both first and second luer-actuated valve assemblies 400 and 402 are preferably identical. Alternatively, two different first and second luer-actuated valve assemblies 400 and 402 can be used with the breakaway intra-medical tubing connector assembly 100.

Both luer-actuated valve assemblies 400 and 402 generally extend along longitudinal axis 101 and generally include a housing 410 having a female luer portion 412 and a male luer portion 414 on an opposite end of the housing 410. The female luer portion 412 has an externally threaded portion 416 and an opening 418. A compressible sealing element 420 is disposed within the housing 410. The compressible sealing element 420 has a first open end 422 and a second selectably openable end 424, including a selectively openable slit 426. The selectably openable end 424 of the sealing element 420 is disposed across the opening 418 of the female luer portion 412 in its normally closed operative orientation and thus is adapted to sealingly close the opening 418 when the sealing element 420 is not compressed. It is noted that the sealing element 420 is biased to its normally closed operative orientation when no stress is applied thereupon. A fluid flow passage 430 is defined by the inner volume of the sealing element 420 and the inner volume of the male luer portion 414, however fluid flow is not permitted through the fluid flow passage 430 when the slit 426 is closed and the second selectably openable end 424 provides a swabbable surface that may be cleaned by the physician in order to prevent contamination.

It is noted that instead of the male luer portion 414, a female luer portion or tube connection element may be utilized.

It is noted that the sealing element 420 is biased to its normally closed position once a compression force exerted thereon is removed.

It is noted that alternatively, any other type of luer-actuated valve assembly can be used in conjunction with the breakaway intra-medical tubing connector assembly 100, constructed and operative in accordance with an embodiment of the present invention.

It is specifically seen in Figs. 7A - 7C that the luer-actuated valve assemblies 400 and 402 are not yet mounted onto the breakaway intra-medical tubing connector assembly 100, thus both luer-actuated valve assemblies 400 and 402 are sealingly closed in this operative orientation.

It is further noted that the male luer portions 414 of each of the luer-actuated valve assemblies 400 and 402 are adapted to be connected to a medical tubing, such as for example, an IV line or a catheter.

Spatial relationships between the various components of the breakaway intra-medical tubing connector assembly 100 preferably remains the same as described with reference to Figs. 6A - 6C.

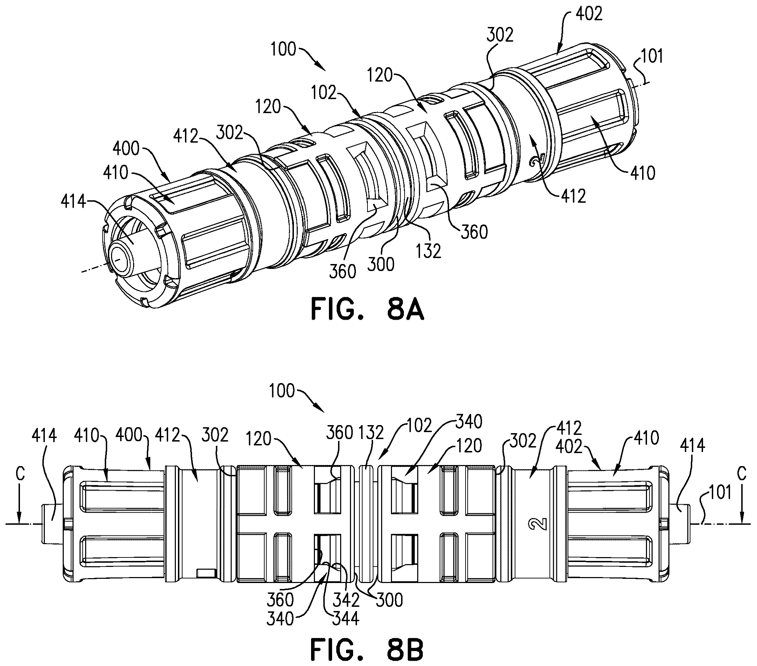

Reference is now made to Figs. 8A - 8C, which are simplified respective perspective view, side view and a sectional illustration taken along lines C - C in Fig. 8B of the breakaway intra-medical tubing connector assembly 100 of Figs. 6A - 6C, shown in a second operative orientation, when the first and the second luer-actuated valve assemblies 400 and 402 of Figs. 7A - 7C are connected to the breakaway intra-medical tubing connector assembly 100.

It is seen in Figs. 8A - 8C that both the first luer-actuated valve assembly 400 and the second luer-actuated valve assembly 402 are threadably connected to the breakaway intra-medical tubing connector assembly 100. The first luer-actuated valve assembly 400 is threadably connected to the first snap-fit fitting 120 by means of engagement between the externally threaded portion 416 of the female luer portion 412 and between the internally threaded portion 310 of the first snap-fit fitting 120. The second luer-actuated valve assembly 402 is threadably connected to the second snap-fit fitting 120 by means of engagement between the externally threaded portion 416 of the female luer portion 412 and between the internally threaded portion 310 of the second snap-fit fitting 120.

It is a particular feature of an embodiment of the present invention that upon connection of both the first and the second luer-actuated valve assemblies 400 and 402 to the breakaway intra-medical tubing connector assembly 100, a fluid flow passage is established between two medical tubes, each of which is adapted to be connected to the respective male luer portion 414 of the first and second luer-actuated valve assemblies 400 and 402.

It is a further particular feature of an embodiment of the present invention that the inner diameter of the fluid flow passage provided between the first and second luer-actuated valve assemblies 400 and 402 is substantially the same as the diameter of the thoroughgoing bore 260 of the connector element 104.

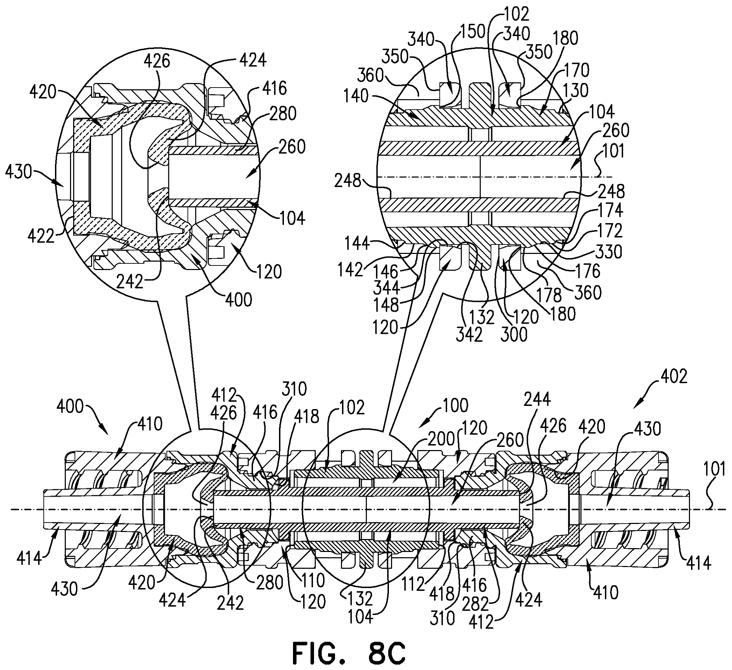

Specifically, it is seen in Fig. 8C that upon threaded engagement of both the first and second luer-actuated valve assemblies 400 and 402, the first tubular portion 280 of the connector element 104 compresses the sealing element 420 of the first luer-actuated valve assembly 400 and thus urges opening of selectively openable slit 426 thereof. Similarly, upon threaded engagement of both the first and second luer-actuated valve assemblies 400 and 402, the second tubular portion 282 of the connector element 104 compresses the sealing element 420 of the second luer-actuated valve assembly 402 and thus urges opening of selectively openable slit 426 thereof. The fluid flow passage is established from a first medical tube that is adapted to be connected to the male luer portion 414 of the first luer-actuated valve assembly 400, through the fluid flow passage 430 thereof, via slit 426 of the sealing element 420 and through the thoroughgoing bore 260 of the connector element 104, further via slit 426 of the sealing element 420 of the second luer-actuated valve assembly 402, through fluid flow passage 430 thereof and finally into a second medical tube that is adapted to be connected to the male luer portion 414 of the second luer-actuated valve assembly 402, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 100 constructed and operative in accordance with an embodiment of the present invention.

It is noted that compression forces exerted on both luer-actuated valve assemblies 400 and 402 by the connector element 104 are enabled by the fact that both luer-actuated valve assemblies 400 and 402 are held in place relative to the breakaway intra-medical tubing connector assembly 100 due to snap-fit connection between snap-fit fittings 120 and the connector body 102.

The breakaway intra-medical tubing connector assembly 100 is disposable in case the respective snap connections 140 and 330 provided between the first snap-fit fitting 120 and the connector body 102 and snap connections 170 and 330 provided between the second snap-fit fitting 120 and the connector body 102 are deformable, such that additional engagement of one of the snap-fit fittings 120 with the connector body 102 is not intended after first use.

It is noted that alternatively, any other type of luer-actuated valve assembly can be used in conjunction with the breakaway intra-medical tubing connector assembly 100, constructed and operative in accordance with an embodiment of the present invention.

It is noted that both the first and the second luer-actuated valve assemblies 400 and 402 may be freely rotated about axis 101 along with the respective snap-fit fittings 120 while being engaged with the intra-medical tubing connector assembly 100, without causing disengagement of the snap-fit fittings 120 with luer-actuated valve assemblies 400 and 402 therefrom.

It is further noted that upon connection of one of the luer actuated valve assemblies 400 and 402, the connector element 104 is axially slidably moveable and thus provides for both of the luer-actuated valve assemblies 400 and 402 to remain sealed up until both of the luer-actuated valve assemblies 400 and 402 are connected to their respective snap-fit fittings 120, which provides for opening of the sealing members 420 of both luer-actuated valve assemblies 400 and 402.

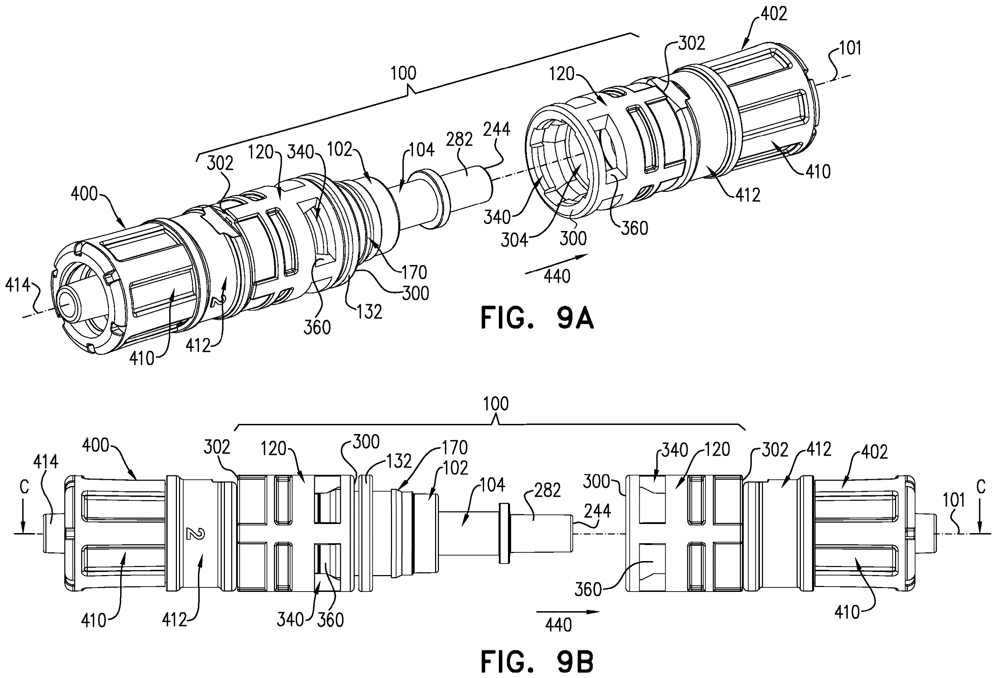

Reference is now made to Figs. 9A - 9C, which are simplified respective perspective view, side view and a sectional illustration taken along lines C - C in Fig. 9B of the breakaway intra-medical tubing connector assembly 100 of Figs. 6A - 6C, shown in a third operative orientation, when one of the luer-actuated valve assemblies of Figs. 7A - 7C is disconnected from the breakaway intra-medical tubing connector assembly 100.

It is seen in Figs. 9A - 9C that a tensile force in a direction indicated by arrow 440 is applied on either the snap-fit fitting 120, the second luer-actuated valve assembly 402 or the second medical tube and thus causes disconnection between the snap-fit fitting 120 and the connector body 102. It is seen that the second luer-actuated valve assembly 402 is threadably connected to the second snap-fit fitting 120, thus can not be disconnected therefrom upon application of tensile force, however the snap fit connection provided between the snap-fit fitting 120 and between the connector body 102 can be broken upon application of sufficient tensile force. According to an embodiment of the present invention, tensile force in the range of 0.5kgf - 3kgf is required in order to break the snap-fit connection between the snap-fit fitting 120 and the connector body 102.

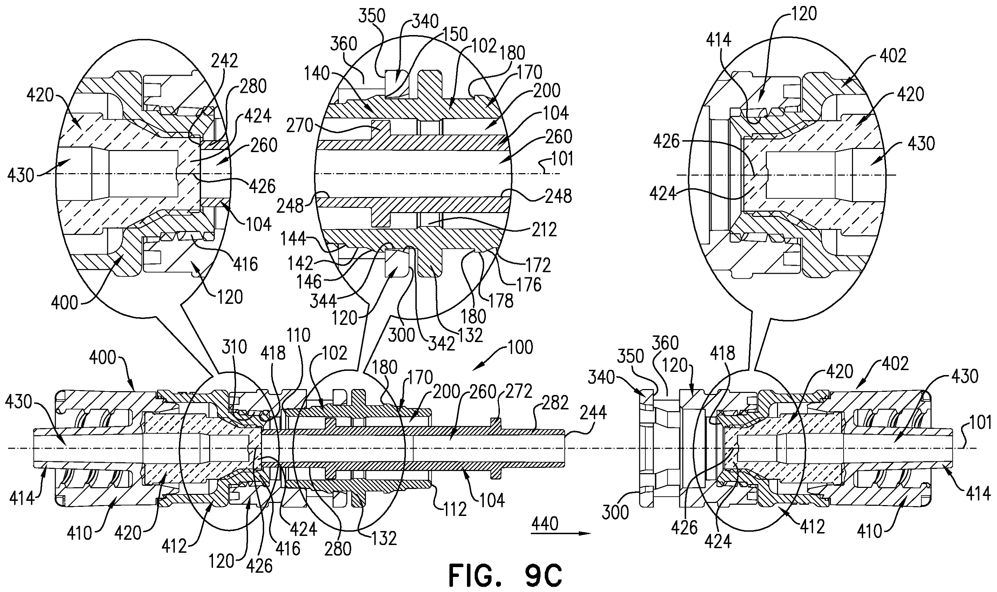

It is specifically seen in Fig. 9C that upon application of tensile force upon the snap fit-fitting 120 along longitudinal axis 101 in the direction of arrow 440, the snap connection 340 of the second snap-fit fitting 120 is disengaged from the second snap connection 170 of the connector body 102, such that shoulder 350 of the snap connection 340 disengages rearwardly facing shoulder 180 of the second snap connection 170 due to the relative resiliency of the snap-fit fitting 120 provided by the material it is formed from and preferably also due to the existence of longitudinal grooves 352 and by openings 360, as described in detail hereinabove.

It is noted that one of the luer actuated valve assemblies 400 and 402 may be disconnected upon application of a different tensile force magnitude. In case that snap connection 170 extends radially outwardly to a lesser extent in comparison with snap connection 140, then the second luer actuated valve assembly 402 is intended to be disconnected first from the connector body 102. Alternatively, the connector body 102 can be designed differently such that the first luer actuated valve assembly 400 is disconnected first, in case snap connection 140 extends radially outwardly to a lesser extent in comparison with snap connection 170.

It is also seen in Fig. 9C that upon disconnection of the second luer-actuated valve assembly 402, the connector element 104 is urged to be displaced axially along longitudinal axis 101 in the direction of arrow 440 and out of the first luer-actuated valve assembly, due to the biasing force of the sealing element 420 of the first luer-actuated valve assembly 400 that is applied upon the connector element 104.

It is a particular feature of an embodiment of the present invention that upon disconnection of the second luer-actuated valve assembly 402 from the breakaway intra-medical tubing connector assembly 100 and according to this particular embodiment upon disconnection of one of the snap-fit fittings 120 along with one of the luer-actuated valve assemblies 400 or 402 from the connector body 102, both of the luer-actuated valve assemblies are automatically bidirectionally closed, thus preventing fluid flow passage from each of the two medical tubes. Upon discarding of the breakaway intra-medical tubing connector assembly 100 along with the two snap-fit fittings 120, two sealed and swabbale luer-actuated valve assemblies 400 and 402 are provided, which obviate the need to replace the entire medical set, and only requires replacement of the breakaway intra-medical tubing connector assembly 100.

In this third operative orientation, the first tubular portion 280 of the connector element 104 is fully surrounded by the internally threaded portion 310 of the first snap-fit fitting 120 and the connector body 102, such that the connector element 104 does not protrude outwardly from the second open end 302 of the first snap-fit fitting 120. The second tubular portion 282 of the connector element 104 protrudes outwardly from the connector body 102.

Upon disconnection of one of the snap fit fittings 120 along with the second luer-actuated valve assembly 402, the connector element 104 is displaced in the direction indicated by arrow 440, preferably up to engagement of first annular protrusion 270 of the connector element 104 with annular protrusion 212 of the connector body 102 to prevent the connector element falling out from the breakaway intra-medical tubing connector assembly 100.

The first snap-fit fitting 120 can be threadably disengaged from the first and second luer-actuated valve assemblies 400 and 402, thus leaving the luer-actuated valve assemblies 400 and 402 along with the medical tubes associated therewith within the desired treatment location. Following disconnection of the snap-fit fittings 120 from the first and second luer-actuated valve assemblies 400 and 402, the valve assemblies are swabbable and can be cleaned by the user.

It is noted that tensile force exerted on the medical set such as IV line may be unintentional, but due to the presence of the breakaway intra-medical tubing connector assembly 100, the catheter or other medical tube associated with one of the luer-actuated valve assemblies 400 and 402 remains in its position within the treatment site and the luer-actuated valve assemblies 400 and 402 are safely sealed once one of the luer-actuated valve assemblies 400 and 402 is disconnected from the breakaway intra-medical tubing connector assembly 100. Therefore, no fluid can unintentionally flow out of the IV line and risk of contamination of the treatment site is prevented due to sealing of the luer-actuated valve assemblies 400 and 402 and the ability to clean the exterior surface of the sealing element 420.

It is noted that alternatively disconnection of one of the luer-actuated valve assemblies 400 and 402 from the breakaway intra-medical tubing connector assembly 100 may be intentional in order to provide the physician with access point to the IV line. Once one of the luer-actuated valve assemblies 400 and 402 is exposed, it may be used for administering medicament into the treatment site within the patient body or alternatively for withdrawing a blood sample from the patient's body.

It is a particular feature of an embodiment of the present invention that the breakaway intra-medical tubing connector assembly 100 associated with medical connectors, such as luer-actuated valve assemblies 400 and 402 enables both safe unintentional disconnection of one of the luer-actuated valve assemblies and provides access point to the IV line through the exposed sealing element 420 when one of the luer-actuated valve assemblies 400 and 402 is intentionally disconnected.

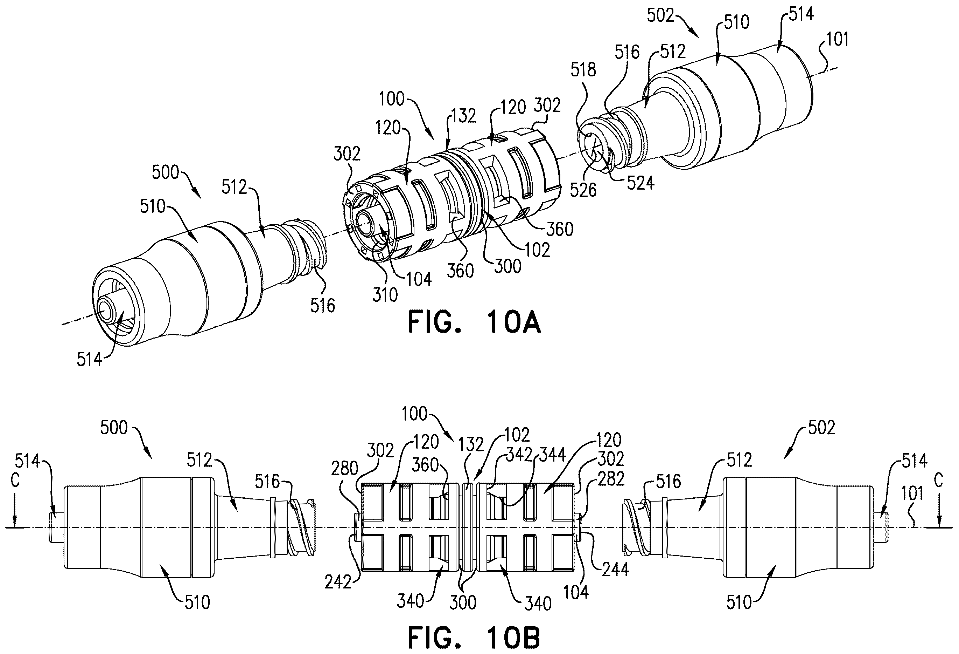

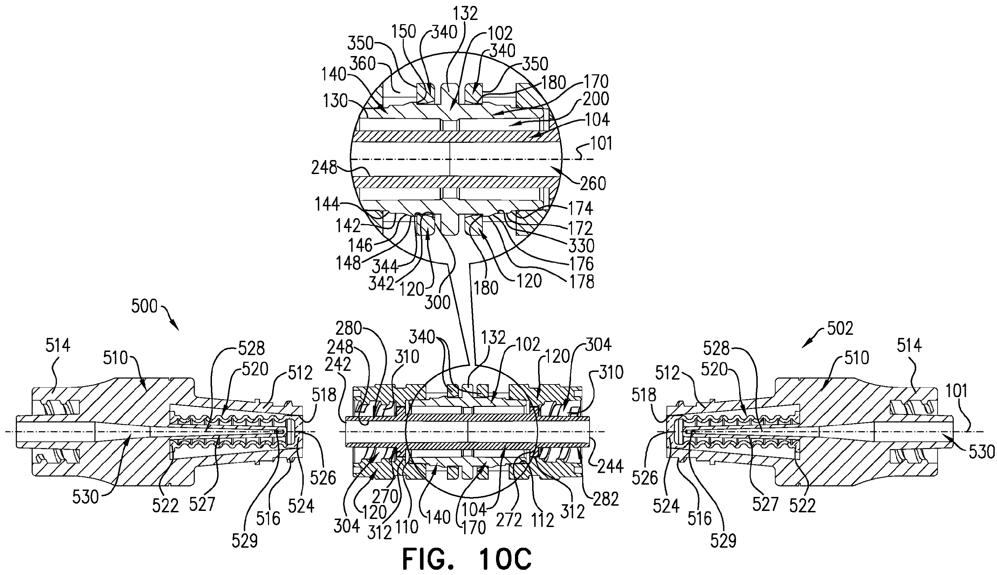

Reference is now made to Figs. 10A - 10C, which are simplified respective perspective view, side view and a sectional illustration taken along lines C - C in Fig. 10B of the breakaway intra-medical tubing connector assembly 100 of Figs. 6A - 6C, shown in a first operative orientation, just prior to connection thereof with a first and a second luer-actuated valve assemblies constructed and operative in accordance with another embodiment of the present invention.

It is seen in Figs. 10A - 10C that a first luer-actuated valve assembly 500 and a second luer-actuated valve assembly 502 are about to be threadably connected to the breakaway intra-medical tubing connector assembly 100. The first luer-actuated valve assembly 500 is adapted to be threadably connected to the first snap-fit fitting 120 and the second luer-actuated valve assembly 502 is adapted to be threadably connected to the second snap-fit fitting 120 of the breakaway intra-medical tubing connector assembly 100.

It is appreciated that the first and second luer-actuated valve assemblies 500 and 502 are commercially available from various manufacturers, such as ICU Medical. It is noted that both first and second luer-actuated valve assemblies 500 and 502 are preferably identical. Alternatively, two different first and second luer-actuated valve assemblies 500 and 502 can be used with the breakaway intra-medical tubing connector assembly 100.

Both luer-actuated valve assemblies 500 and 502 generally extend along longitudinal axis 101 and generally include a housing 510 having a female luer portion 512 and a male luer portion 514 on an opposite end of the housing 510. The female luer portion 512 has an externally threaded portion 516 and an opening 518. A compressible sealing element 520 is disposed within the housing 510. The compressible sealing element 520 has a first open end 522 and a second selectably openable end 524, including a selectively openable slit 526. The selectably openable end 524 of the sealing element 520 is disposed across the opening 518 of the female luer portion 512 in its normally closed operative orientation and thus is adapted to sealingly close the opening 518 when the sealing element 520 is not compressed. It is noted that the sealing element 520 is biased to its normally closed operative orientation when no stress is applied thereupon. A rigid spike 527 is disposed within the sealing element 520. The rigid spike 527 preferably is hollow and defines an inner volume 528 and an opening 529, which is configured to communicate therewith. A fluid flow passage 530 is defined by the inner volume 528 of the rigid spike 527 and the inner volume of the male luer portion 514, however fluid flow is not permitted through the fluid flow passage 530 when the slit 526 is closed and the second selectably openable end 524 provides a swabbable surface that may be cleaned by the physician in order to prevent contamination.

It is noted that instead of the male luer portion 514, a female luer portion or tube connection element may be utilized.

It is noted that the sealing element 520 is biased to its normally closed position once a compression force exerted thereon is removed, whereas in this position the opening 529 of the rigid spike 527 is sealed by the sealing element 520 and fluid flow passage through the luer-actuated valve assembly 500 or 502 is not permitted.

It is noted that alternatively, any other type of luer-actuated valve assembly can be used in conjunction with the breakaway intra-medical tubing connector assembly 100, constructed and operative in accordance with an embodiment of the present invention. The breakaway intra-medical tubing connector assembly 100 used in conjunction with luer-actuated valve assemblies 500 and 502 is generally identical to the breakaway intra-medical tubing connector assembly 100 described with reference to Figs. 1 - 9C, other than the longitudinal dimension of the internally threaded portion 310 of the snap-fit fittings 120, which is made longer to suit the geometry of luer-actuated valve assembly, such as 500 and 502.

It is specifically seen in Figs. 10A - 10C that the luer-actuated valve assemblies 500 and 502 are not yet mounted onto the breakaway intra-medical tubing connector assembly 100, thus both luer-actuated valve assemblies 500 and 502 are sealingly closed in this operative orientation.

It is further noted that the male luer portions 514 of each of the luer-actuated valve assemblies 500 and 502 are adapted to be connected to a medical tubing, such as for example, an IV line or a catheter.

Spatial relationships between the various components of the breakaway intra-medical tubing connector assembly 100 are the same as described with reference to Figs. 6A -6C.

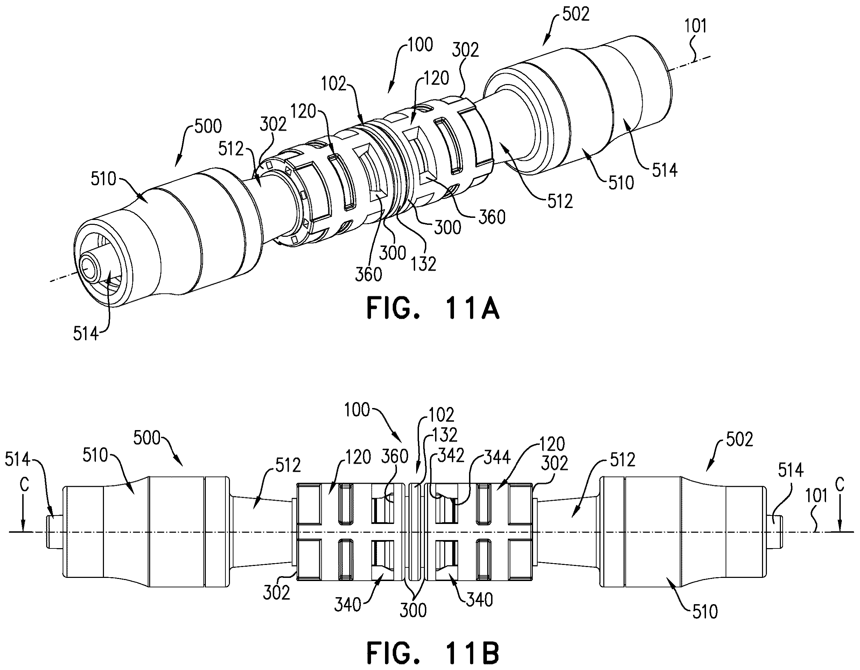

Reference is now made to Figs. 11A - 11C, which are simplified respective perspective view, side view and a sectional illustration taken along lines C - C in Fig. 11B of the breakaway intra-medical tubing connector assembly 100 of Figs. 6A - 6C, shown in a second operative orientation, when the first and the second luer-actuated valve assemblies 500 and 502 of Figs. 10A - 10C are connected to the breakaway intra-medical tubing connector assembly 100.

It is seen in Figs. 11A - 11C that both the first luer-actuated valve assembly 500 and the second luer-actuated valve assembly 502 are threadably connected to the breakaway intra-medical tubing connector assembly 100. The first luer-actuated valve assembly 500 is threadably connected to the first snap-fit fitting 120 by means of engagement between the externally threaded portion 516 of the female luer portion 512 and between the internally threaded portion 310 of the first snap-fit fitting 120. The second luer-actuated valve assembly 502 is threadably connected to the second snap-fit fitting 120 by means of engagement between the externally threaded portion 516 of the female luer portion 512 and between the internally threaded portion 310 of the second snap-fit fitting 120.

It is a particular feature of an embodiment of the present invention that upon connection of both the first and the second luer-actuated valve assemblies 500 and 502 to the breakaway intra-medical tubing connector assembly 100, a fluid flow passage is established between two medical tubes, each of which is adapted to be connected to the respective male luer portion 514 of the first and second luer-actuated valve assemblies 500 and 502.

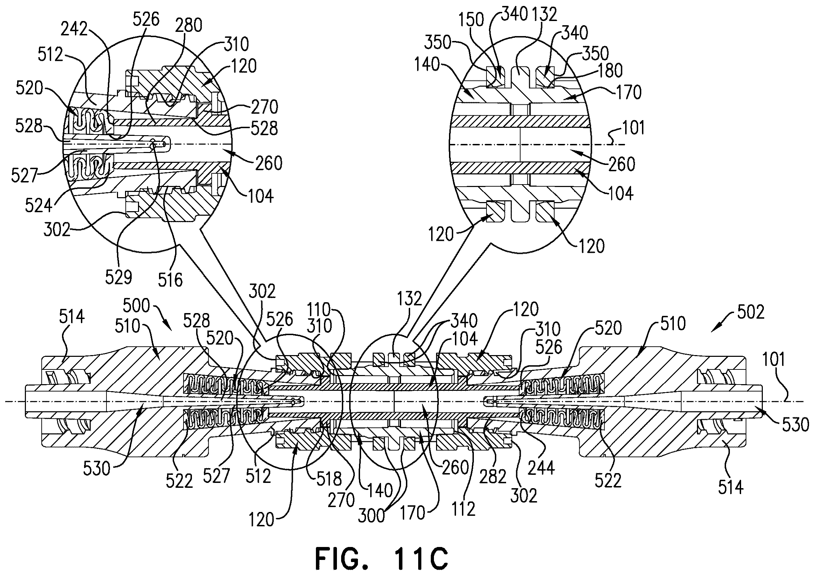

Specifically, it is seen in Fig. 11C that upon threaded engagement of both the first and second luer-actuated valve assemblies 500 and 502, the first tubular portion 280 of the connector element 104 compresses the sealing element 520 of the first luer-actuated valve assembly 500 and thus urges opening of selectively openable slit 526 thereof, thereby exposing the opening 529 of rigid spike 527 of the luer-actuated valve assembly 500. Similarly, upon threaded engagement of both the first and second luer-actuated valve assemblies 500 and 502, the second tubular portion 282 of the connector element 104 compresses the sealing element 520 of the second luer-actuated valve assembly 502 and thus urges opening of selectively openable slit 526 thereof, thereby exposing the opening 529 of rigid spike 527 of the luer-actuated valve assembly 502. The fluid flow passage is established from a first medical tube that is adapted to be connected to the male luer portion 514 of the first luer-actuated valve assembly 500, through the fluid flow passage 530 thereof, via opening 529 of the rigid spike 527 of the sealing element 520 and through the thoroughgoing bore 260 of the connector element 104, further via opening 529 of the rigid spike 527 of the sealing element 520 of the second luer-actuated valve assembly 502, through fluid flow passage 530 thereof and finally into a second medical tube that is adapted to be connected to the male luer portion 514 of the second luer-actuated valve assembly 502, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 100 constructed and operative in accordance with an embodiment of the present invention.

It is noted that compression forces exerted on both luer-actuated valve assemblies 500 and 502 by the connector element 104 are enabled by the fact that both luer-actuated valve assemblies 500 and 502 are held in place relative to the breakaway intra-medical tubing connector assembly 100 due to snap-fit connection between snap-fit fittings 120 and the connector body 102.

The breakaway intra-medical tubing connector assembly 100 is preferably disposable in case the respective snap connections 140 and 330 provided between the first snap-fit fitting 120 and the connector body 102 and snap connections 170 and 330 provided between the second snap-fit fitting 120 and the connector body 102 are deformable, such that additional engagement of one of the snap-fit fittings 120 with the connector body 102 is not intended after first use.

It is noted that alternatively, any other type of luer-actuated valve assembly can be used in conjunction with the breakaway intra-medical tubing connector assembly 100, constructed and operative in accordance with an embodiment of the present invention.

It is noted that both the first and the second luer-actuated valve assemblies 500 and 502 may be freely rotated about axis 101 along with the respective snap-fit fittings 120 while being engaged with the intra-medical tubing connector assembly 100, without causing disengagement of the snap-fit fittings 120 with luer-actuated valve assemblies 500 and 502 therefrom.

It is further noted that upon connection of one of the luer actuated valve assemblies 500 and 502, the connector element 104 is axially slidably moveable and thus provides for both of the luer-actuated valve assemblies 500 and 502 to remain sealed up until both of the luer-actuated valve assemblies 500 and 502 are connected to their respective snap-fit fittings 120, which provides for opening of the sealing members 520 of both luer-actuated valve assemblies 500 and 502.

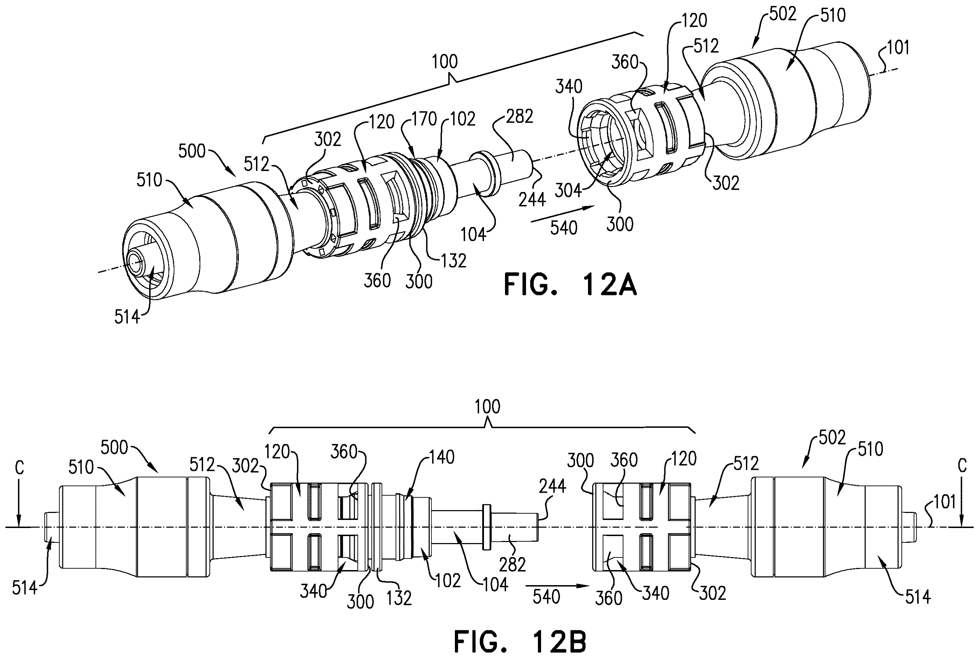

Reference is now made to Figs. 12A - 12C, which are simplified respective perspective view, side view and a sectional illustration taken along lines C - C in Fig. 12B of the breakaway intra-medical tubing connector assembly 100 of Figs. 6A - 6C, shown in a third operative orientation, when one of the luer-actuated valve assemblies 500 and 502 of Figs. 10A -10C is disconnected from the breakaway intra-medical tubing connector assembly.

It is seen in Figs. 12A - 12C that a tensile force in a direction indicated by arrow 540 is applied on either the snap-fit fitting 120, the second luer-actuated valve assembly 502 or the second medical tube and thus causes disconnection between the snap-fit fitting 120 and the connector body 102. It is seen that the second luer-actuated valve assembly 502 is threadably connected to the second snap-fit fitting 120, thus can not be disconnected therefrom upon application of tensile force, however the snap fit connection provided between the snap-fit fitting 120 and between the connector body 102 can be broken upon application of sufficient tensile force. According to an embodiment of the present invention, tensile force in the range of 0.5kgf - 3kgf is required in order to break the snap-fit connection between the snap-fit fitting 120 and the connector body 102.

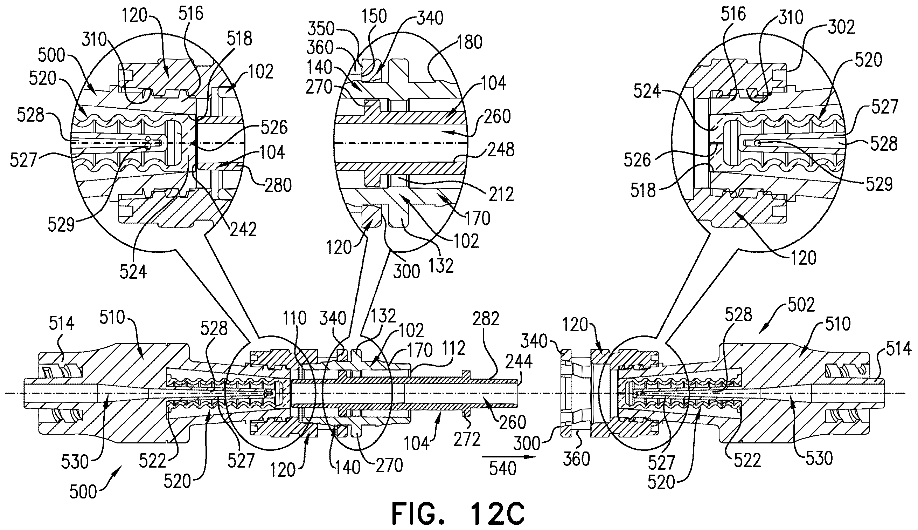

It is specifically seen in Fig. 12C that upon application of tensile force upon the snap fit-fitting 120 along longitudinal axis 101 in the direction of arrow 540, the snap connection 340 of the second snap-fit fitting 120 is disengaged from the second snap connection 170 of the connector body 102, such that shoulder 350 of the snap connection 340 disengages rearwardly facing shoulder 180 of the second snap connection 170 due to the relative resiliency of the snap-fit fitting 120 provided by provided by the material it is formed of as well as by the longitudinal grooves 352 and by openings 360, as described in detail hereinabove.

It is noted that one of the luer actuated valve assemblies 500 and 502 may be disconnected upon application of a different tensile force magnitude. In case that snap connection 170 extends radially outwardly to a lesser extent in comparison with snap connection 140, then the second luer actuated valve assembly 502 is intended to be disconnected first from the connector body 102. Alternatively, the connector body 102 can be designed differently such that the first luer actuated valve assembly 500 is disconnected first, in case snap connection 140 extends radially outwardly to a lesser extent in comparison with snap connection 170.

It is also seen in Fig. 12C that upon disconnection of the second luer-actuated valve assembly 502, the connector element 104 is urged to be displaced axially along longitudinal axis 101 in the direction of arrow 540 and out of the first luer-actuated valve assembly 500, due to the biasing force of the sealing element 520 of the first luer-actuated valve assembly 500 that is applied upon the connector element 104.

Upon disconnection of one of the snap fit fittings 120 along with the second luer-actuated valve assembly 402, the connector element 104 is displaced in the direction indicated by arrow 540, preferably up to engagement of first annular protrusion 270 of the connector element 104 with annular protrusion 212 of the connector body 102 to prevent the connector element falling out from the breakaway intra-medical tubing connector assembly 100. It is a particular feature of an embodiment of the present invention that upon disconnection of the second luer-actuated valve assembly 502 from the breakaway intra-medical tubing connector assembly 100 and according to this particular embodiment upon disconnection of one of the snap-fit fittings 120 along with one of the luer-actuated valve assemblies 500 or 502 from the connector body 102, both of the luer-actuated valve assemblies are automatically bidirectionally closed, thus preventing fluid flow passage out of each of the two medical tubes. Upon discarding of the breakaway intra-medical tubing connector assembly 100 along with the two snap-fit fittings 120, two sealed and swabbale luer-actuated valve assemblies 500 and 502 are provided, which obviate the need to replace the entire medical set, and only requires replacement of the breakaway intra-medical tubing connector assembly 100.

In this third operative orientation, the first tubular portion 280 of the connector element 104 is fully surrounded by the internally threaded portion 310 of the first snap-fit fitting 120 and the connector body 102, such that the connector element 104 does not protrude outwardly from the second open end 302 of the first snap-fit fitting 120. The second tubular portion 282 of the connector element 104 protrudes outwardly from the connector body 102.

The first snap-fit fitting 120 can be threadably disengaged from the first and second luer-actuated valve assemblies 500 and 502, thus leaving the luer-actuated valve assemblies 500 and 502 along with the medical tubes associated therewith within the desired treatment location.

It is noted that tensile force exerted on the medical set such as IV line may be unintentional, but due to the presence of the breakaway intra-medical tubing connector assembly 100, the catheter or other medical tube associated with one of the luer-actuated valve assemblies 500 and 502 remains in its position within the treatment site and the luer-actuated valve assemblies 500 and 502 are safely sealed once one of the luer-actuated valve assemblies 500 and 502 is disconnected from the breakaway intra-medical tubing connector assembly 100. Therefore, no fluid can unintentionally flow out of the IV line and risk of contamination of the treatment site is prevented due to sealing of the luer-actuated valve assemblies 500 and 502 and the ability to clean the exterior surface of the sealing element 520.

It is noted that alternatively disconnection of one of the luer-actuated valve assemblies 500 and 502 from the breakaway intra-medical tubing connector assembly 100 may be intentional in order to provide the physician with access point to the IV line. Once one of the luer-actuated valve assemblies 500 and 502 is exposed, it may be used for administering medicament into the treatment site within the patient body or alternatively for withdrawing a blood sample from the patient's body.

It is a particular feature of an embodiment of the present invention that the breakaway intra-medical tubing connector assembly 100 associated with medical connectors, such as luer-actuated valve assemblies 500 and 502 enables both safe unintentional disconnection of one of the luer-actuated valve assemblies and provides access point to the IV line through the exposed sealing element 520 when one of the as luer-actuated valve assemblies 500 and 502 is intentionally disconnected.

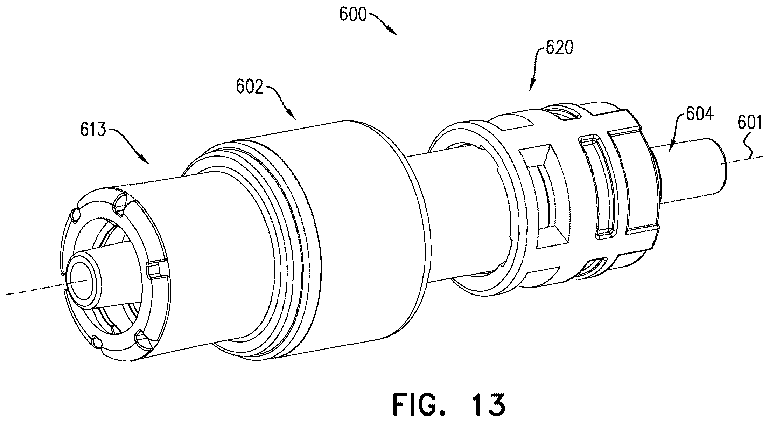

Reference is now made to Fig. 13, which is a simplified pictorial illustration of an assembled breakaway intra-medical tubing connector assembly, constructed and operative in accordance with another embodiment of the present invention. Reference is additionally made to

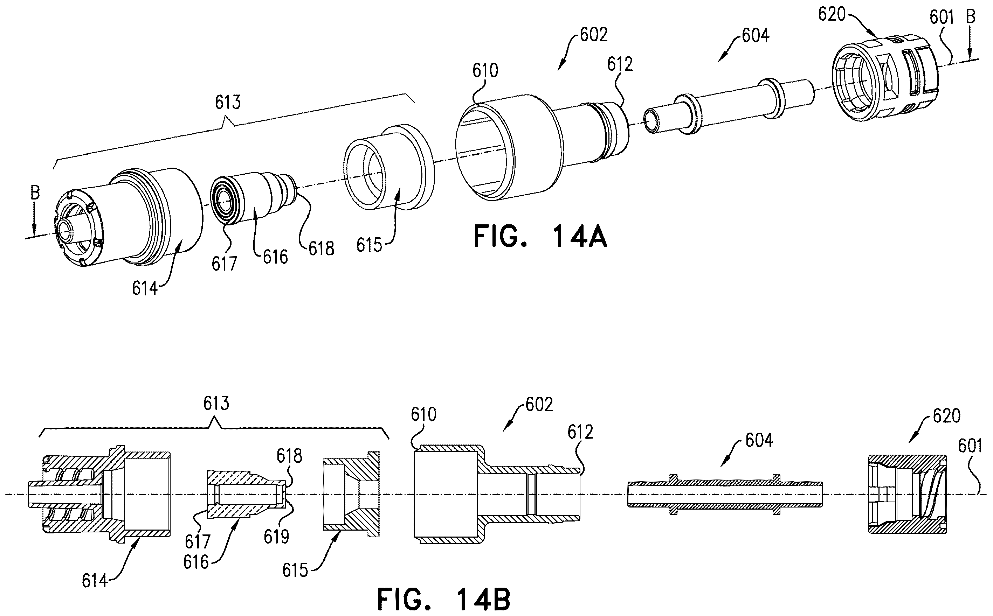

Figs. 14A and 14B, which are respectively a simplified exploded illustration and a sectional exploded illustration of the breakaway intra-medical tubing connector assembly of Fig. 13, section being taken along lines B - B in Fig. 14A.

A breakaway intra-medical tubing connector assembly 600 arranged along a longitudinal axis 601 is seen in Figs. 13 - 14B. The breakaway intra-medical tubing connector assembly 600 preferably includes a hollow connector body 602 and a generally cylindrical hollow connector element 604, which is adapted to be partially inserted through the inner volume of the connector body 602. The connector body 602 and the connector element 604 are mutually arranged along the longitudinal axis 601. The connector element 604 is adapted to be freely axially slidable within the connector body 602 along the longitudinal axis 601. It is noted that the connector element 604 is preferably identical to connector element 104, which is described in detail with reference to Figs. 4A - 4D.

It is a particular feature of an embodiment of the present invention, as seen in Figs. 14A & 14B, that the connector body 602 has a first open end 610 and a second open end 612. A first luer-actuated valve assembly 613 is integrally made or fixedly attached to the first end 610 of the connector body 602, such as by means of heat welding. The first luer-actuated valve assembly 613 includes a male luer portion 614, a cover element 615 and a compressible sealing element 616 snugly fitted therebetween. The sealing element 616 has an open first end 617, a closed second end 618 and a selectably openable slit 619 formed through the second end 618 of the sealing element 616. A snap-fit fitting 620 is disconnectably connected to the second side 612 of the connector body 602 in a tensile force responsive disconnectable snap fit connection manner. It is noted that the snap-fit fitting 620 is preferably identical to snap-fit fitting 120, which is described in detail with reference to Figs. 5A - 5E.

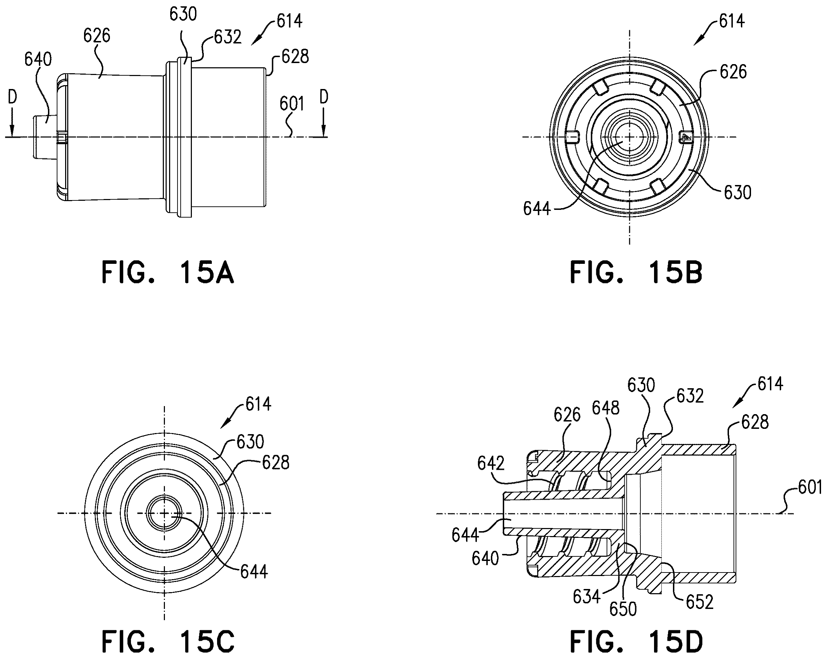

Reference is now made to Figs. 15A - 15D, which are simplified respective side view, top view, bottom view and a sectional illustration taken along lines D - D in Fig. 15A of the male luer portion 614 of the first luer-actuated valve assembly 613, forming part of the breakaway intra-medical tubing connector assembly 600 of Figs. 13 - 14B.

It is seen in Figs. 15A - 15D that the male luer portion 614 is an integrally made element arranged along longitudinal axis 601.

The male luer portion 614 of the first luer-actuated valve assembly 613 has a generally cylindrical first end 626, a generally cylindrical second end 628 and a central flange 630 disposed therebetween. An annular shoulder 632 is defined by the central flange 630.

An annular flange 634 is formed within the first end 626 and extends generally transversely with respect to longitudinal axis 601. A male luer 640 extends axially longitudinally from annular flange 634 and away from the central flange 630. An internally threaded portion 642 is defined on the inner diameter of the first end 626. The male luer 640 defines an inner volume 644.

Annular flange 634 defines a first shoulder 648 facing the internally threaded portion 642 and a second shoulder 650 facing in an opposite direction.

It is noted that instead of the male luer portion 614, a female luer portion or tube connection element may be utilized.

A radially inwardly directed shoulder 652 is formed within the second end 628, which extends generally transversely with respect to longitudinal axis 601.

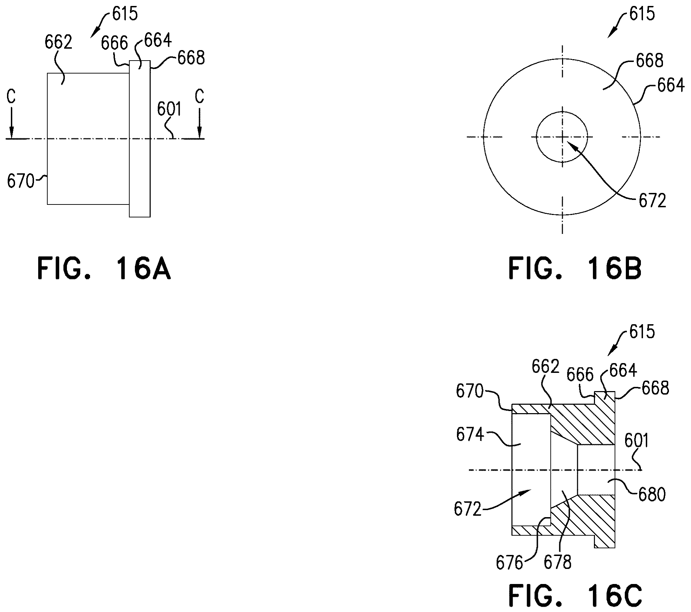

Reference is now made to Figs. 16A - 16C, which are simplified respective side view, top view and a sectional illustration taken along lines C - C in Fig. 16A of the cover element 615 of the first luer-actuated valve assembly 613, forming part of the breakaway intra-medical tubing connector assembly 600 of Figs. 13 - 14B.

It is seen in Figs. 16A - 16C that a cover element 660 is an integrally made element arranged along longitudinal axis 601.

The cover element 615 has a generally cylindrical portion 662 and an annular flange 664 formed at one end thereof. The annular flange 664 has a forwardly facing shoulder 666 and a forwardly facing end surface 668. The cylindrical portion 662 extends from the annular flange 664 to an end surface 670.

A thoroughgoing bore 672 extends longitudinally through the cover element 660. The thoroughgoing bore 672 has several bore portions, a first bore portion 674 extending longitudinally from end surface 670 to an annular shoulder 676 and having a first diameter. A tapered bore portion 678 extends generally axially from annular shoulder 676 to a third bore portion 680 having a second diameter, that is generally smaller than the first diameter. The third bore portion 680 extends from the tapered bore portion 678 to the forwardly facing end surface 668.

It is noted that the cover element 615 can alternatively be integrally made with connector body 602 or replaced thereby.

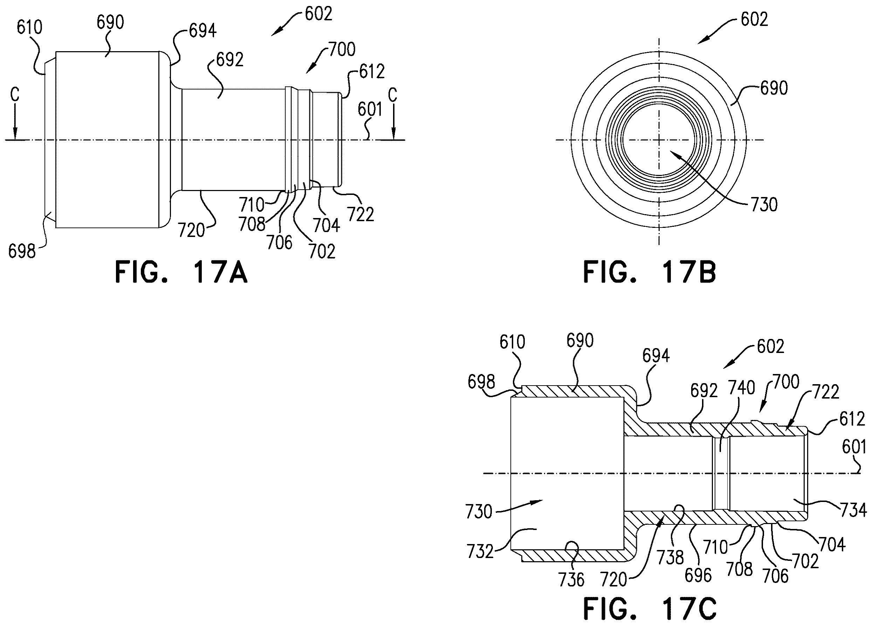

Reference is now made to Figs. 17A - 17C, which are simplified respective side view, top view and a sectional illustration taken along lines C - C in Fig. 17A of the connector body 602, forming part of the breakaway intra-medical tubing connector assembly 600 of Figs. 13 - 14B.

The connector body 602 is an integrally made hollow element arranged along longitudinal axis 601, and preferably made of plastic, such as Polypropylene, Polycarbonate or Polyethylene. The connector body 602 has first open end 610 and second open end 612, as mentioned hereinabove. The connector body 602 has a first relatively wide portion 690 and a second relatively narrow portion 692 extending therefrom and defining an annular shoulder 694 therebetween, generally located between the first end 610 and the second end 612. The narrow portion 692 has an outer surface 696.

An annular protrusion 698 extends longitudinally outwardly and slightly away from first end 610 of the connector body 602 and serves for attachment of the connector body 602 with the male luer portion 614, such as by heat welding, ultrasonic welding or adhesive connection.

A snap connection 700 is disposed generally adjacent the second end 612 of the connector body 602. The snap connection 700 includes a first annular protrusion 702 extending generally radially outwardly from the outer surface 696 of the narrow portion 692 of the connector body 602 and having a first diameter. The first annular protrusion 702 defines a rearwardly facing shoulder 704 at one side thereof, which faces the second end 612 and lies in a plane that is generally perpendicular to the longitudinal axis 601. At another side, the first annular protrusion 702 continues to a forwardly tapered annular protrusion 706, which in turn continues to a second annular protrusion 708, which extends generally radially outwardly from the outer surface 696 of the narrow portion 692 of the connector body 602 and having a second diameter, generally greater than the first diameter. The second annular protrusion 708 defines a forwardly facing shoulder 710, which faces annular shoulder 694 and lies in a plane that is generally perpendicular to the longitudinal axis 601.

A first annular portion 720 is formed between the snap connection 700 and the annular shoulder 694, and a second annular portion 722 is formed between the snap connection 700 and the second end 612 of the connector body 602. Both the first annular portion 720 and the second annular portion 722 extend generally axially along longitudinal axis 601.

A through bore 730 extends axially longitudinally along axis 601, through connector body 602. The through bore 730 has a first bore portion 732 of a first diameter extending along wide portion 690 and a second bore portion 734 of a second diameter, relatively smaller than the first diameter, which extends along the narrow portion 692. The first bore portion 732 defines an inner surface 736 and the second bore portion 734 defines an inner surface 738. An annular protrusion 740 is generally disposed within second bore portion 734 at a central location of the narrow portion 692 and extends slightly radially inwardly.

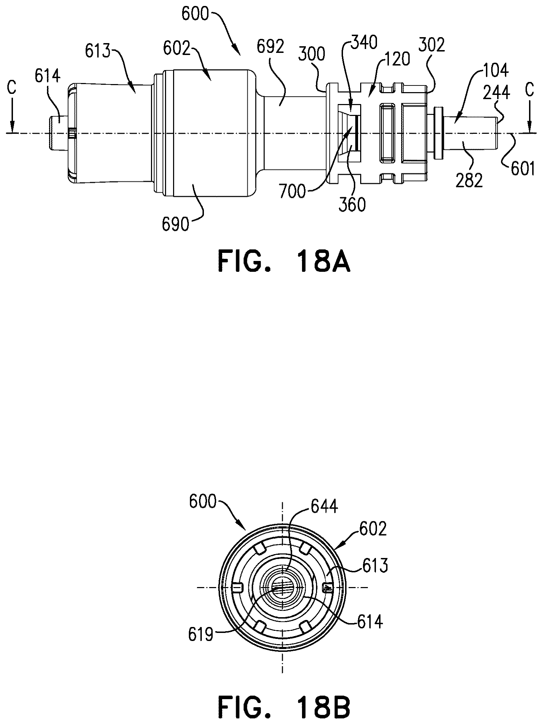

Reference is now made to Figs. 18A - 18C, which are simplified respective side view, top view and a sectional illustration taken along lines C - C in Fig. 18A of the assembled breakaway intra-medical tubing connector assembly 600 of Figs. 13 - 14B.

It is seen in Figs. 18A - 18C that the connector element 104 is at least partially inserted into the through bore 730 of the connector body 602, such that the connector element 104 and the connector body 602 extend along mutual longitudinal axis 601.

It is a particular feature of an embodiment of the present invention that the connector element 104 is freely slidable with respect to the connector body 602 along longitudinal axis 601 up to engagement of either the first or the second annular protrusion 270 or 272 of the connector element 104 with the annular protrusion 740 of the connector body 602.

The snap-fit fitting 120 is mounted onto the second end 612 of the connector body 602. The second annular portion 722 of connector body 602 is disposed in a sealing engagement with the respective annular bore portion 320 of the snap-fit fittings 120.

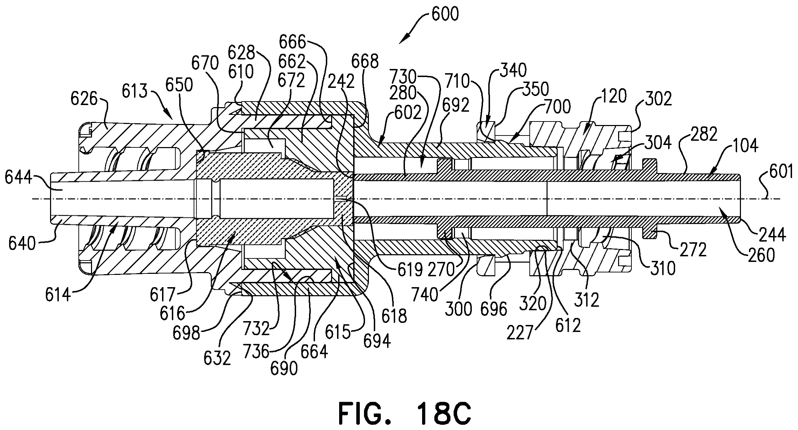

It is a further particular feature of an embodiment of the present invention and is particularly seen in Fig. 18C that the snap-fit fitting 120 is disconnectably snap-fittingly connected to the snap connection 700 of the connector body 602 and can be disconnected from the connector body 602 in response to tensile force exerted on the snap-fit fitting 120 along longitudinal axis 601. Specifically, it is seen in Fig. 18C that snap connection 340 of the snap-fit fitting 120 is disconnectably connected with the snap connection 700 of the connector body 602, such that shoulder 350 of the snap connection 340 engages the forwardly facing shoulder 710 of the snap connection 700.

It is seen in Fig. 18C that the second open end 612 of the connector body 602 is disposed adjacent the annular protrusion 312 of the snap-fit fitting 120.

It is noted that the outer surface 696 of the connector body 602 generally corresponds to the geometry of the annular bore portion 320 and to the snap-fit bore portion 330 of the thoroughgoing bore 304 of the snap-fit fitting 120.

It is a particular feature of an embodiment of the present invention that the first luer-actuated valve assembly 613 is fixedly and irremovably attached to the connector body 602. Specifically, it is seen that the compressible sealing element 616 is snugly fitted between the male luer portion 614 and the cover element 615. The sealing element 616 is seated such that the open end 617 thereof engages the second shoulder 650 of the male luer portion 614 and the closed second end 618 is generally aligned with end surface 668 of the cover element 615. The sealing element 616 extends at least through the thoroughgoing bore 672 of the cover element 615 and partially through the inner volume of the cylindrical second end 628 of the male luer portion 614.

The cover element 615 is mounted onto the male luer portion 614 such that forwardly facing surface 666 of the annular flange 664 of the cover element 615 engages an edge of second end 628 of the male luer portion 614.

The luer-actuated valve assembly 613 is partially inserted into first bore portion 732 of the connector body 602 and fixedly attached thereto, such that second end 628 of the male luer portion 614 engages the inner surface 736 of the wide portion 690 of the connector body 602, annular shoulder 632 of the male luer portion 614 engages the annular protrusion 698 of the connector body 602 and end surface 668 of the cover element 615 engages the annular shoulder 694 of the connector body 602.

Fluid flow passage from the thoroughgoing bore 260 of the connector element 104 to the inner volume 644 of the male luer portion 614 or vice-versa is prevented in this operative orientation since the slit 619 of the sealing element 616 is closed.

It is noted that in this assembled operative orientation of the breakaway intra-medical tubing connector assembly 600, the connector element 104 is inserted through the bore 730 of the connector body 602 and at least partially through the thoroughgoing bore 304 of the snap-fit fitting 120, such that the first annular protrusion 270 of the connector element 104 is disposed within bore 730 of the connector body 602 and the second annular protrusion 272 of the connector element 104 is generally disposed outside the snap-fit fitting 120.

It is particularly seen in Figs. 18A - 18C that the first tubular portion 280 of the connector element 104 is partially surrounded by the narrow portion 692 of the connector body 602 and the second tubular portion 282 of the connector element 104 at least partially protrudes outwardly from the second open end 302 of the second snap-fit fitting 120.

It is a particular feature of an embodiment of the present invention that the connector element 104 is axially slidable with respect to both the connector body 602 and the snap-fit fitting 120.

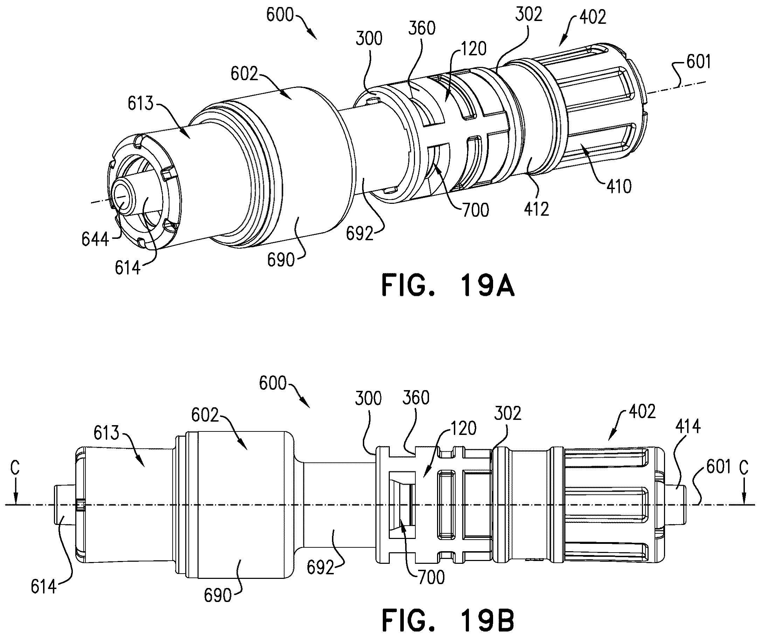

Reference is now made to Figs. 19A - 19C, which are simplified respective perspective view, side view and a sectional illustration taken along lines C - C in Fig. 19B of the breakaway intra-medical tubing connector assembly 600 of Figs. 18A - 18C, shown when a second luer-actuated valve assembly is connected to the breakaway intra-medical tubing connector assembly 600.

It is seen in Figs. 19A - 19C that a second luer-actuated valve assembly, which is preferably identical to the the second luer-actuated valve assembly 402 described with reference to Figs. 7A - 7C is now threadably connected to the breakaway intra-medical tubing connector assembly 600. The second luer-actuated valve assembly 402 is threadably connected to the snap-fit fitting 120 by means of engagement between the externally threaded portion 416 of the female luer portion 412 and between the internally threaded portion 310 of the snap-fit fitting 120.

It is a particular feature of an embodiment of the present invention that upon connection of the second luer-actuated valve assembly 402 to the breakaway intra-medical tubing connector assembly 600, a fluid flow passage is established between two medical tubes, each of which is adapted to be connected to the respective male luer portion 614 and 414 of the first and second luer-actuated valve assemblies 613 and 402.

It is a further particular feature of an embodiment of the present invention that the inner diameter of the fluid flow passage provided between the first and second luer-actuated valve assemblies 613 and 402 is substantially the same as the diameter of the thoroughgoing bore 260 of the connector element 104.

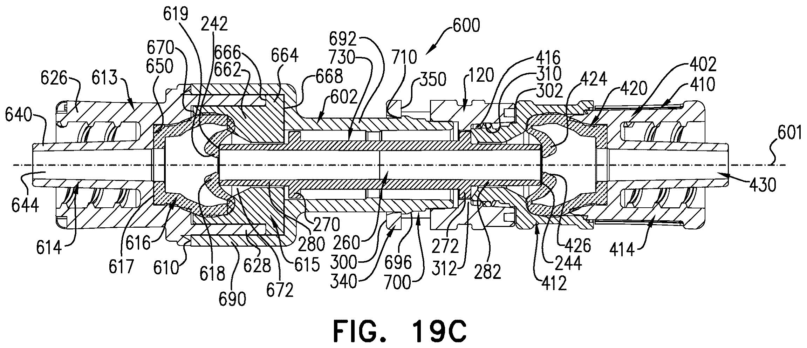

Specifically, it is seen in Fig. 19C that upon threaded engagement of the second luer-actuated valve assembly 402 with the snap-fit fitting 120, the first tubular portion 280 of the connector element 104 compresses the sealing element 616 of the first luer-actuated valve assembly 613 and thus urges opening of selectively openable slit 619 thereof. Similarly, upon threaded engagement of the second luer-actuated valve assembly 402, the second tubular portion 282 of the connector element 104 compresses the sealing element 420 of the second luer-actuated valve assembly 402 and thus urges opening of selectively openable slit 426 thereof. The fluid flow passage is established from a first medical tube that is adapted to be connected to the male luer portion 614 of the first luer-actuated valve assembly 613, through the inner volume 644 thereof, via slit 619 of the sealing element 616 and through the thoroughgoing bore 260 of the connector element 104, further via slit 426 of the sealing element 420 of the second luer-actuated valve assembly 402, through fluid flow passage 430 thereof and finally into a second medical tube that is adapted to be connected to the male luer portion 414 of the second luer-actuated valve assembly 402, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 100 constructed and operative in accordance with an embodiment of the present invention.

It is noted that compression forces exerted on both luer-actuated valve assemblies 613 and 402 by the connector element 104 are enabled by the fact that the second luer-actuated valve assembly 402 is held in place relative to the breakaway intra-medical tubing connector assembly 100 due to snap-fit connection between snap-fit fitting 120 and the connector body 602.

The breakaway intra-medical tubing connector assembly 600 is preferably disposable in case the snap connections 700 and 330 provided between the snap-fit fitting 120 and the connector body 602 are deformable, such that additional engagement of the snap-fit fitting 120 with the connector body 602 is not intended after first use.

It is noted that alternatively, any other type of luer-actuated valve assembly can be used in conjunction with the breakaway intra-medical tubing connector assembly 600, constructed and operative in accordance with an embodiment of the present invention.

It is noted that the second luer-actuated valve assembly 402 may be freely rotated about axis 101 along with the respective snap-fit fitting 120 while being engaged with the intra-medical tubing connector assembly 600, without causing disengagement of the snap-fit fitting 120 with luer-actuated valve assembly 402 therefrom.

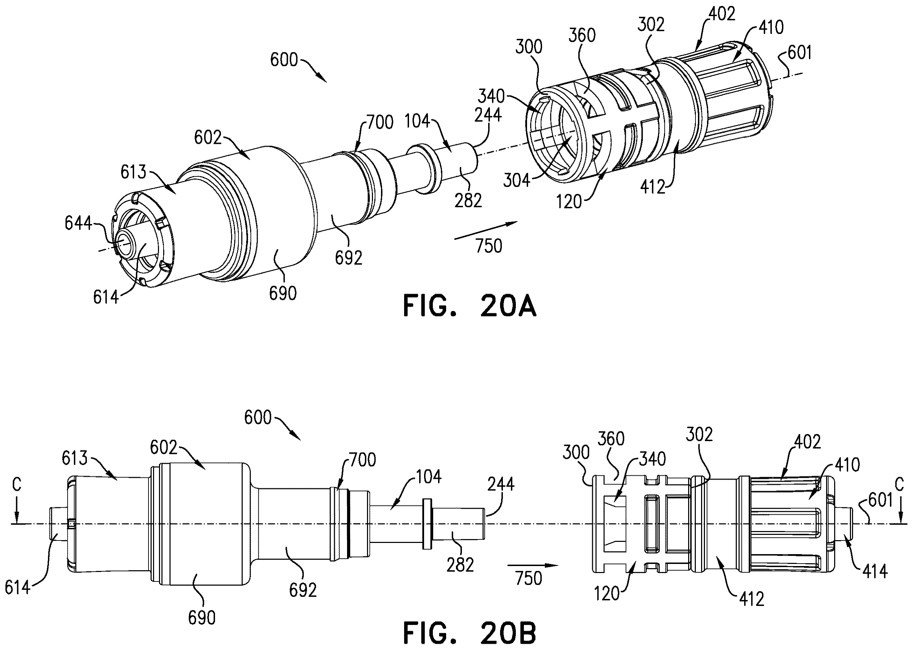

Reference is now made to Figs. 20A - 20C, which are simplified respective perspective view, side view and a sectional illustration taken along lines C - C in Fig. 20B of the breakaway intra-medical tubing connector assembly 600 of Figs. 18A - 18C, shown when the second luer-actuated valve assembly 402 is disconnected from the breakaway intra-medical tubing connector assembly 600.

It is seen in Figs. 20A - 20C that a tensile force in a direction indicated by arrow 750 is applied on the snap-fit fitting 120, the second luer-actuated valve assembly 402 or the second medical tube and thus causes disconnection between the snap-fit fitting 120 and the connector body 602. It is seen that the second luer-actuated valve assembly 402 is threadably connected to the snap-fit fitting 120, thus cannot be disconnected therefrom upon application of tensile force, however the snap fit connection provided between the snap-fit fitting 120 and between the connector body 602 can be broken upon application of sufficient tensile force. According to an embodiment of the present invention, tensile force in the range of 0.5kgf - 3kgf is required in order to break the snap-fit connection between the snap-fit fitting 120 and the connector body 602.

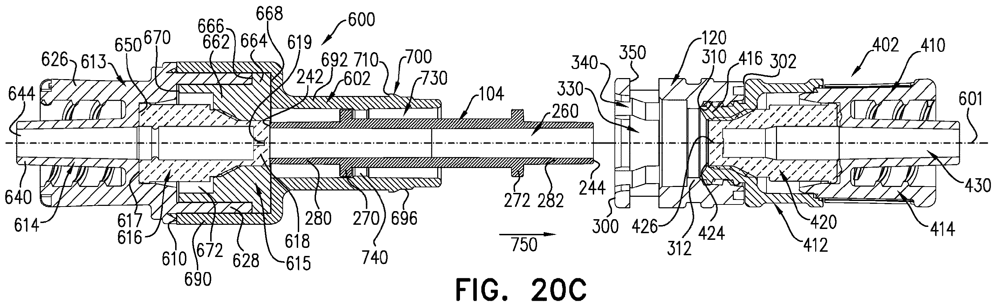

It is specifically seen in Fig. 20C that upon application of tensile force upon the snap fit-fitting 120 along longitudinal axis 601 in the direction of arrow 750, the snap connection 340 of the snap-fit fitting 120 is disengaged from the snap connection 700 of the connector body 602, such that shoulder 350 of the snap connection 340 disengages shoulder 710 of the snap connection 700 due to the relative resiliency of the snap-fit fitting 120 provided by the material it is formed of as well as by the longitudinal grooves 352 and by openings 360, as described in detail hereinabove.

It is also seen in Fig. 20C that upon disconnection of the second luer-actuated valve assembly 402, the connector element 104 is urged to be displaced axially along longitudinal axis 601 in the direction of arrow 750 and out of the first luer-actuated valve assembly 613, due to the biasing force of the sealing element 616 of the first luer-actuated valve assembly 613 that is applied upon the connector element 104. The connector element 104 is displaced axially along axis 601 up to engagement with protrusion 740, which prevents the connector element from falling out of the connector body 602.

It is a particular feature of an embodiment of the present invention that upon disconnection of the second luer-actuated valve assembly 402 from the breakaway intra-medical tubing connector assembly 600 and according to this particular embodiment upon disconnection of the snap-fit fitting 120 along with the second luer-actuated valve assembly 402 from the connector body 602, both of the luer-actuated valve assemblies 613 and 402 are automatically bidirectionally closed, thus preventing fluid flow passage out of each of the two medical tubes.

In this operative orientation, the first tubular portion 280 of the connector element 104 is fully surrounded by the narrow portion 692 of the connector body 602, and the second tubular portion 282 of the connector element 104 protrudes outwardly from the connector body 602.

It is noted that tensile force exerted on the medical set such as IV line may be unintentional, but due to the presence of the breakaway intra-medical tubing connector assembly 600, the catheter or other medical tube associated with one of the luer-actuated valve assemblies 613 and 402 remains in its position within the treatment site and the luer-actuated valve assemblies 613 and 402 are safely sealed once one of the luer-actuated valve assemblies 613 and 402 is disconnected from the breakaway intra-medical tubing connector assembly 600. Therefore, no fluid can unintentionally flow out of the IV line and risk of contamination of the treatment site is prevented due to sealing of the luer-actuated valve assemblies 613 and 402.

It is noted that alternatively disconnection of the second luer-actuated valve assembly 402 from the breakaway intra-medical tubing connector assembly 600 may be intentional in order to provide the physician with access point to the IV line. Once the second luer-actuated valve assembly 402 is exposed, it may be used for administering medicament into the treatment site within the patient body or alternatively for withdrawing a blood sample from the patient's body.

It is a particular feature of an embodiment of the present invention that the breakaway intra-medical tubing connector assembly 600 associated with medical connectors, such as luer-actuated valve assemblies 613 and 402 enables both safe unintentional disconnection of one of the luer-actuated valve assemblies and provides access point to the IV line through the exposed sealing element 420 when the second luer-actuated valve assembly 402 is intentionally disconnected.

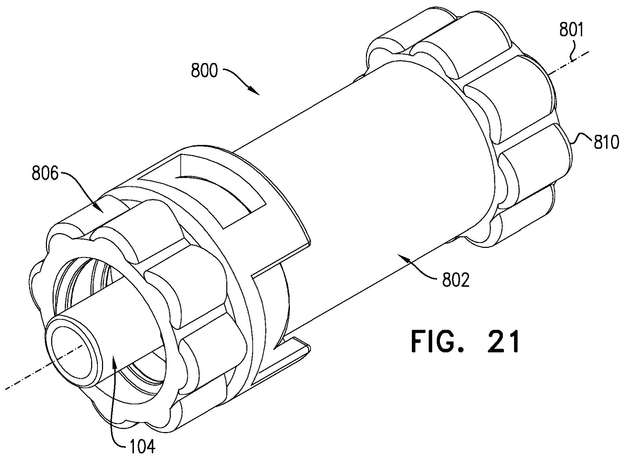

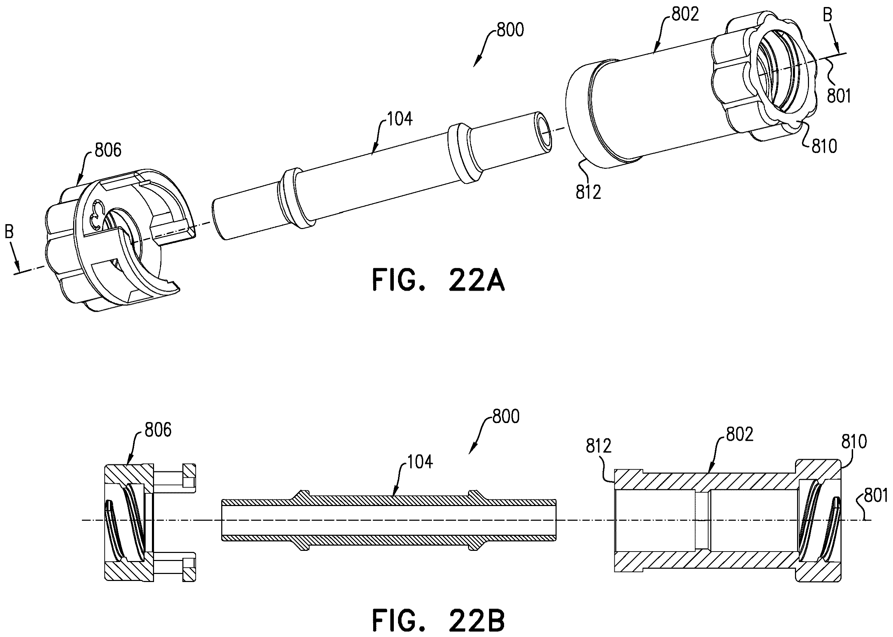

Reference is now made to Fig. 21, which is a simplified pictorial illustration of an assembled breakaway intra-medical tubing connector assembly, constructed and operative in accordance with still another embodiment of the present invention. Reference is additionally made to Figs. 22A and 22B, which are respectively a simplified exploded illustration and a sectional exploded illustration of the breakaway intra-medical tubing connector assembly of Fig. 21, section being taken along lines B - B in Fig. 22A.

A breakaway intra-medical tubing connector assembly 800 arranged along a longitudinal axis 801 is seen in Figs. 21 - 22B. The breakaway intra-medical tubing connector assembly 800 preferably includes a hollow connector body 802 and a generally cylindrical hollow connector element 804, which is adapted to be partially inserted through the inner volume of the connector body 802. The connector body 802 and the connector element 804 are mutually arranged along the longitudinal axis 801. The connector element 804 is adapted to be freely axially slidable within the connector body 802 along the longitudinal axis 801. It is noted that the connector element 804 is substantially similar to connector element 104, which is described in detail with reference to Figs. 4A - 4D. The breakaway intra-medical tubing connector assembly 800 preferably also includes a snap-fit fitting 806, which is adapted to be disconnectably attached to the connector body 802 in a snap-fit manner.

It is a particular feature of an embodiment of the present invention, as seen in Figs. 22A & 22B, that the connector body 802 has a first end 810 and a second end 812. The snap-fit fitting 806 is disconnectably connected to the second end 812 of the connector body 802 in a tensile force responsive disconnectable snap fit connection manner.

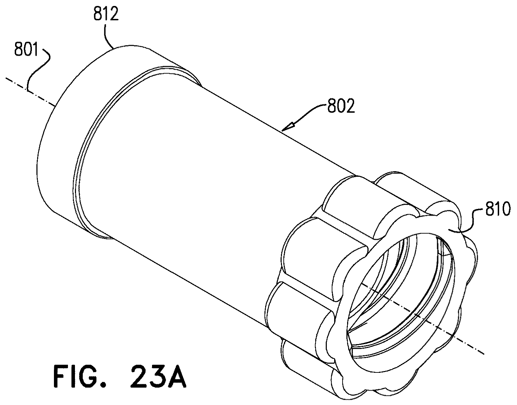

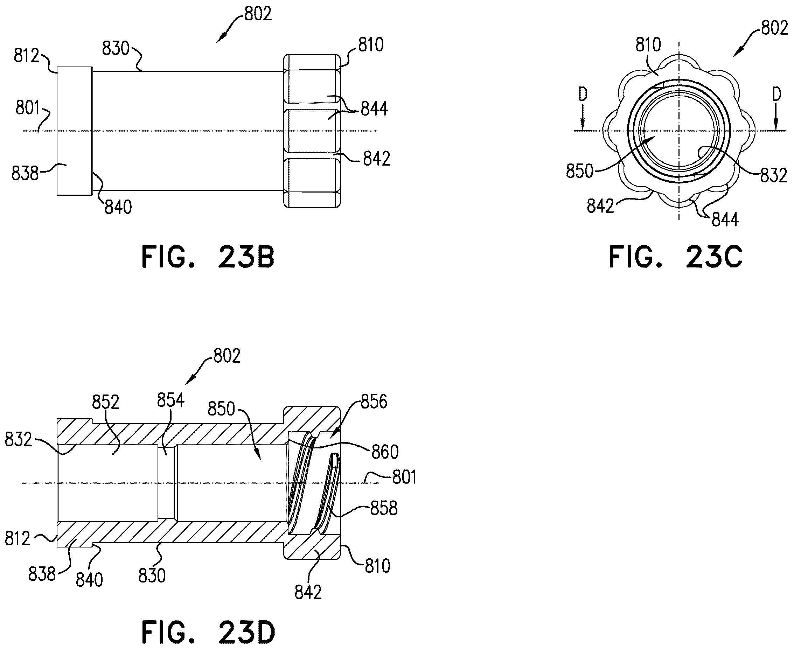

Reference is now made to Figs. 23A - 23D, which are simplified respective perspective, side view, top view and a sectional illustration taken along lines D - D in Fig. 23C of the connector body 802, forming part of the breakaway intra-medical tubing connector assembly 800 of Figs. 21 - 22B.

The connector body 802 is an integrally made cylindrical hollow element arranged along longitudinal axis 801, and preferably made of plastic, such as Polypropylene, Polycarbonate or Polyethylene. The connector body 802 has first open end 810 and second open end 812, as mentioned hereinabove. The connector body 802 has an outer surface 830 and an inner surface 832. A snap protrusion 838 is formed adjacent the second end 812 of the connector body 802 and extends radially outwardly from the outer surface 830 and forms a rearwardly facing shoulder 840 with respect to the outer surface 830 of the connector body 802. A gripping protrusion 842 is formed adjacent the first end 810 of the connector body 802. Curved gripping surface 844 is formed on the gripping protrusion 842. A through bore 850 is formed within connector body 802 and extends longitudinally therethrough along axis 801.

The through bore 850 has a first portion 852 having a first diameter and an annular protrusion 854 is generally disposed within said first portion 852 between the first and second ends 810 and 812 and generally extends slightly radially inwardly. The through bore 850 also has a second portion 856, having a second portion with a second diameter that is generally larger than the first diameter. An inwardly threaded portion 858 is formed in said second portion 856. A rearwardly facing annular shoulder 860 is formed between the first portion 852 and the second portion 856.

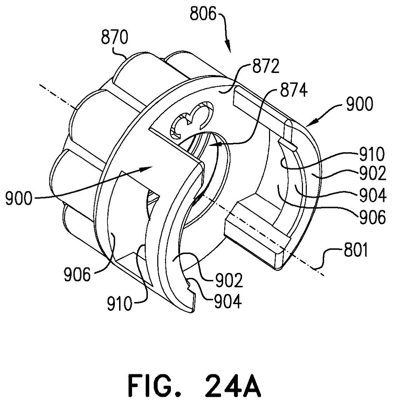

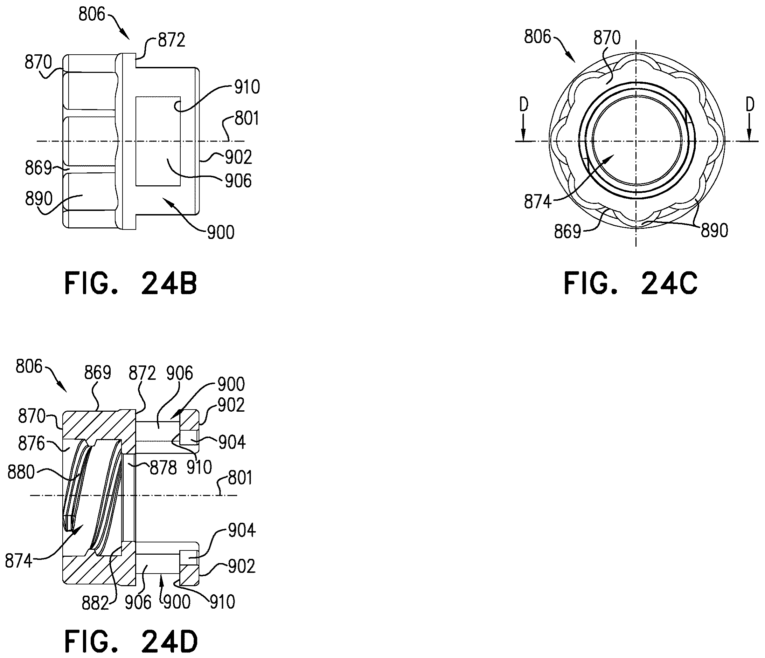

Reference is now made to Figs. 24A - 24D, which are simplified respective perspective, side view, top view and a sectional illustration taken along lines D - D in Fig. 24C of the snap-fit fitting 806, forming part of the breakaway intra-medical tubing connector assembly 800 of Figs. 21 - 22B.

The snap-fit fitting 806 is an integrally made element arranged along longitudinal axis 801, and preferably made of plastic, such as Polypropylene, Polycarbonate, Polyethylene. The snap-fit fitting 806 has a generally disc-shaped hollow element 869 having a first open end 870 and a second open end 872 and a thoroughgoing bore 874 extending along longitudinal axis 801. The thoroughgoing bore 874 has a first portion 876 having a first diameter and a second portion 878 having a second diameter, which is generally smaller than a first diameter. An inwardly threaded portion 880 is formed along the first portion 876 of the thoroughgoing bore 874 and a forwardly facing shoulder 882 is formed between the first and second portions 876 and 878. A curved gripping surface 890 is formed on the outer surface of the hollow element 869.

Typically, two rearwardly extending curved arms 900 are preferably diametrically opposed to each other and extend axially rearwardly from the circumference of the second open end 872. The curved arms 900 extend from second open end 872 to a rearwardly facing edge surface 902. A radially inwardly extending protrusion 904 is formed adjacent each one of rearwardly facing edge surfaces 902. The protrusions 904 extend axially preferably forwardly. An opening 906 is formed through each of the curved arms 900. A forwardly facing snap edge 910 is formed between each of the protrusions 904 and its respective openings 906.

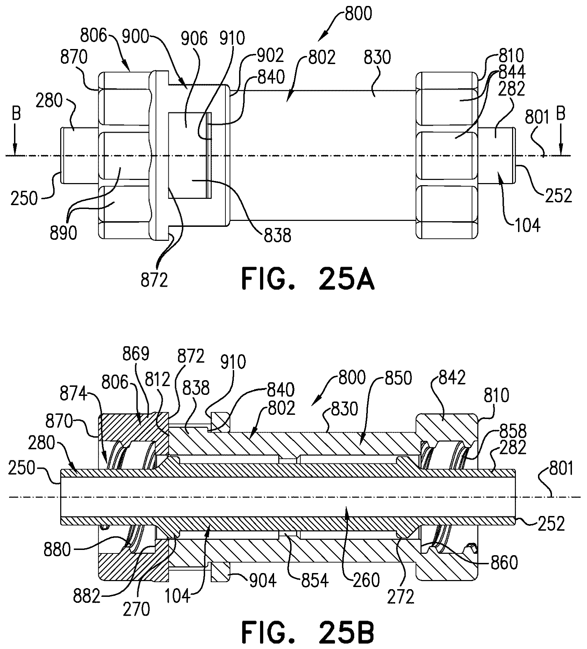

Reference is now made to Figs. 25A and 25B, which are simplified respective side view and a sectional illustration taken along lines B - B in Fig. 25A of the assembled breakaway intra-medical tubing connector assembly 800 of Figs. 21 - 22B.

It is seen in Figs. 25A and 25B that the connector element 104 is at least partially inserted into the through bore 850 of the connector body 802 and the through bore 874 of the snap-fit fitting 806, such that the connector element 104 and the connector body 802 extend along mutual longitudinal axis 801.

It is a particular feature of an embodiment of the present invention that the connector element 104 is freely slidable with respect to the connector body 802 along longitudinal axis 801 up to engagement of either the first or the second annular protrusion 270 or 272 of the connector element 104 with the annular protrusion 854 of the connector body 802.

The snap-fit fitting 806 is mounted onto the second end 812 of the connector body 802, such that the forwardly facing snap edge 910 of protrusion 904 of the snap-fit fitting 806 engages the rearwardly facing surface 840 of the connector body 802 in a snap-fit manner.

It is a further particular feature of an embodiment of the present invention and is particularly seen in Fig. 25B that the snap-fit fitting 806 is disconnectably snap-fittingly connected to the snap protrusion 838 of the connector body 802. The snap-fit fitting 806 can be disconnected from the connector body 802 in response to tensile force exerted thereon along longitudinal axis 801.

It is seen in Fig 25B that the second open end 812 of the connector body 802 is disposed adjacent the second open end 872 of the snap-fit fitting 806.

It is noted that in this assembled operative orientation of the breakaway intra-medical tubing connector assembly 800, the first annular protrusion 270 of the connector element 104 is disposed adjacent the second open end 872 of the snap-fit fitting 806 and the second annular protrusion 272 of the connector element 104 is disposed adjacent annular shoulder 860 of the connector body 802.

It is particularly seen in Figs. 25A and 25B that the first tubular portion 280 of the connector element 104 is partially surrounded by internally threaded portion 880 of the snap-fit fitting 806 and partially protrudes outwardly from the first open end 870 of the first snap-fit fitting 806. Additionally, the second tubular portion 282 of the connector element 104 is partially surrounded by internally threaded portion 858 of the connector body 802 and partially protrudes outwardly from the first open end 810 of the connector body 802.

It is a particular feature of an embodiment of the present invention that the connector element 104 is axially slidable with respect to both the connector body 802 and the snap-fit fitting 806.

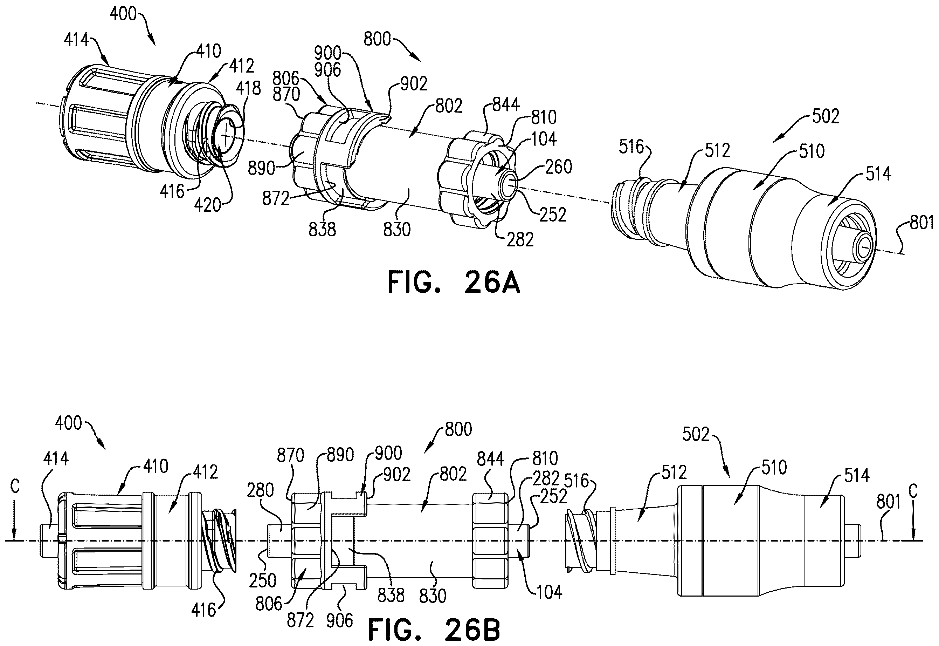

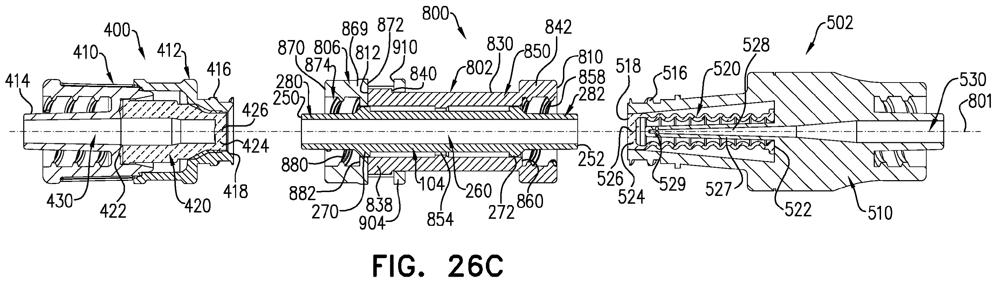

Reference is now made to Figs. 26A - 26C, which are simplified respective perspective view, side view and a sectional illustration taken along lines C - C in Fig. 26B of the breakaway intra-medical tubing connector assembly 800 of Figs. 25A and 25B, shown in a first operative orientation, just prior to connection thereof with a first and a second luer-actuated valve assemblies constructed and operative in accordance with an embodiment of the present invention.

It is seen in Figs. 26A - 26C that a first luer-actuated valve assembly, preferably identical to luer-actuated valve assembly 400 as described in detail with reference to Figs. 7A -7C and a second luer-actuated valve assembly, preferably identical to luer-actuated valve assembly 502 as described in detail with reference to Figs. 10A - 10C are about to be threadably connected to the breakaway intra-medical tubing connector assembly 800. The first luer-actuated valve assembly 400 is adapted to be threadably connected to the inwardly threaded portion 880 of snap-fit fitting 806 and the second luer-actuated valve assembly 502 is adapted to be threadably connected to the internally threaded portion 858 of the connector body 802 of the breakaway intra-medical tubing connector assembly 800.

It is noted that alternatively, any other type of luer-actuated valve assembly can be used in conjunction with the breakaway intra-medical tubing connector assembly 800, constructed and operative in accordance with an embodiment of the present invention.

It is specifically seen in Figs. 26A - 26C that the luer-actuated valve assemblies 400 and 502 are not yet mounted onto the breakaway intra-medical tubing connector assembly 800, thus both luer-actuated valve assemblies 400 and 502 are sealingly closed in this operative orientation.

It is further noted that the respective male luer portions 414 and 514 of the luer-actuated valve assemblies 400 and 502 are adapted to be connected to a medical tubing, such as for example, an IV line or a catheter.

Spatial relationships between the various components of the breakaway intra-medical tubing connector assembly 800 preferably remains the same as described with reference to Figs. 25A and 25B.

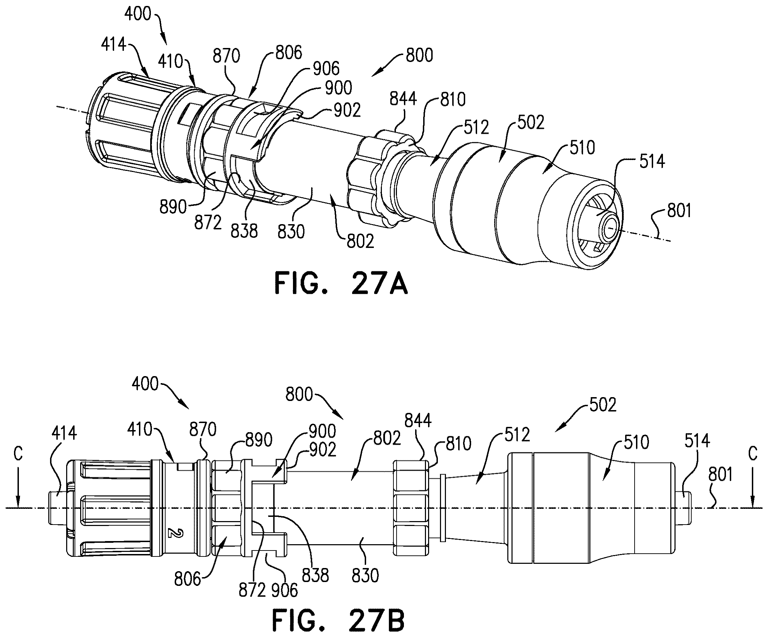

Reference is now made to Figs. 27A - 27C, which are simplified respective perspective view, side view and a sectional illustration taken along lines C - C in Fig. 27B of the breakaway intra-medical tubing connector assembly 800 of Figs. 25A and 25B, shown in a second operative orientation, when the first and the second luer-actuated valve assemblies 400 and 502 of Figs. 26A - 26C are connected to the breakaway intra-medical tubing connector assembly 800.

It is seen in Figs. 27A - 27C that both the first luer-actuated valve assembly 400 and the second luer-actuated valve assembly 502 are threadably connected to the breakaway intra-medical tubing connector assembly 800. The first luer-actuated valve assembly 400 is threadably connected to the snap-fit fitting 806 by means of engagement between the externally threaded portion 416 of the female luer portion 412 of the first luer-actuated valve assembly 400 and between inwardly threaded portion 880 of snap-fit fitting 806. The second luer-actuated valve assembly 502 is threadably connected to the connector body 802 by means of engagement between externally threaded portion 516 of the female luer portion 512 of the second luer-actuated valve assembly 502 and between the internally threaded portion 858 of the connector body 802 of the breakaway intra-medical tubing connector assembly 800.

It is a particular feature of an embodiment of the present invention that upon connection of both the first and the second luer-actuated valve assemblies 400 and 502 to the breakaway intra-medical tubing connector assembly 800, a fluid flow passage is established between two medical tubes, each of which is adapted to be connected to the respective male luer portion 414 and 514 of the first and second luer-actuated valve assemblies 400 and 502.

It is a further particular feature of an embodiment of the present invention that the inner diameter of the fluid flow passage provided between the first and second luer-actuated valve assemblies 400 and 502 is substantially the same as the diameter of the thoroughgoing bore 260 of the connector element 104.

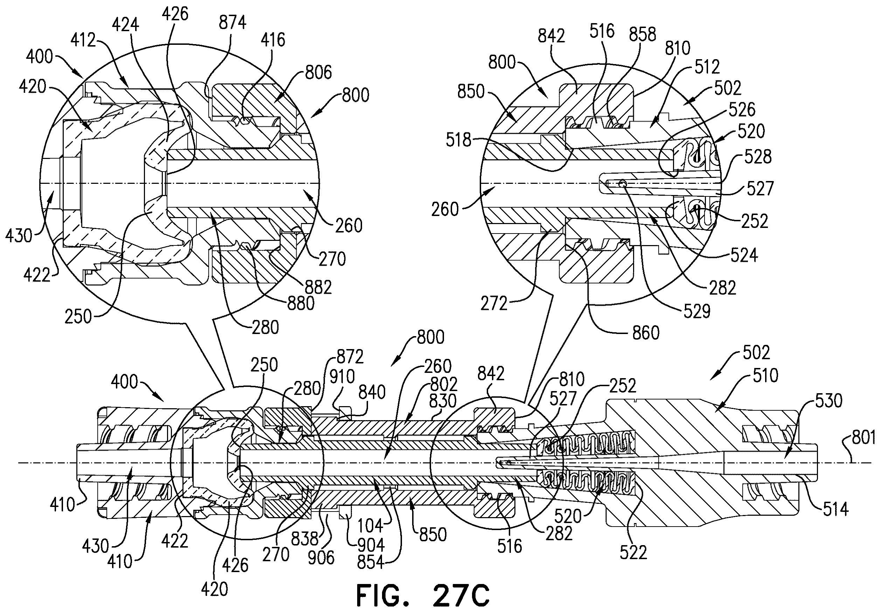

Specifically, it is seen in Fig. 27C that upon threaded engagement of both the first and second luer-actuated valve assemblies 400 and 502, the first tubular portion 280 of the connector element 104 compresses the sealing element 420 of the first luer-actuated valve assembly 400 and thus urges opening of selectively openable slit 426 thereof. Similarly, upon threaded engagement of both the first and second luer-actuated valve assemblies 400 and 502, the second tubular portion 282 of the connector element 104 compresses the sealing element 520 of the second luer-actuated valve assembly 502 and thus urges opening of selectively openable slit 526 thereof. The fluid flow passage is established from a first medical tube that is adapted to be connected to the male luer portion 414 of the first luer-actuated valve assembly 400, through the fluid flow passage 430 thereof, via slit 426 of the sealing element 420 and through the thoroughgoing bore 260 of the connector element 104, further via opening 529 of the sealing element 520 of the second luer-actuated valve assembly 502, through fluid flow passage 530 thereof and finally into a second medical tube that is adapted to be connected to the male luer portion 514 of the second luer-actuated valve assembly 502, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 800 constructed and operative in accordance with an embodiment of the present invention.

It is noted that compression forces exerted on both luer-actuated valve assemblies 400 and 502 by the connector element 104 are enabled by the fact that the first luer-actuated valve assembly 400 is held in place relative to the breakaway intra-medical tubing connector assembly 800 due to snap-fit connection between snap-fit fitting 806 and the connector body 802 and the second luer-actuated valve assembly 502 is held in place relative to the breakaway intra-medical tubing connector assembly 800 due to threadable connection thereof with the connector body 802.

The breakaway intra-medical tubing connector assembly 800 is preferably disposable in case the snap protrusion 838 provided between the snap-fit fitting 806 and the connector body 802 is deformable, such that additional engagement of the snap-fit fitting 806 with the connector body 802 is not intended after first use.

It is noted that alternatively, any other type of luer-actuated valve assembly can be used in conjunction with the breakaway intra-medical tubing connector assembly 800, constructed and operative in accordance with an embodiment of the present invention.

It is noted that the first luer-actuated valve assembly 400 may be freely rotated about axis 801 along with the respective snap-fit fitting 806 while being engaged with the intra-medical tubing connector assembly 800, without causing disengagement of the snap-fit fitting 806 with luer-actuated valve assembly 400 therefrom.

It is further noted that upon connection of one of the luer actuated valve assemblies 400 and 502, the connector element 104 is axially slidably moveable and thus provides for both of the luer-actuated valve assemblies 400 and 502 to remain sealed up until luer-actuated valve assembly 502 is connected to the connector body 802 and the luer actuated valve assembly 400 is connected to the snap-fit fitting 806, which provides for opening of the sealing members 420 and 520 of both luer-actuated valve assemblies 400 and 502 respectively.

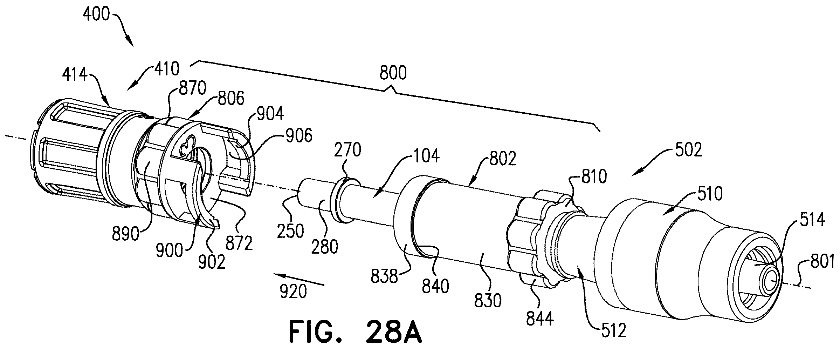

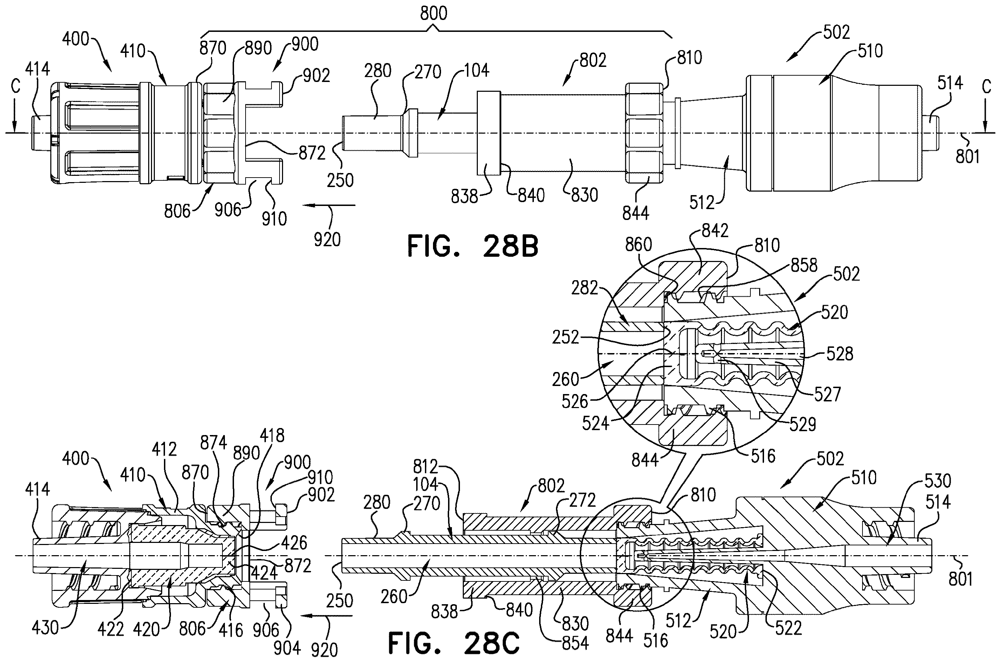

Reference is now made to Figs. 28A - 28C, which are simplified respective perspective view, side view and a sectional illustration taken along lines C - C in Fig. 28B of the breakaway intra-medical tubing connector assembly 800 of Figs. 25A and 25B, shown in a third operative orientation, when one of the luer-actuated valve assemblies 400 of Figs. 26A - 26C is disconnected from the breakaway intra-medical tubing connector assembly 800.

It is seen in Figs. 28A - 28C that a tensile force in a direction indicated by arrow 920 is applied on either the snap-fit fitting 806, the first luer-actuated valve assembly 400 or the first medical tube and thus causes disconnection between the snap-fit fitting 806 and the connector body 802. It is seen that the second luer-actuated valve assembly 502 is threadably connected to the connector body 802, thus cannot be disconnected therefrom upon application of tensile force. According to an embodiment of the present invention, tensile force in the range of 0.5kgf - 3kgf is required in order to break the snap-fit connection between the snap-fit fitting 806 and the connector body 802.

It is specifically seen in Fig. 28C that upon application of tensile force upon the snap fit-fitting 806 along longitudinal axis 801 in the direction of arrow 920, the protrusion 904 of the snap-fit fitting 806 is disengaged from the snap protrusion 838 of the connector body 802, such that forwardly facing snap edge 910 of the snap-fit fitting 806 disengages from rearwardly facing shoulder 840 of the connector body 802 due to the relative resiliency of the snap-fit fitting 806 provided by outward deflection of the curved arms 900.

It is also seen in Fig. 28C that upon disconnection of the first luer-actuated valve assembly 400, the connector element 104 is urged to be displaced axially along longitudinal axis 801 in the direction of arrow 920 and out of the second luer-actuated valve assembly 502, due to the biasing force of the sealing element 520 of the second luer-actuated valve assembly 502 that is applied upon the connector element 104.

It is a particular feature of an embodiment of the present invention that upon disconnection of the first luer-actuated valve assembly 400 from the breakaway intra-medical tubing connector assembly 800 and according to this particular embodiment upon disconnection of the snap-fit fitting 806 along with the first luer-actuated valve assembly 400 from the connector body 802, both of the luer-actuated valve assemblies 400 and 502 are automatically bidirectionally closed, thus preventing fluid flow passage from the two medical tubes. Upon discarding of the breakaway intra-medical tubing connector assembly 800 along with the snap-fit fitting 806, two sealed and swabbale luer-actuated valve assemblies 400 and 502 are provided, which obviate the need to replace the entire medical set, and only requires replacement of the breakaway intra-medical tubing connector assembly 800.

In this third operative orientation, the first tubular portion 280 of the connector element 104 protrudes outwardly from the connector body 802, to a longitudinal extent provided by the fact that the second annular protrusion 272 of connector element 104 abuts the annular protrusion 854 of the connector body 802. The second tubular portion 282 of the connector element 104 is fully surrounded by the connector body 802.

The snap-fit fitting 806 can be threadably disengaged from the first luer-actuated valve assembly 400 and the second luer-actuated valve assembly 502 can be threadably disengaged from the connector body 802, thus leaving the luer-actuated valve assemblies 400 and 502 along with the medical tubes associated therewith within the desired treatment location.

It is noted that tensile force exerted on the medical set such as IV line may be unintentional, but due to the presence of the breakaway intra-medical tubing connector assembly 800, the catheter or other medical tube associated with one of the luer-actuated valve assemblies 400 and 502 remains in its position within the treatment site and the luer-actuated valve assemblies 400 and 502 are safely sealed once one of the luer-actuated valve assemblies 400 and 502 is disconnected from the breakaway intra-medical tubing connector assembly 800. Therefore, no fluid can unintentionally flow out of the IV line and risk of contamination of the treatment site is prevented due to sealing of the luer-actuated valve assemblies 400 and 502 and the ability to clean the exterior surface of the sealing element 420 and 520 respectively.

It is noted that alternatively disconnection of one of the luer-actuated valve assemblies 400 and 502 from the breakaway intra-medical tubing connector assembly 800 may be intentional in order to provide the physician with access point to the IV line. Once one of the luer-actuated valve assemblies 400 and 502 is exposed, it may be used for administering medicament into the treatment site within the patient body or alternatively for withdrawing a blood sample from the patient's body.

It is a particular feature of an embodiment of the present invention that the breakaway intra-medical tubing connector assembly 800 associated with medical connectors, such as luer-actuated valve assemblies 400 and 502 enables both safe unintentional disconnection of one of the luer-actuated valve assemblies and provides access point to the IV line through the exposed sealing element 420 or 520 when one of the as luer-actuated valve assemblies 400 and 502 is intentionally disconnected.

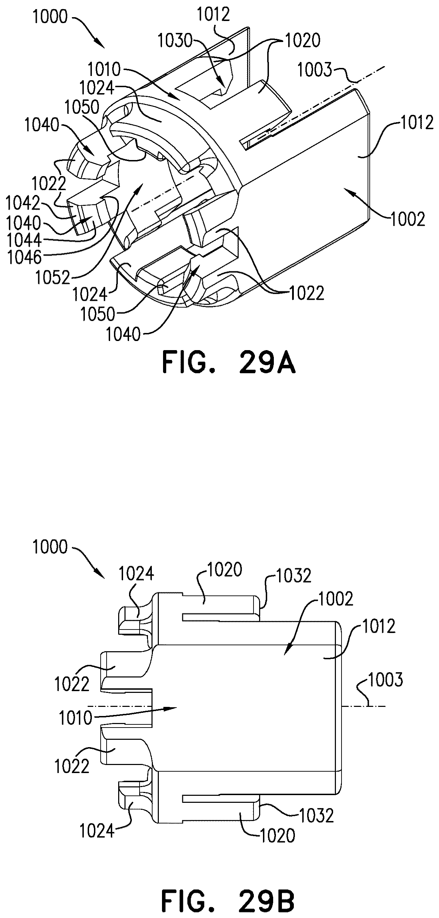

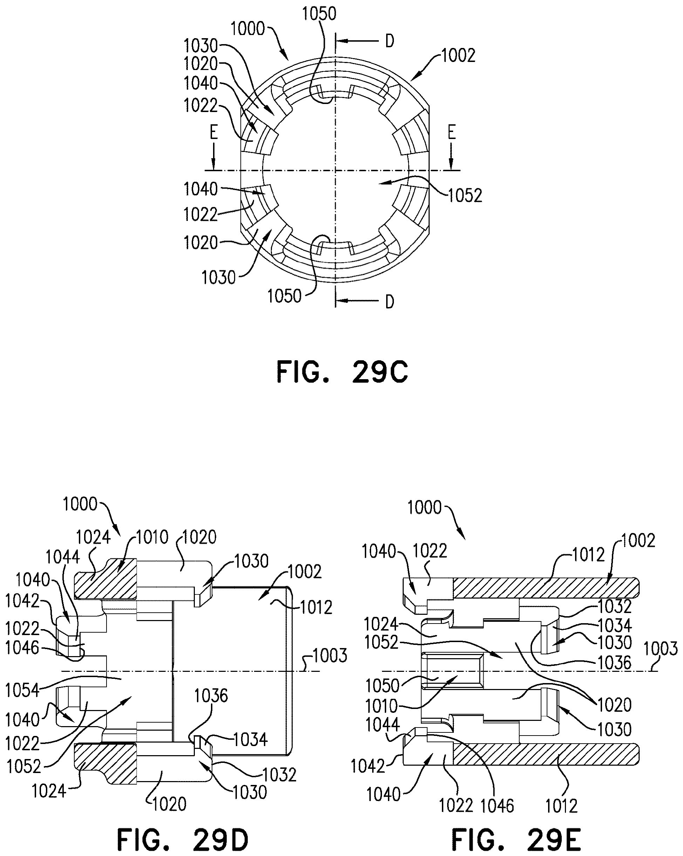

Reference is now made to Figs. 29A - 29C, which are simplified respective perspective view, side view, and top view of a connector body constructed and operative in accordance with yet another embodiment of the present invention and to Fig. 29D and 29E, which are simplified orthogonal sectional illustrations of the connector body of Figs. 29A - 29C, sections being taken along lines D - D and E - E in Fig. 29C.

A connector body 1000 is seen in Figs. 29A - 29E. The connector body 1000 preferably includes a generally longitudinal hollow sleeve 1002 arranged along a longitudinal axis 1003. The hollow sleeve 1002 is an integrally made generally cylindrical hollow element preferably made of plastic, such as Polypropylene, Polycarbonate, Polyethylene.

Sleeve 1002 preferably includes a central hub 1010 having two longitudinally extending arms 1012 extending rearwardly from the circumference of central hub 1010 and are mutually diametrically opposed to each other. A pair of first snap fingers 1020 extend longitudinally rearwardly from the circumference of the central hub 1010 and generally disposed between and radially spaced from arms 1012. The first snap fingers 1020 extend along a portion of the longitudinal extent of the arms 1012.

A pair of second snap fingers 1022 extend longitudinally forwardly from each of the arms 1012. Guiding portions 1024 extend longitudinally forwardly from the circumference of the central hub 1010, disposed forwardly of first snap fingers 1020 and radially spaced from second snap fingers 1022.

First snap fingers 1020 each include a radially inwardly extending snap portion 1030 at its rearward end. Snap portion 1030 includes a rearwardly facing surface 1032, a forwardly tapered surface 1034 and a forwardly facing shoulder 1036. Second snap fingers 1022 each include a snap portion 1040 at its forward end. Snap portion 1040 includes a forwardly facing surface 1042, a rearwardly tapered surface 1044 and a rearwardly facing shoulder 1046.

Guiding portions 1024 each include a radially inwardly directed protrusion 1050.

A thoroughgoing bore 1052 extends along longitudinal axis 1003 through the connector body 1002, including a generally circular bore portion 1054 located at the region of hub 1010 between second snap fingers 1022 and arms 1012.

It is noted that two pairs of first snap fingers 1020 are radially disposed and diametrically opposite to each other, forming an opening between the pairs of first snap fingers 1020. Two pairs of second snap fingers 1022 are radially disposed and diametrically opposite to each other, forming an opening between the pairs of second snap fingers 1022. It is noted that each pair of first snap fingers 1020 is radially disposed between two pairs of second snap fingers 1022. The two pairs of first snap fingers 1020 extend in a first longitudinal direction and the two pairs of second snap fingers 1022 extend in a second longitudinal direction, the first and the second longitudinal directions being opposite to each other.

It is noted that the two pairs of first snap fingers 1020 are resiliently deformable relative to each other due to the fact they are radially spaced from each other, thus allowing radial deformation of the first snap fingers 1020 upon exertion of radial force thereon. The two pairs of second snap fingers 1022 are also resiliently deformable relative to each other due to the fact they are radially spaced from each other, thus allowing radial deformation of the second snap fingers 1022 upon exertion of radial force thereon.

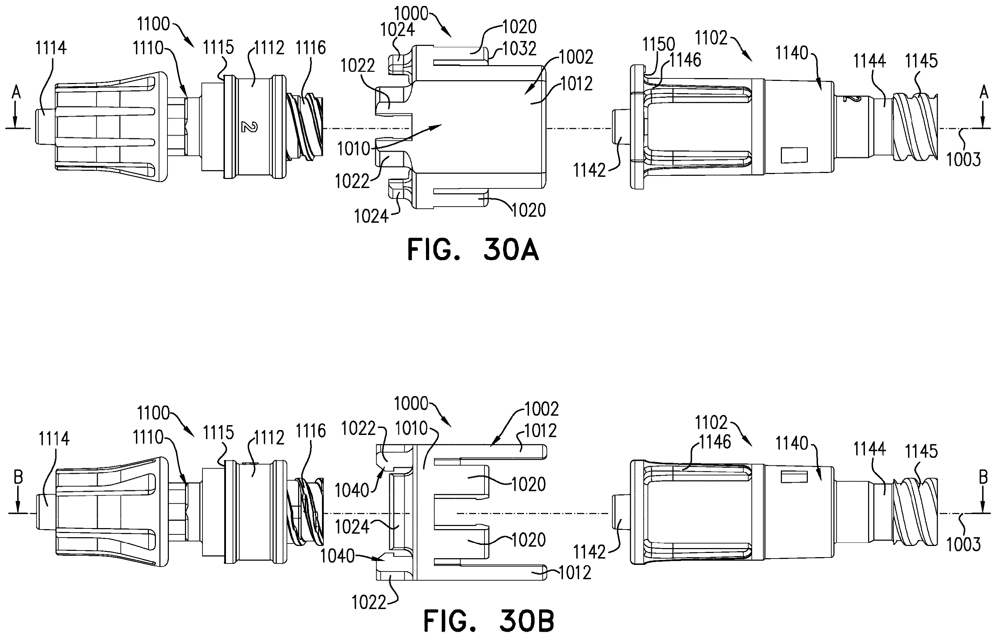

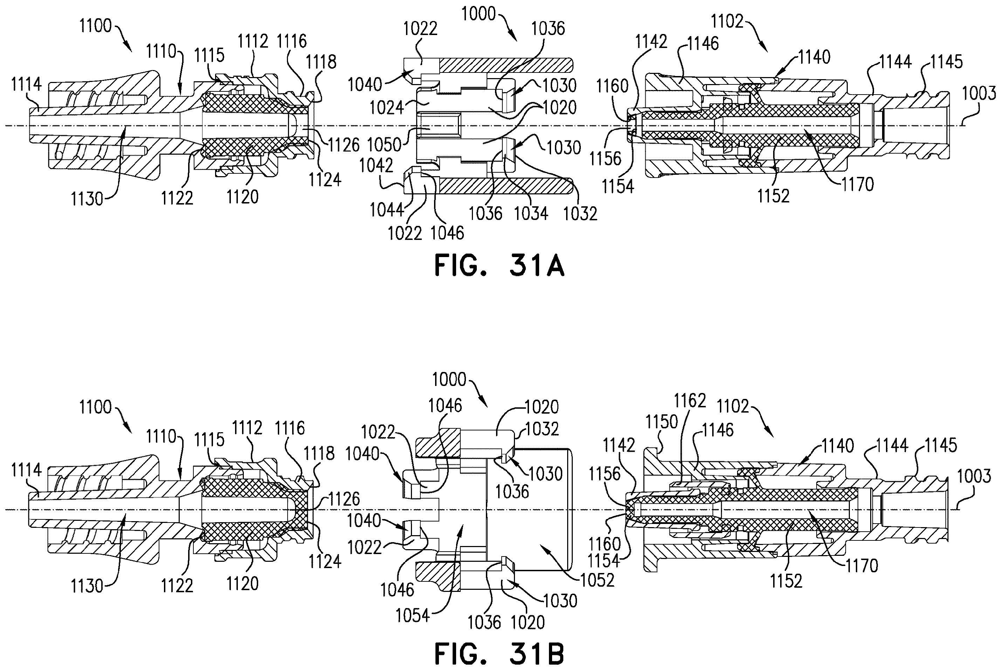

Reference is now made to Figs. 30A and 30B, which are simplified orthogonal side views of the connector body 1000 of Figs. 29A - 29E, shown in a first operative orientation, just prior to connection thereof with a first and a second luer-actuated valve assemblies constructed and operative in accordance with an embodiment of the present invention. Reference is additionally made to Figs. 31A and 31B, which are respective simplified orthogonal sectional views of Figs. 30A and 30B, sections taken along lines A - A in Fig. 30A and lines B - B in Fig. 30B.

It is seen in Figs. 30A - 31B that a first luer-actuated valve assembly 1100 and a second luer-actuated valve assembly 1102 are about to be connected to the connector body 1000 by means of disconnectable snap-fit connection. The first luer-actuated valve assembly 1100 is adapted to be connected with the two pair of second snap fingers 1022 of the connector body 1000 and the second luer-actuated valve assembly 1102 is adapted to be connected with the two pairs of first snap fingers 1020 of the connector body 1000. It is noted that attachment of the first luer-actuated valve assembly 1100 is enabled due to the resilient characteristics provided by the two pairs of second snap fingers 1022. Attachment of the second luer-actuated valve assembly 1102 is enabled due to the resilient characteristics provided by the two pairs of first snap fingers 1020.

It is appreciated that the first luer-actuated valve assembly 1100 is a female luer-actuated valve assembly, which is either similar to or commercially available from various manufacturers, such as Haemopharm, Halkey-Roberts, Paolo Gobbi Frattini S.r.l.

The first luer-actuated valve assembly 1000 generally extend along longitudinal axis 1003 and generally includes a housing 1110 having a female luer portion 1112 and a male luer portion 1114 on an opposite end of the housing 1110 and a forwardly facing shoulder surface 1115 formed therebetween. The female luer portion 1112 has an externally threaded portion 1116 and an opening 1118. A compressible sealing element 1120 is disposed within the housing 1110. The compressible sealing element 1120 has a first open end 1122 and a second selectably openable end 1124, including a selectively openable slit 1126. The selectably openable end 1124 of the sealing element 1120 is disposed across the opening 1118 of the female luer portion 1112 in its normally closed operative orientation and thus is adapted to sealingly close the opening 1118 when the sealing element 1120 is not compressed. It is noted that the sealing element 1120 is biased to its normally closed operative orientation when no stress is applied thereupon. A fluid flow passage 1130 is defined by the inner volume of the sealing element 1120 and the inner volume of the male luer portion 1114, however fluid flow is not permitted through the fluid flow passage 1130 when the slit 1126 is closed and the second selectably openable end 1124 provides a swabbable surface that may be cleaned by the physician in order to prevent contamination.

It is noted that the sealing element 1120 is biased to its normally closed position once a compression force exerted thereon is removed.

It is appreciated that the second luer-actuated valve assembly 1102 is a male luer - actuated valve assembly which is similar to or commercially available from various manufacturers, such as Elcam Medical ACAL, Kibbutz Bar'am, Israel.

The second luer-actuated valve assembly 1102 preferably includes a housing 1140 having a male luer portion 1142 at one end and a female luer portion 1144 with an externally threaded portion 1145 at an opposite end. A forward housing portion 1146 generally surrounds the male luer portion 1142 and defines a rearwardly facing shoulder surface 1150 adjacent the forward end thereof. The housing 1140 is adapted for receiving a resilient sealing element 1152, which has a forward end 1154 with a selectably openable slit 1156. It is noted that the male luer portion 1142 has an opening 1160 and when no pressure is exerted on the sealing element 1152, the forward end 1154 of the sealing element 1152 is disposed near the opening 1160 of the male luer portion 1142, thereby urging sealable closing of the openable slit 1156. Once pressure is exerted on the sealing element 1152 by an actuator 1162, the sealing element 1152 is urged to be displaced rearwardly, thus disengaging the forward end 1154 thereof from opening 1160 and thus causing opening of the slit 1156.

It is noted that the sealing element 1152 is biased to its normally closed operative orientation when no stress is applied thereupon. A fluid flow passage 1170 is defined by the inner volume of the sealing element 1152 and partially the inner volume of the male luer portion 1142, however fluid flow is not permitted through the fluid flow passage 1170 when the slit 1156 is closed and the opening 1160 provides a swabbable surface that may be cleaned by the physician in order to prevent contamination.

It is noted that the sealing element 1152 is biased to its normally closed position once force exerted thereon is removed.

It is noted that alternatively, any other type of male luer-actuated valve actuated by a female luer-actuated valve can be used in conjunction with the connector body 1000, constructed and operative in accordance with an embodiment of the present invention.

It is specifically seen in Figs. 30A - 31B that the luer-actuated valve assemblies 1100 and 1102 are not yet mounted onto the connector body 1000, thus both luer-actuated valve assemblies 1100 and 1102 are sealingly closed in this operative orientation.

It is further noted that the male luer portion 1114 of the first luer-actuated valve assembly 1100 is adapted to be connected to an infusion bag through medical tubing, such as for example, an IV line or a catheter. The female luer portion 1144 of the second luer-actuated valve assembly 1102 is adapted to be connected through an IV line to a catheter or a needle, which is placed within a particular treatment site within the body of the patient. The catheter or the needle is preferably fixed to the skin of the patient by means of an adhesive tape or alike.

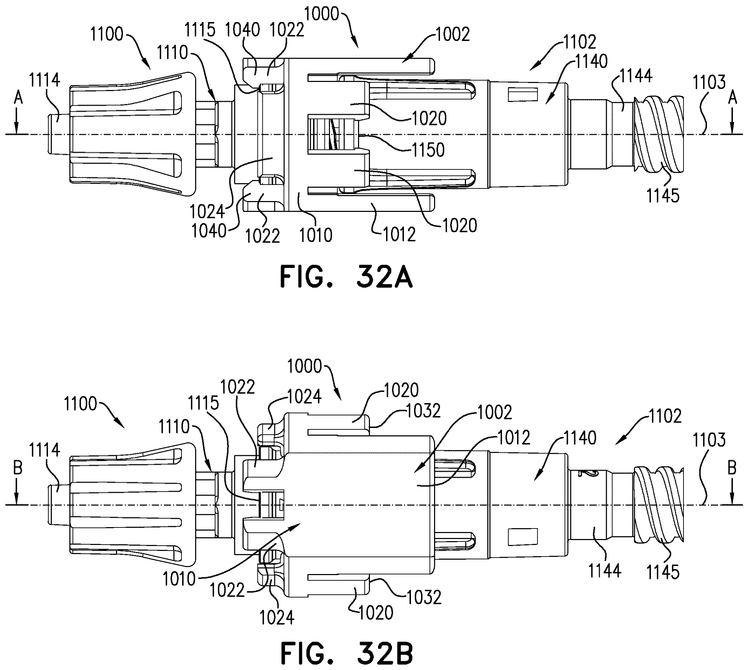

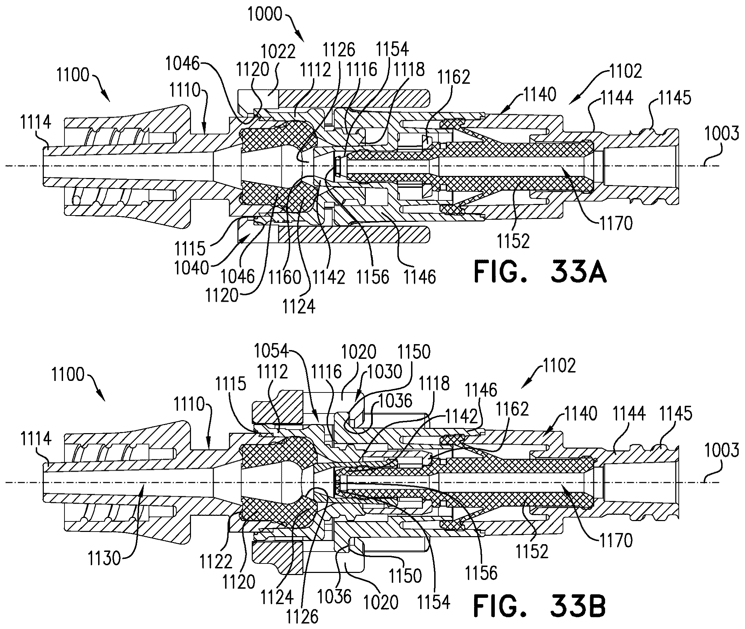

Reference is now made to Figs. 32A and 32B, which are simplified orthogonal side views of the connector body 1000 of Figs. 29A - 29E, shown in a second operative orientation, when the first and the second luer-actuated valve assemblies 1100 and 1102 of Figs. 30A and 30B are connected to the connector body 1000 of Figs. 29A - 29E. Reference is also made to Figs. 33A and 33B, which are respective simplified orthogonal sectional views of Figs. 31A and 31B, sections taken along lines A - A in Fig. 31A and lines B - B in Fig. 31B.

It is seen in Figs. 32A - 33B that both the first luer-actuated valve assembly 1100 and the second luer-actuated valve assembly 1102 are connected to the connector body 1000 in a snap-fit manner. The first luer-actuated valve assembly 1100 is connected to the connector body 1000 by means of engagement between the forwardly facing shoulder surface 1115 of the first luer-actuated valve assembly 1100 and rearwardly facing shoulder 1046 of second snap fingers 1022 of the connector body 1000. The second luer-actuated valve assembly 1102 is connected to the connector body 1000 by means of engagement between the rearwardly facing shoulder surface 1150 of the second luer-actuated valve assembly 1102 and forwardly facing shoulder 1036 of first snap fingers 1020 of the connector body 1000.

It is a particular feature of an embodiment of the present invention that upon connection of both the first and the second luer-actuated valve assemblies 1100 and 1102 to the connector body 1000, a fluid flow passage is established between two medical tubes, each of which is adapted to be connected to the respective male luer portion 1114 of the first luer-actuated valve assembly 1000 and to the female luer portion 1144 of the second luer-actuated valve assembly 1102.

Specifically, it is seen in Figs. 33A and 33B that upon engagement of both the first and second luer-actuated valve assemblies 1100 and 1102, the rearward end of the female luer portion 1112 of the first luer-actuated valve assembly 1100 applies pressure on the actuator 1162, thereby pulling the sealing element 1152 of the second luer-actuated valve assembly 1102 rearwardly, thus urges rearward displacement of the sealing element 1152 and opening of slit 1156. Simultaneously, the male luer portion 1142 of the second luer-actuated valve assembly 1102 is partially inserted into the female luer portion 1112 of the first luer-actuated valve assembly 1100 and thus compresses the sealing element 1120 thereof and urges forward displacement of the sealing element 1120 and in turn causing opening of the slit 1126.

The fluid flow passage is established from a first medical tube that is adapted to be connected to the male luer portion 1114 of the first luer-actuated valve assembly 1100, through the fluid flow passage 1130 thereof, via slit 1126 of the sealing element 1120 and further through the inner volume of the male luer portion 1142 of the second luer-actuated valve assembly 1102 and through slit 1156 of the sealing element 1152 into fluid flow passage 1170 of the second luer-actuated valve assembly 1102 and finally into a second medical tube that is adapted to be connected to the female luer portion 1144 of the second luer-actuated valve assembly 1102, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the connector body 1000 constructed and operative in accordance with an embodiment of the present invention.

It is noted that compression forces that the luer-actuated valve assemblies 1100 and 1102 exert on each other are enabled by the fact that both luer-actuated valve assemblies 1100 and 1102 are held in place relative to the connector body 1000 due to snap-fit connection therebetween.

The connector body 1000 is preferably disposable in case the respective snap connections between snap portion 1040 and forwardly facing shoulder surface 1115 provided between the connector body 1000 and the first luer-actuated valve assembly 1100 and the snap portion 1030 and rearwardly facing shoulder surface 1150 provided between the connector body 1000 and the second luer-actuated valve assembly 1102 are deformable, such that additional engagement of one of the luer-actuated valve assemblies 1100 and 1102 with the connector body 1000 is not intended after first use.

It is a particular feature of an embodiment of the present invention that in this operative orientation there is no relative axial displacement along longitudinal axis 1003 between the connector body 1000, the first luer-actuated valve assembly 1100 and the second luer-actuated valve assembly 1102 up until a predetermined tensile force is exerted on one of the second luer-actuated valve assemblies 1100 and 1102. This predetermined tensile force can either enable disengagement of the first luer-actuated valve assembly 1100 from the snap portion 1040 of second snap fingers 1022 of the connector body 1000 or disengagement of the second luer-actuated valve assembly 1102 from the snap portion 1030 of the first snap fingers 1020 of the connector body 1000.

It is noted that one of the luer actuated valve assemblies 1100 and 1102 may be disconnected upon application of a different tensile force magnitude, specifically seen in this embodiment of the present invention that snap portions 1040 extend radially inwardly to a lesser extent in comparison with snap portions 1030, thus the first luer actuated valve assembly 1100 is intended to be disconnected first from the connector body 1000. Alternatively, the connector body 1000 can be designed differently such that the second luer actuated valve assembly 1102 is disconnected first, in case snap portions 1030 extend radially inwardly to a lesser extent than snap portions 1040.

It is a particular feature of an embodiment of the present invention that preferably the engagement between snap portion 1040 of second snap fingers 1022 of the connector body 1000 and the forwardly facing shoulder 1115 of the first luer-actuated valve assembly 1100 is designed to be weaker than the engagement between snap portion 1030 of the first snap fingers 1020 of the connector body 1000 and the 1020 of the second luer-actuated valve assembly 1102, such that upon exertion of tensile force, the first luer-actuated valve assembly 1100 is disconnected first from the connector body 1000. Alternatively, engagement between the connector body 1000 and the first and second luer-actuated valve assemblies 1100 and 1102 may be of a similar extent.

It is noted that both the first and the second luer-actuated valve assemblies 1100 and 1102 are freely rotatable about axis 1003 while being engaged with the connector body 1000 without causing disengagement of the luer-actuated valve assemblies 1100 and 1102 therefrom.

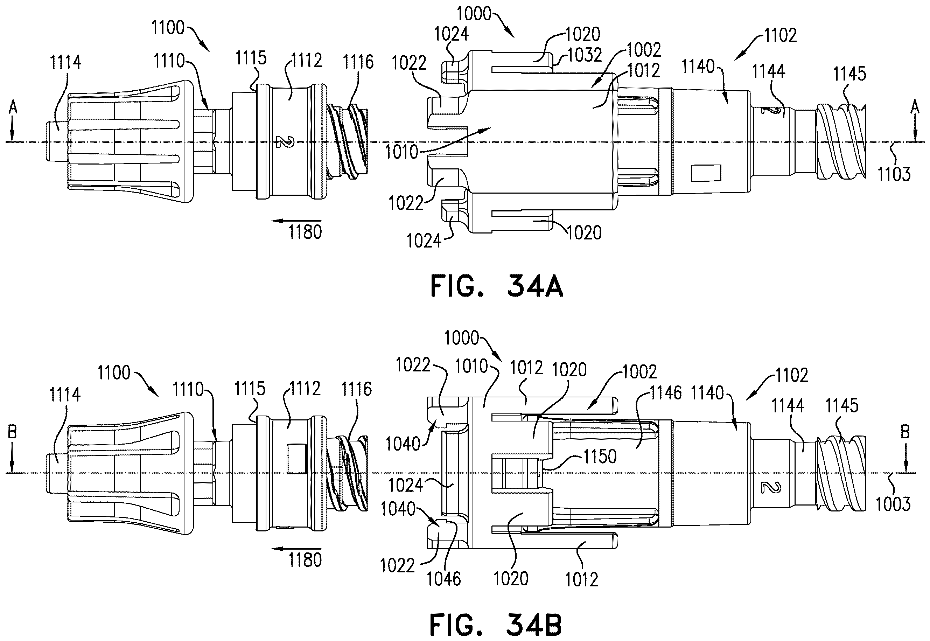

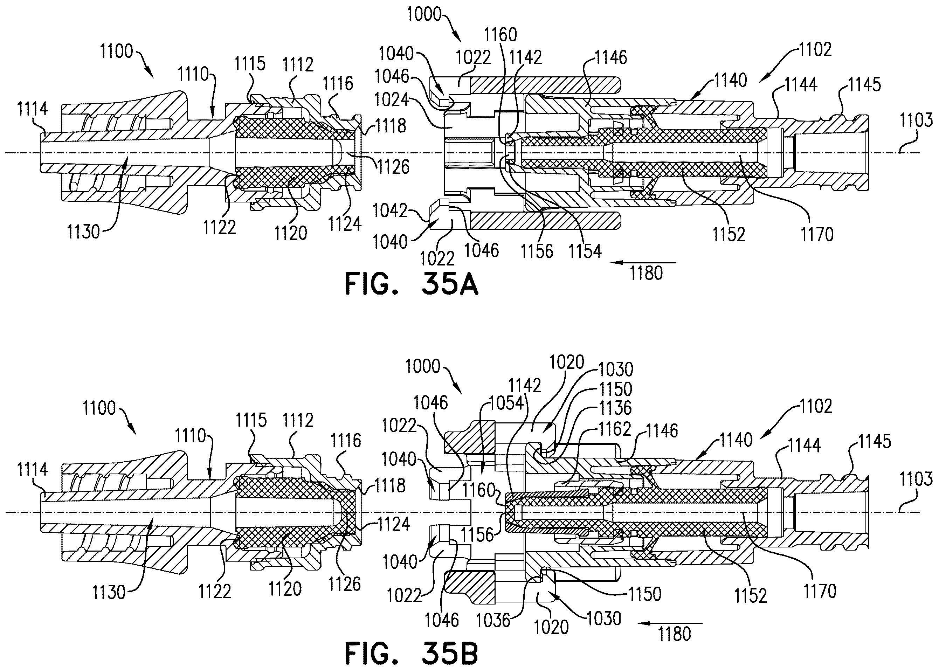

Reference is now made to Figs. 34A and 34B, which are simplified orthogonal side views of the connector body 1000 of Figs. 29A - 29E, shown in a third operative orientation, when one of the luer-actuated valve assemblies 1100 and 1102 of Figs. 30A and 30B is disconnected from the connector body 1000 of Figs. 29A - 29E. Reference is also made to Figs. 35A and 35B, which are respective simplified orthogonal sectional views of Figs. 34A and 34B, sections taken along lines A - A in Fig. 34A and lines B - B in Fig. 34B.

It is seen in Figs. 34A - 35B that a tensile force in a direction indicated by arrow 1180 is applied on the first luer-actuated valve assembly 1100 or the first medical tube and thus causes disconnection between the first luer-actuated valve assembly 1100 and the connector body 1000 by means of breaking the snap fit connection provided between the snap portion 1040 of second snap fingers 1022 of connector body 1000 and between the first luer-actuated valve assembly 1000 due to application of sufficient tensile force. According to an embodiment of the present invention, tensile force in the range of 0.5kgf - 3kgf is required in order to break the snap-fit connection between the first luer-actuated valve assembly 1100 and the connector body 1000.

It is specifically seen in Figs. 35A & 35B that upon application of tensile force upon the first luer-actuated valve assembly 1100 along longitudinal axis 1003 in the direction of arrow 1180, the second snap fingers 1022 are radially outwardly deflected, such that the rearwardly facing shoulder 1046 of snap portion 1040 is disengaged from the forwardly facing shoulder surface 1115 of the first luer-actuated valve assembly 1100 due to the relative resiliency of the second snap fingers 1022 of the connector body 1000 provided by radial spacing therebetween. In accordance with an embodiment of the present invention, the second luer-actuated valve assembly 1102 remains attached to the connector body 1000. Alternatively, the second luer-actuated valve assembly 1102 may be disconnected from the connector body 1000 as well.

It is a particular feature of an embodiment of the present invention that upon disconnection of the first luer-actuated valve assembly 1100 from the connector body 1000, both of the luer-actuated valve assemblies 1100 and 1102 are automatically bidirectionally closed, thus preventing fluid flow passage out of each of the two medical tubes due to the fact that the first and second luer-actuated valve assemblies 1100 and 1102 activate each other once they are both engaged with the connector body 1000.
Upon disengagement of the first luer-actuated valve assembly 1100 from the connector body 1000, the female luer portion 1112 of the first luer-actuated valve assembly 1100 urges biasing of the sealing element 1152 of the second luer-actuated valve assembly 1102 into its normally closed operative orientation, when slit 1156 is closed. Additionally, and simultaneously, male luer portion 1142 of the second luer-actuated valve assembly 1102 disengages from sealing element 1120 of the first luer-actuated valve assembly 1100, thus causes biasing thereof to its normally closed operative orientation, when the slit 1126 is closed.

Closing of both slits 1156 and 1126 prevents fluid flow passage from the first luer-actuated valve assembly 1100 to the second luer-actuated valve assembly 1102 and eventually into the treatment site within the body of the patient, thus also preventing possible contamination of the treatment site.

Upon discarding of the breakaway intra-medical tubing connector assembly 1000, two sealed and swabbale luer-actuated valve assemblies 1100 and 1102 are provided, which obviate the need to replace the entire medical set, and only requires replacement of the connector body 1000.

It is noted that tensile force exerted on the medical set such as IV line may be unintentional, but due to the presence of the connector body 1000 the catheter or other medical tube associated with one of the luer-actuated valve assemblies 1100 and 1102 remains in its position within the treatment site and the luer-actuated valve assemblies 1100 and 1102 are safely sealed once one of the luer-actuated valve assemblies 1100 and 1102 is disconnected from the connector body 1000. Therefore, no fluid can unintentionally flow out of the IV line and risk of contamination of the treatment site is prevented due to sealing of the luer-actuated valve assemblies 1100 and 1102 and the ability to clean the openable end 1124 of the sealing element 1120.

It is noted that alternatively disconnection of one of the luer-actuated valve assemblies 1100 and 1102 from the connector body 1000 may be intentional in order to provide the physician with access point to the IV line. Once one of the luer-actuated valve assemblies 1100 and 1102 is exposed, it may be used for administering medicament into the treatment site within the patient body or alternatively for withdrawing a blood sample from the patient's body.

It is a particular feature of an embodiment of the present invention that the connector body 1000 associated with medical connectors, such as luer-actuated valve assemblies 1100 and 1102 enables both safe unintentional disconnection of one of the luer-actuated valve assemblies and provides access point to the IV line through the exposed sealing element 1120 or 1152 when one of the luer-actuated valve assemblies 1100 and 1102 is intentionally disconnected.

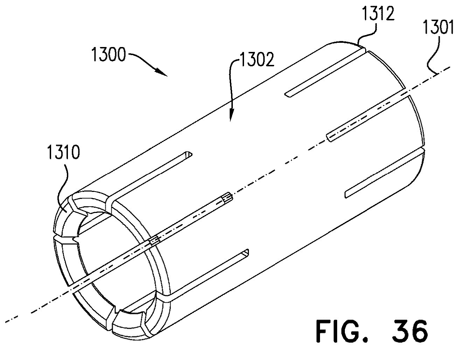

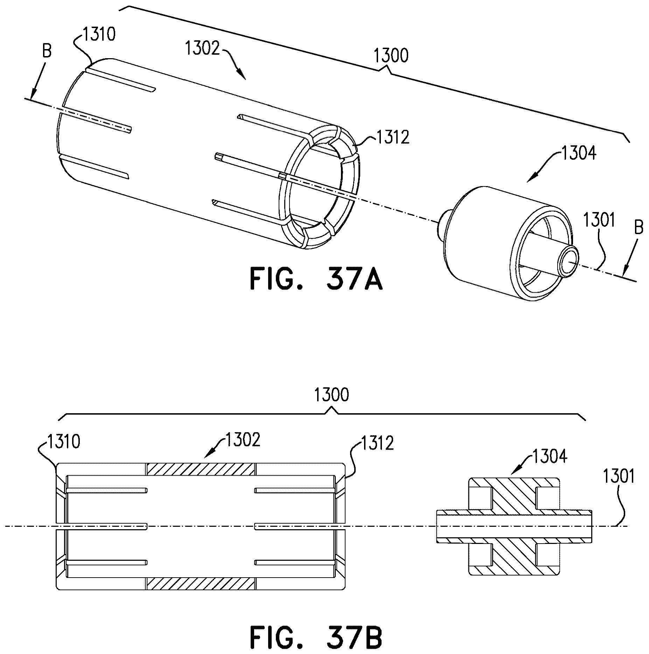

Reference is now made to Fig. 36, which is a simplified pictorial illustration of an assembled breakaway intra-medical tubing connector assembly, constructed and operative in accordance with still another embodiment of the present invention. Reference is additionally made to Figs. 37A and 37B, which are respectively a simplified exploded illustration and a sectional exploded illustration of the breakaway intra-medical tubing connector assembly of Fig. 36, section being taken along lines B - B in Fig. 37A.

A breakaway intra-medical tubing connector assembly 1300 arranged along a longitudinal axis 1301 is seen in Figs. 36 - 37B. The breakaway intra-medical tubing connector assembly 1300 preferably includes a generally cylindrical hollow connector body 1302 and a generally cylindrical hollow connector element 1304, which is adapted to be partially inserted through the inner volume of the connector body 1302. The connector body 1302 and the connector element 1304 are mutually arranged along the longitudinal axis 1301. The connector element 1304 is adapted to be freely axially slidable within the connector body 1302 along the longitudinal axis 1301.

It is seen in Figs. 37A & 37B that the connector body 1302 has a first end 1310 and a second end 1312.

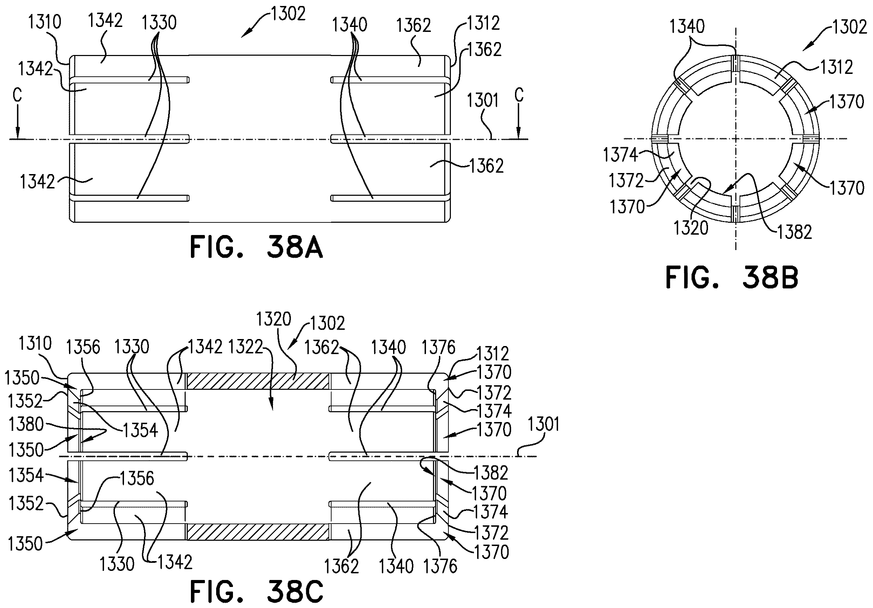

Reference is now made to Figs. 38A - 38C, which are simplified respective side view, top view and a sectional illustration taken along lines C - C in Fig. 38A of the connector body 1302, forming part of the breakaway intra-medical tubing connector assembly 1300 of Figs. 36 - 37B.

The connector body 1302 is an integrally made cylindrical hollow element arranged along longitudinal axis 1301, and preferably made of plastic, such as Polypropylene, Polycarbonate or Polyethylene. The connector body 1302 has first open end 1310 and second open end 1312, as mentioned hereinabove. It is further seen in Figs. 38A - 38C that the connector body 1302 has an inner surface 1320 defining an interior volume 1322 between the first and second open ends 1310 and 1312.

A plurality of first cut-outs 1330 is formed around the circumference of the connector body 1302. First cut-outs 1330 extend longitudinally from the first open end 1310 and rearwardly to a certain longitudinal extent of the connector body 1302. A plurality of second cut-outs 1340 is additionally formed around the circumference of the connector body 1302. Second cut-outs 1340 extend longitudinally from the second open end 1312 and forwardly to a certain longitudinal extent of the connector body 1302.

A plurality of radially arranged spaced apart first arms 1342 are formed between each of the plurality of first cut-outs 1330 and each of the first arms 1342 includes a snap portion 1350 located at the first open end 1310 and extending generally perpendicularly with respect to longitudinal axis 1301. Each of the snap portions 1350 include an outwardly facing edge 1352, which extends radially inwardly and generally perpendicularly to first arms 1342, a generally inwardly tapered edge 1354 extending towards the center of the cylindrical connector body 1302 and an inwardly facing shoulder 1356 facing the inner volume 1322 of the connector body 1302.

A plurality of radially arranged spaced apart second arms 1362 are formed between each of the plurality of second cut-outs 1340 and each of the second arms 1362 includes a snap portion 1370 located at the second open end 1312 and extending generally perpendicularly with respect to longitudinal axis 1301. Each of the snap portions 1370 includes an outwardly facing edge 1372, which extends radially inwardly and generally perpendicularly to second arms 1362, a generally inwardly tapered edge 1374 extending towards the center of the connector body 1302 and an inwardly facing shoulder 1376 facing the inner volume 1322 of the connector body 1302.

A first aperture 1380 is defined by the plurality of snap portions 1350 at the first open end 1310 of the connector body 1302 and a second aperture 1382 is defined by the plurality of snap portions 1370 at the second open end 1312 of the connector body 1302. A through bore 1390 extends axially longitudinally along axis 1301, through connector body 1302 from the first aperture 1380 to the second aperture 1382.

It is noted that the plurality of first cut-outs 1330 provides for relative resiliency of the first open end 1310 of the connector body 1302 and the plurality of second cut-outs 1340 provides for relative resiliency of the second open end 1312 of the connector body 1302, thus allowing for radial deformation of the first and second open ends 1310 and 1312 of the connector body 1302 upon exertion of pressure thereon.

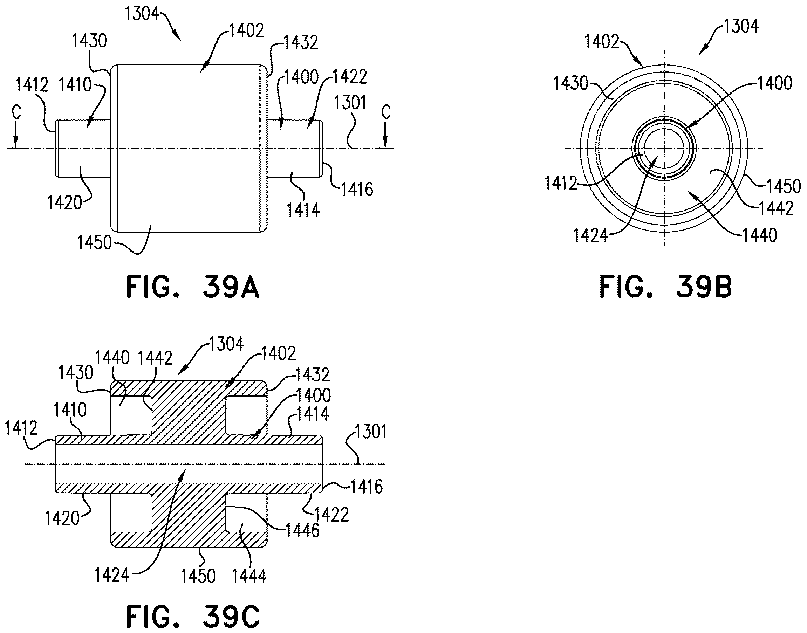

Reference is now made to Figs. 39A - 39C, which are simplified respective side view, top view and a sectional illustration taken along lines C - C in Fig. 39A of the connector element 1304, forming part of the breakaway intra-medical tubing connector assembly 1300 of Figs. 36 - 37B.

The connector element 1304 is an integrally made element arranged along longitudinal axis 1301, and preferably made of plastic, such as Polypropylene, Polycarbonate, Polyethylene. The connector element 1304 includes a generally cylindrical hollow shuttle portion 1400 having a first diameter and an annular sleeve 1402 having a second diameter, which is substantially greater than the first diameter, surrounding a portion of the longitudinal extent of the shuttle portion 1400.

The shuttle portion 1400 has a first tubular end portion 1410 having a first open end 1412 and a second tubular end portion 1414 having a second open end 1416. The first tubular end portion 1410 defines an outer circumferential surface 1420 and the second tubular end portion 1414 defines an outer circumferential surface 1422. The shuttle portion 1400 defines a throuhgoing bore 1424.

The annular sleeve 1402 is located generally at an intermediate location of the shuttle portion 1400 and extends longitudinally, such that a first end 1430 of the annular sleeve 1402 is located generally adjacent the first open end 1412 of the shuttle portion 1400 and a second end 1432 of the annular sleeve 1402 is located generally adjacent the second open end 1416 of the shuttle portion 1400.

A first annular socket 1440 is formed at the first end 1430 of the annular sleeve 1402 and extends towards the middle thereof along longitudinal axis 1301. The first annular socket 1440 defines an outwardly facing annular edge surface 1442. A second annular socket 1444 is formed at the second end 1432 of the annular sleeve 1402 and extends towards the middle thereof along longitudinal axis 1301. The second annular socket 1444 defines an outwardly facing annular edge surface 1446. Annular edge surface 1442 and annular edge surface 1446 are facing opposite directions. The annular sleeve 1402 has a cylindrical exterior surface 1450.

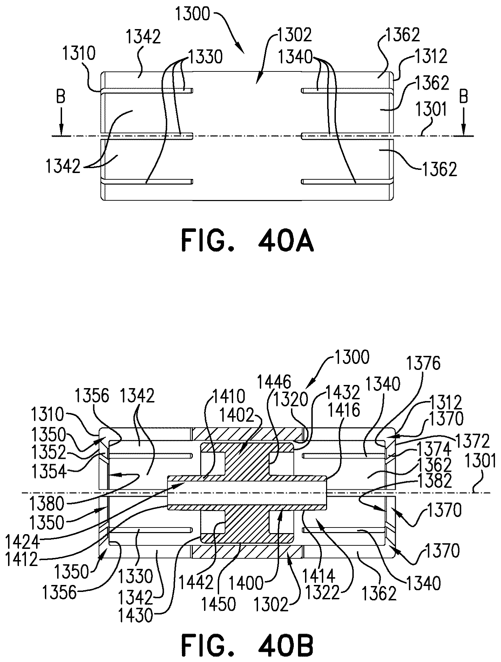

Reference is now made to Figs. 40A and 40B, which are simplified respective side view and a sectional illustration taken along lines B - B in Fig. 40A of the assembled breakaway intra-medical tubing connector assembly 1300 of Figs. 36 - 37B.

It is seen in Figs. 40A and 40B that the connector element 1304 is preferably fully inserted into the connector body 1302. It is noted that the connector element 1304 is shorter than the connector body 1302 in accordance with an embodiment of the present invention. The outer diameter of the annular sleeve 1402 of the connector element 1304 is smaller than the inner diameter of the connector body 1302, thus the connector element 1304 is freely axially displaceable within the interior volume 1322 of the connector body 1302. It is particularly seen that the exterior surface 1450 of the annular sleeve 1402 of the connector element 1304 is slightly spaced from the inner surface 1320 of the connector body 1302.

It is a particular feature of an embodiment of the present invention that the connector element 1304 is adapted to be axially slidable along longitudinal axis 1301 relative to the connector body 1302. The connector element 1304 is displaceable towards the first open end 1310 of the connector body 1302 up to engagement of the first end 1430 of the annular sleeve 1402 of the connector element 1304 with the plurality of snap portions 1350 of the connector body 1302. The connector element 1304 is also displaceable towards the second open end 1312 of the connector body 1302 up to engagement of the second end 1432 of the annular sleeve 1402 of the connector element 1304 with the plurality of snap portions 1370 of the connector body 1302.

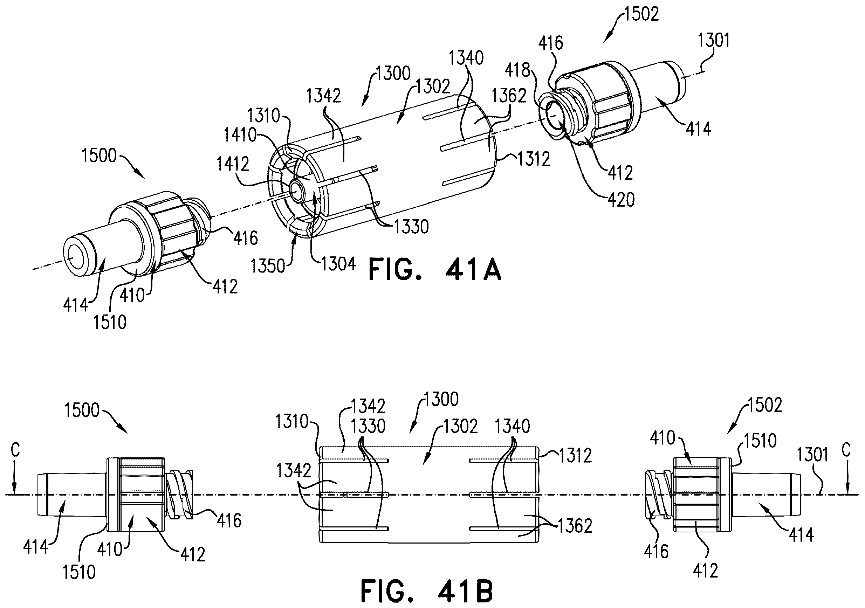

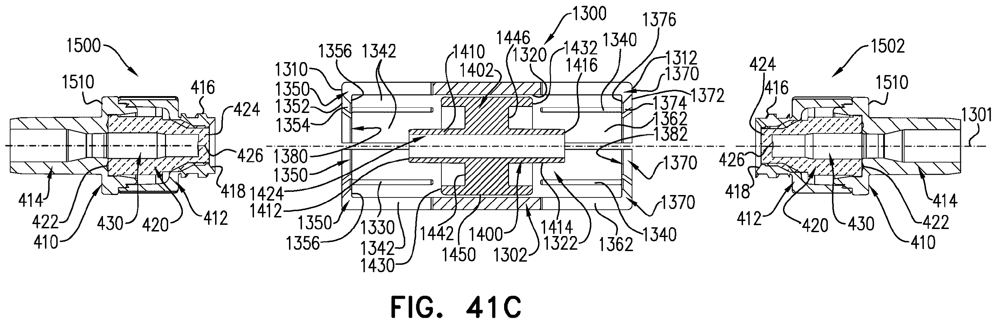

Reference is now made to Figs. 41A - 41C, which are simplified respective perspective view, side view and a sectional illustration taken along lines C - C in Fig. 41B of the breakaway intra-medical tubing connector assembly 1300 of Figs. 40A and 40B, shown in a first operative orientation, just prior to connection thereof with a first and a second luer-actuated valve assemblies constructed and operative in accordance with an embodiment of the present invention.

It is seen in Figs. 41A - 41C that a first luer-actuated valve assembly 1500 and a second luer-actuated valve assembly 1502 are about to be connected to the breakaway intra-medical tubing connector assembly 1300 in a disconnectable snap-fit manner. The first luer-actuated valve assembly 1500 is adapted to be connected to the first end 1310 of the connector body 1302 and the second luer-actuated valve assembly 1502 is adapted to be connected to the second end 1312 of the connector body 1302.

It is appreciated that the first and second luer-actuated valve assemblies 1500 and 1502 are commercially available from various manufacturers, such as Haemopharm, Halkey-Roberts, Paolo Gobbi Frattini S.r.l. It is noted that both first and second luer-actuated valve assemblies 1500 and 1502 are preferably identical. Alternatively, two different first and second luer-actuated valve assemblies 1500 and 1502 can be used with the breakaway intra-medical tubing connector assembly 1300.

It is noted that first and second luer-actuated valve assemblies 1500 and 1502 are preferably substantially similar to first and second luer-actuated valve assemblies 400 and 402 which are described in detail with reference to Figs. 7A - 7C, other than an outwardly facing shoulder surface 1510 formed between the female luer portion 412 and the male luer portion 414 thereof.

It is noted that alternatively, any other type of luer-actuated valve assembly can be used in conjunction with the breakaway intra-medical tubing connector assembly 1300, constructed and operative in accordance with an embodiment of the present invention.

It is specifically seen in Figs. 41A - 41C that the luer-actuated valve assemblies 1500 and 1502 are not yet mounted onto the breakaway intra-medical tubing connector assembly 1300, thus both luer-actuated valve assemblies 1500 and 1502 are sealingly closed in this operative orientation.

It is further noted that the male luer portions 414 of each of the luer-actuated valve assemblies 1500 and 1502 are adapted to be connected to a medical tubing, such as for example, an IV line or a catheter.

Spatial relationships between the various components of the breakaway intra-medical tubing connector assembly 130 preferably remains the same as described with reference to Figs. 40A - 40C.

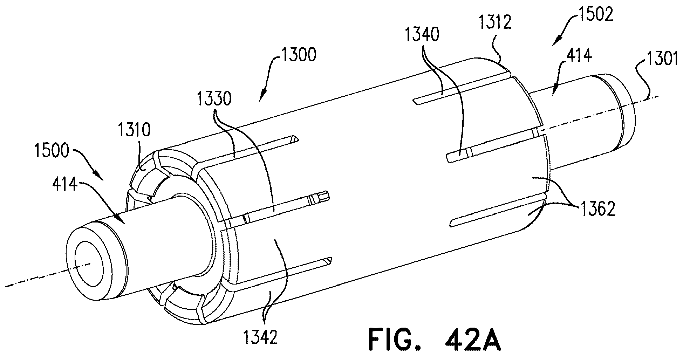

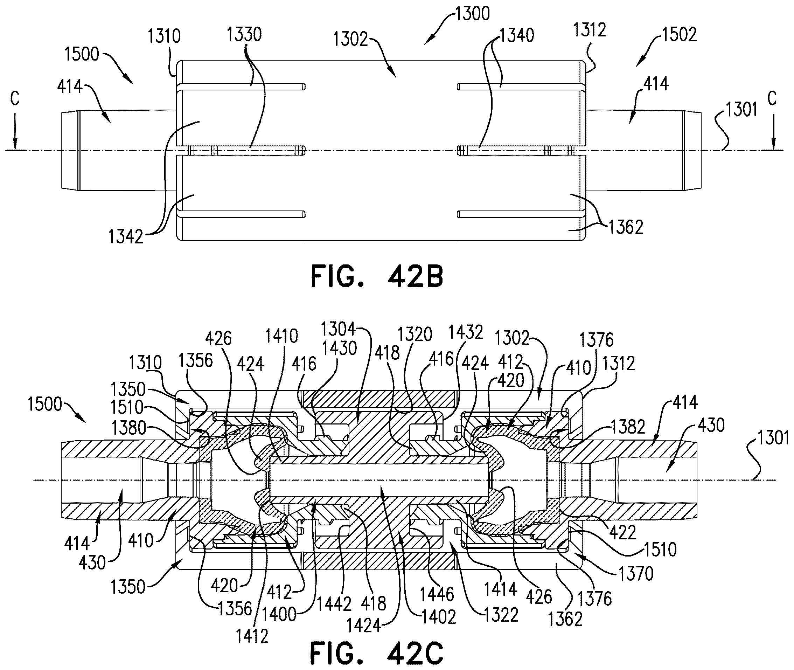

Reference is now made to Figs. 42A - 42C, which are simplified respective perspective view, side view and a sectional illustration taken along lines C - C in Fig. 42B of the breakaway intra-medical tubing connector assembly 1300 of Figs. 40A and 40B, shown in a second operative orientation, when the first and the second luer-actuated valve assemblies 1500 and 1502 of Figs. 41A - 41C are connected to the breakaway intra-medical tubing connector assembly 1300.

It is seen in Figs. 42A - 42C that both the first luer-actuated valve assembly 1500 and the second luer-actuated valve assembly 1502 are connected to the breakaway intra-medical tubing connector assembly 1300. The first luer-actuated valve assembly 1500 is inserted into the connector body 1302 through first aperture 1380 and connected to the connector body 1302 by means of snap-fit engagement between the outwardly facing shoulder surface 1510 of the first luer-actuated valve assembly 1500 and inwardly facing shoulder 1356 of snap portion 1350 of the connector body 1302. The second luer-actuated valve assembly 1502 is inserted into the connector body 1302 through the second aperture 1382 and connected to the connector body 1302 by means of snap-fit engagement between the outwardly facing shoulder surface 1510 of the second luer-actuated valve assembly 1502 and inwardly facing shoulder 1376 of snap portion 1370 of the connector body 1302.

It is noted that attachment of the first and second luer-actuated valve assemblies 1500 and 1502 to the connector body 1302 is enabled due to the resilient characteristics of the connector body 1302, which enables outward deflection of first arms 1342 due to first cut-outs 1330 formed therebetween. Similarly, second arms 1362 can deflect outwardly due to second cut-outs 1340 formed therebetween.

It is a particular feature of an embodiment of the present invention that upon connection of both the first and the second luer-actuated valve assemblies 1500 and 1502 to the breakaway intra-medical tubing connector assembly 1300, a fluid flow passage is established between two medical tubes, each of which is adapted to be connected to the respective male luer portion 414 of the first and second luer-actuated valve assemblies 1500 and 1502.

It is a further particular feature of an embodiment of the present invention that the inner diameter of the fluid flow passage provided between the first and second luer-actuated valve assemblies 1500 and 1502 is substantially the same as the diameter of the thoroughgoing bore 1424 of the connector element 1304.

Specifically, it is seen in Fig. 42C that upon engagement of both the first and second luer-actuated valve assemblies 1500 and 1502, the first tubular portion 1410 of the connector element 1304 compresses the sealing element 420 of the first luer-actuated valve assembly 1500 and thus urges opening of selectively openable slit 426 thereof. Similarly, upon engagement of both the first and second luer-actuated valve assemblies 1500 and 1502, the second tubular portion 1414 of the connector element 1304 compresses the sealing element 420 of the second luer-actuated valve assembly 1502 and thus urges opening of selectively openable slit 426 thereof. The fluid flow passage is established from a first medical tube that is adapted to be connected to the male luer portion 414 of the first luer-actuated valve assembly 1500, through the fluid flow passage 430 thereof, via slit 426 of the sealing element 420 and through the thoroughgoing bore 1424 of the connector element 1304, further via slit 426 of the sealing element 420 of the second luer-actuated valve assembly 1502, through fluid flow passage 430 thereof and finally into a second medical tube that is adapted to be connected to the male luer portion 414 of the second luer-actuated valve assembly 1502, which leads to a desired treatment site within the body of the patient. It is noted that the fluid flow direction can be established in an opposite direction.

It is seen in Fig. 42C that when both luer-actuated valve assemblies 1500 and 1502 are connected to the connector body 1302, the female luer portion 412 of the first luer-actuated valve assembly 1500 engages annular edge surface 1442 of the connector element 1304 and the female luer portion 412 of the second luer-actuated valve assembly 1502 engages annular edge surface 1446 of the connector element 1304.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 1300 constructed and operative in accordance with an embodiment of the present invention.

It is noted that compression forces exerted on both luer-actuated valve assemblies 1500 and 1502 by the connector element 1304 and subsequent opening of the respective slits 423 of the luer-actuated valve assemblies 1500 and 1502 are enabled by the fact that both luer-actuated valve assemblies 1500 and 1502 are held in place relative to the breakaway intra-medical tubing connector assembly 1300 due to snap-fit connection between both luer-actuated valve assemblies 1500 and 1502 and the connector body 1302.

It is a particular feature of an embodiment of the present invention that in this second operative orientation of the intra-medical tubing connector assembly 1300, there is no relative axial displacement along longitudinal axis 1301 between the connector body 1302, the connector element 1304, the first luer-actuated valve assembly 1500 and the second luer-actuated valve assembly 1502, up until a predetermined tensile force is exerted on one of the luer-actuated valve assemblies 1500 or 1502, which can either enable disengagement of the first luer-actuated valve assembly 1500 from the plurality of first snap portions 1350 of the connector body 1302 or disengagement of the second luer-actuated valve assembly 1502 from the plurality of second snap portions 1370 of the connector body 1302.

The breakaway intra-medical tubing connector assembly 1300 is preferably disposable in case the respective snap connections 1350 and 1370 provided on the connector body 1302 are deformable, such that additional engagement of one of luer-actuated valve assemblies 1500 and 1502 with the connector body 1302 is not intended after first use.

It is noted that alternatively, any other type of luer-actuated valve assembly can be used in conjunction with the breakaway intra-medical tubing connector assembly 1300, constructed and operative in accordance with an embodiment of the present invention.

It is noted that both the first and the second luer-actuated valve assemblies 1500 and 1502 may be freely rotated about axis 1301 while being engaged with the intra-medical tubing connector assembly 1300, without causing disengagement of the luer-actuated valve assemblies 1500 and 1502 therefrom.

It is further noted that upon connection of one of the luer actuated valve assemblies 1500 and 1502, the connector element 1304 is axially slidably moveable and thus provides for both of the luer-actuated valve assemblies 1500 and 1502 to remain sealed up until both luer-actuated valve assemblies 1500 and 1502 are connected to the connector body 1302, which provides for opening of the sealing members 420 of both luer-actuated valve assemblies 1500 and 1502.

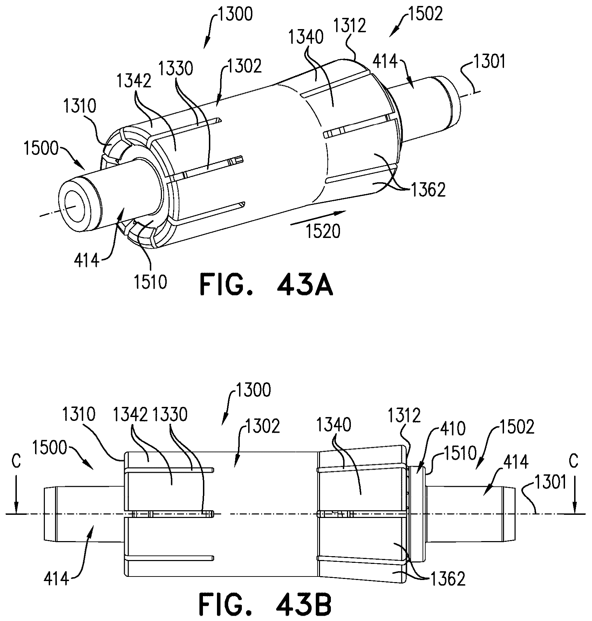

Reference is now made to Figs. 43A - 43C, which are simplified respective perspective view, side view and a sectional illustration taken along lines C - C in Fig. 43B of the breakaway intra-medical tubing connector assembly 1300 of Figs. 40A and 40B, shown in a third operative orientation, which is a transition operative orientation when one of the luer-actuated valve assemblies 1500 and 1502 of Figs. 41A - 41C is in the process of disconnection from the breakaway intra-medical tubing connector assembly 1300.

It is seen in Figs. 43A - 43C that a tensile force in a direction indicated by arrow 1520 is applied on the second luer-actuated valve assembly 1502 or the second medical tube and thus causes at least partial disconnection between the connector body 1302 and the second luer-actuated valve assembly 1502. It is seen that the first luer-actuated valve assembly 1500 remains attached to the connector body 1302. The snap fit connection provided between the connector body 1302 and the second luer-actuated valve assembly 1502 can be broken upon application of sufficient tensile force. According to an embodiment of the present invention, tensile force in the range of 0.5kgf - 3kgf is required in order to break this snap-fit connection.

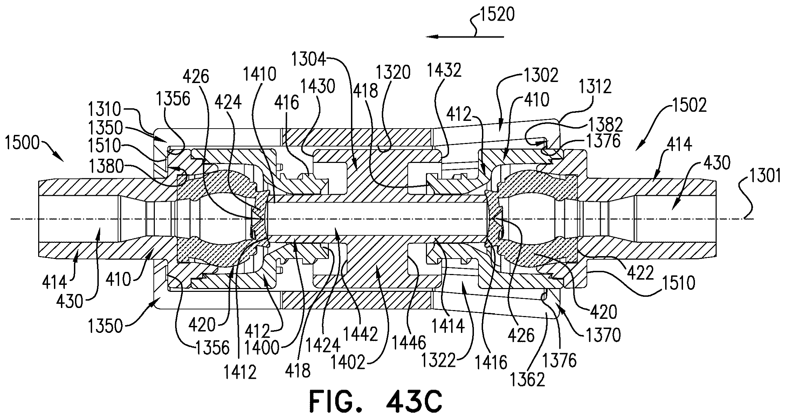

It is specifically seen in Fig. 43C that upon application of tensile force upon the second luer-actuated valve assembly 1502 along longitudinal axis 1301 in the direction of arrow 1520, outwardly facing shoulder surface 1510 of the second luer-actuated valve assembly 1502 disengages from inwardly facing shoulder 1376 of snap portion 1370 of the connector body 1302 due to the relative resiliency of the second end 1312 of the connector body 1302 provided by provided by the material it is formed of as well as by the the second cut-outs 1340 formed between arms 1362, which allow outward deflection of the arms 1362.

It is noted that one of the luer actuated valve assemblies 1500 and 1502 may be disconnected upon application of a different tensile force magnitude. In case that snap portion 1370 extends radially inwardly to a lesser extent in comparison with snap portion 1350, then the second luer actuated valve assembly 1502 is intended to be disconnected first from the connector body 1300. Alternatively, the connector body 1300 can be designed differently such that the first luer actuated valve assembly 1500 is disconnected first, in case snap portion 1350 extends radially inwardly to a lesser extent in comparison with snap portion 1370.

It is noted that in this third operative orientation of the intra-medical tubing connector assembly 1300, the second luer-actuated valve assembly 1502 remains partially connected to the connector body 1302 such that the first tubular portion 1410 of the connector element 1304 still protrudes into female luer portion 412 of the first luer actuated valve assembly 1500 and the second tubular portion 1414 of the connector element 1304 still protrudes into female luer portion 412 of the second luer-actuated valve assembly 1502.

It is also seen in Fig. 43C that during disconnection of the second luer-actuated valve assembly 1502, the connector element 1304 is urged to be displaced axially along longitudinal axis 1301 in the direction of arrow 1520 and out of the first luer-actuated valve assembly 1500, due to the biasing force of the sealing element 420 of the first luer-actuated valve assembly 1500 that is applied upon the connector element 1304. It is seen that in this intermediate operative orientation the second luer-actuated valve assembly 1502 is in the process of disconnection from the connector body 1302.

It is a particular feature of an embodiment of the present invention that even during partial disconnection of the second luer-actuated valve assembly 1502 from the breakaway intra-medical tubing connector assembly 1300, both of the luer-actuated valve assemblies 1500 and 1502 are immediately automatically bidirectionally closed due to the fact that the connector element 1304 is axially floating within the connector body 1302, thus preventing fluid flow passage out of each of the two medical tubes.

It is seen in Fig. 43C that upon partial disengagement of the second luer-actuated valve assembly 1502 from the breakaway intra-medical tubing connector assembly 1300, second tubular end portion 1414 of the shuttle portion 1400 of connector element 1304 partially disengages the sealing element 420 of the second luer-actuated valve assembly 1502, and thereby causing the sealing element 420 to assume its normally closed operative orientation, when slit 456 is closed.

Further, upon partial disengagement of the second luer-actuated valve assembly 1502 from the breakaway intra-medical tubing connector assembly 1300, the first tubular end portion 1410 of the shuttle portion 1400 of connector element 1304 partially disengages the sealing element 420 of the first luer-actuated valve assembly 1500, and thereby causing the sealing element 420 to assume its normally closed operative orientation, when slit 426 is closed. Upon disengagement of the connector element 1304 from one of the luer-actuated valve assemblies 1500 and 1502, both valves are automatically biased to their normally closed operative orientations, where slits 426 are closed, thus effectively sealing both luer-actuated valve assemblies 1500 and 1502 and preventing passage of any fluid passage through the IV line.

Closing of both slits 426 prevents fluid flow passage from the first luer-actuated valve assembly 1500 via throuhgoing bore 1424 of connector element 1304 into the second luer-actuated valve assembly 1502 and eventually into the treatment site within the body of the patient, thus also preventing possible contamination of the treatment site.

It is also seen in Fig. 43C that in this intermediate operative orientation that the connector element 1304 is slightly displaced axially along longitudinal axis 1301 towards the second open end 1312 of the connector body 1302. The female luer portion 412 of the first luer-actuated valve assembly 1502 is now spaced from outwardly facing annular edge surface 1442 of the annular sleeve 1402 of the connector element 1304. Additionally, the female luer portion 412 of the second luer-actuated valve assembly 1502 is now spaced from the outwardly facing annular edge surface 1446 of the annular sleeve 1402 of the connector element 1304.

It is a further particular feature of an embodiment of the present invention that the sealing elements 420 of the first and second luer-actuated valve assemblies 1500 and 1502 are biased to their closed operative orientation by means of the partial disengagement of shuttle portion 1400 of the connector element 1304 of the intra-medical tubing connector assembly 1300 from sealing elements 420. This partial disengagement is provided by the fact that one of the housings 410 of the first or the second luer-actuated valve assemblies 1500 or 1502 is no longer supported and held in place by the respective snap portions 1350 or 1370 of the connector body 1302 of the intra-medical tubing connector assembly 1300.

It is noted that tensile force exerted on the medical set such as IV line may be unintentional, but due to the presence of the breakaway intra-medical tubing connector assembly 1300, the catheter or other medical tube associated with one of the luer-actuated valve assemblies 1500 and 1502 remains in its position within the treatment site and the luer-actuated valve assemblies 1500 and 1502 are safely sealed once one of the luer-actuated valve assemblies 1500 and 1502 is even slightly disconnected from the breakaway intra-medical tubing connector assembly 1300. Therefore, no fluid can unintentionally flow out of the IV line and risk of contamination of the treatment site is prevented due to sealing of the luer-actuated valve assemblies 1500 and 1502.

It is noted that alternatively disconnection of one of the luer-actuated valve assemblies 1500 and 1502 from the breakaway intra-medical tubing connector assembly 1300 may be intentional in order to provide the physician with access point to the IV line. Once one of the luer-actuated valve assemblies 1500 and 1502 is exposed, it may be used for administering medicament into the treatment site within the patient body or alternatively for withdrawing a blood sample from the patient's body.

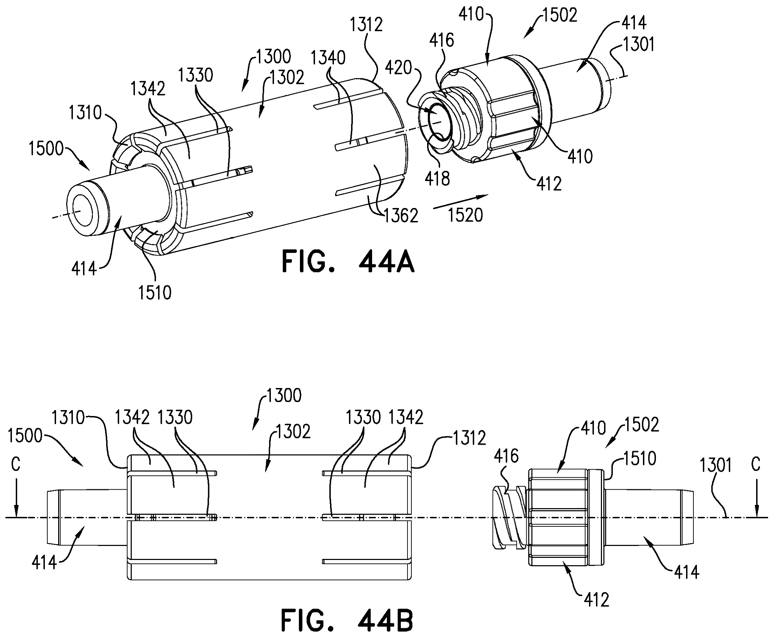

Reference is now made to Figs. 44A - 44C, which are simplified respective perspective view, side view and a sectional illustration taken along lines C - C in Fig. 44B of the breakaway intra-medical tubing connector assembly 1300 of Figs. 40A and 40B, shown in a fourth operative orientation, when one of the luer-actuated valve assemblies 1500 and 1502 of Figs. 41A - 41C is disconnected from the breakaway intra-medical tubing connector assembly 1300.

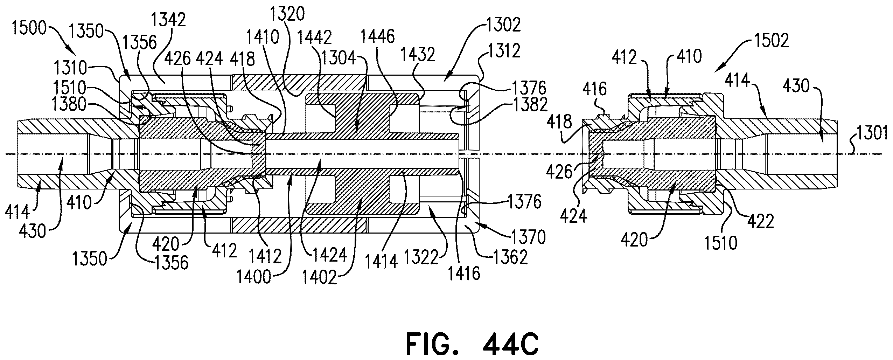

It is particularly seen in Fig. 44C that in this operative orientation the first tubular end portion 1410 of the shuttle portion 1400 of the connector element 1304 does not penetrate the sealing element 420 of the first luer-actuated valve assembly 1500 anymore. The second luer-actuated valve assembly 1502 is now fully disconnected from the second tubular end portion 1414 of the shuttle portion 1400 of the connector element 1304 and from the connector body 1302.

It is further particularly seen in Fig. 44C that upon disconnection of the second luer-actuated valve assembly 1502, the second open end 1312 of the connector body 1302 is radially inwardly deflected due to the resilient characteristics of the end portion of the conector body 1302 provided by the plurality of second cut-outs 1340, thus resuming the normal operative orientation of the connector body 1302, such as seen in Figs. 40A and 40B.

It is seen in Fig. 44C that the outwardly facing shoulder surface 1510 of the first luer-actuated valve assembly 1500 remains engaged with inwardly facing shoulder 1356 of the plurality of snap portions 3150 at the first open end 1310 of the connector body 1302.

It is also seen in Fig. 44C that in this operative orientation the connector element 1304 is displaced axially along longitudinal axis 1301 towards the second open end 1312 of the connector body 1312. The female luer portion 412 of the first luer-actuated valve assembly 1500 is now more spaced from outwardly facing annular edge surface 1442 of the annular sleeve 1402 of the connector element 1304.

It is a particular feature of an embodiment of the present invention that upon disconnection of the second luer-actuated valve assembly 402 from the breakaway intra-medical tubing connector assembly 1300 from the connector body 102, both of the luer-actuated valve assemblies are automatically bidirectionally closed, thus preventing fluid flow passage out of each of the two medical tubes. Upon discarding of the breakaway intra-medical tubing connector assembly 1300, two sealed and swabbale luer-actuated valve assemblies 1500 and 1502 are provided, which obviate the need to replace the entire medical set, and only requires replacement of the breakaway intra-medical tubing connector assembly 1300.

It is noted that tensile force exerted on the medical set such as IV line may be unintentional, but due to the presence of the breakaway intra-medical tubing connector assembly 1300, the catheter or other medical tube associated with one of the luer-actuated valve assemblies 1500 and 1502 remains in its position within the treatment site and the luer-actuated valve assemblies 1500 and 1502 are safely sealed once one of the luer-actuated valve assemblies 1500 and 1502 is disconnected from the breakaway intra-medical tubing connector assembly 1300. Therefore, no fluid can unintentionally flow out of the IV line and risk of contamination of the treatment site is prevented due to sealing of the luer-actuated valve assemblies 1500 and 1502 and the ability to clean the exterior surface of the sealing element 420.

It is noted that alternatively disconnection of one of the luer-actuated valve assemblies 1500 and 1502 from the breakaway intra-medical tubing connector assembly 1300 may be intentional in order to provide the physician with access point to the IV line. Once one of the luer-actuated valve assemblies 1500 and 1502 is exposed, it may be used for administering medicament into the treatment site within the patient body or alternatively for withdrawing a blood sample from the patient's body.

It is a particular feature of an embodiment of the present invention that the breakaway intra-medical tubing connector assembly 1300 associated with medical connectors, such as luer-actuated valve assemblies 1500 and 1502 enables both safe unintentional disconnection of one of the luer-actuated valve assemblies and provides access point to the IV line through the exposed sealing element 420 when one of the luer-actuated valve assemblies 1500 and 1502 is intentionally disconnected.

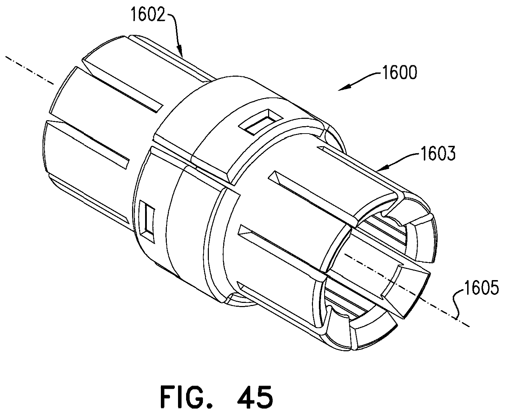

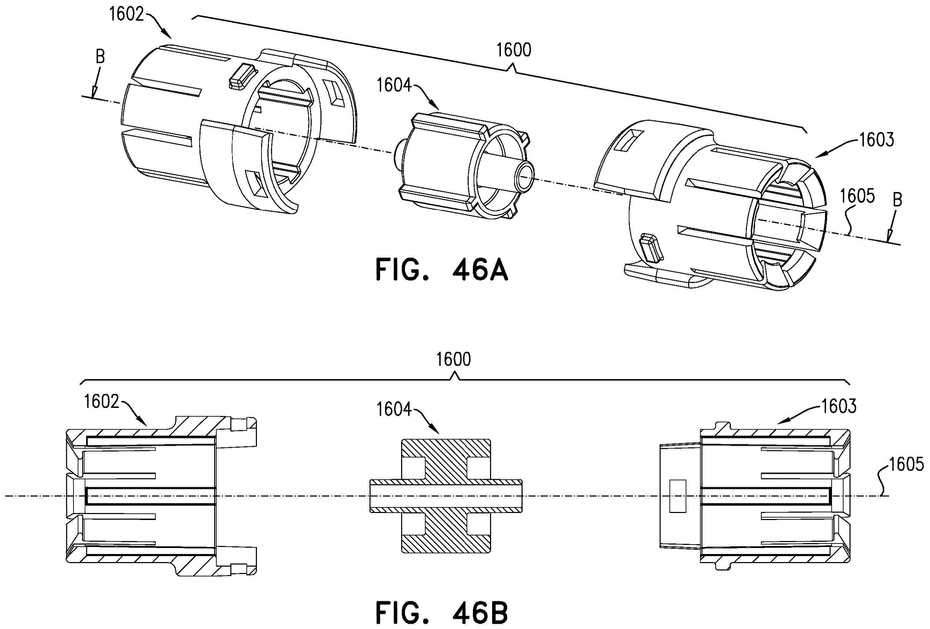

Reference is now made to Fig. 45, which is a simplified pictorial illustration of an assembled breakaway intra-medical tubing connector assembly, constructed and operative in accordance with yet another embodiment of the present invention. Reference is additionally made to Figs. 46A and 46B, which are respectively a simplified exploded illustration and a sectional exploded illustration of the breakaway intra-medical tubing connector assembly of Fig. 45, section being taken along lines B - B in Fig. 46A.

A breakaway intra-medical tubing connector assembly 1600 is seen in Figs. 45 -46B, constructed and operative in accordance with another embodiment of the present invention. The breakaway intra-medical tubing connector assembly 1600 is generally similar to the breakaway intra-medical tubing connector assembly 1300 in all respects other than the fact that the connector body of the breakaway intra-medical tubing connector assembly 1600 is composed of two parts attachable to each other, rather than a single integral part, as shown in Figs. 38A -38C.

The breakaway intra-medical tubing connector assembly 1600 preferably includes a first connector body portion 1602, a second connector body portion 1603 and a connector element 1604, all are mutually arranged a longitudinal axis 1605.

It is a particular feature of an embodiment of the present invention that each of the first connector body portion 1602 and the second connector body portion 1603 preferably includes at least one of a plurality of protrusions or recesses, which correspond to each other and are adapted for snap-engagement and thereby locking the first connector body portion 1602 and the second connector body portion 1603 and form an interior volume therewithin.

It is noted that the first connector body portion 1602 and the second connector body portion 1603 are preferably identical to each other and attached to each other such that one is rotated with respect to another, preferably, the rotation is approximately of 90 degrees, however any other extent of rotation is possible.

The connector element 1604 is adapted to be inserted into the interior volume formed within both the first connector body portion 1602 and the second connector body portion 1603, which are locked to each other. It is appreciated that alternatively the two connector body portions 1602 and 1603 may be welded to each other or otherwise fixedly connected to each other.

It is noted that the fact that the connector body is formed of two portions 1602 and 1603 facilitates the insertion of the connector element 1604 into the interior volume thereof.

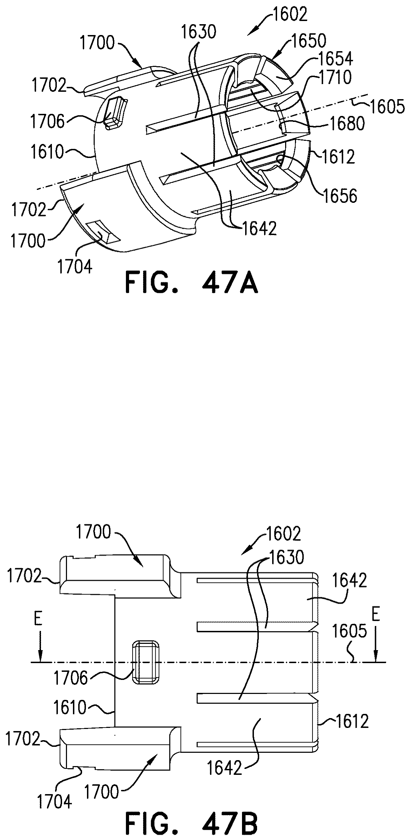

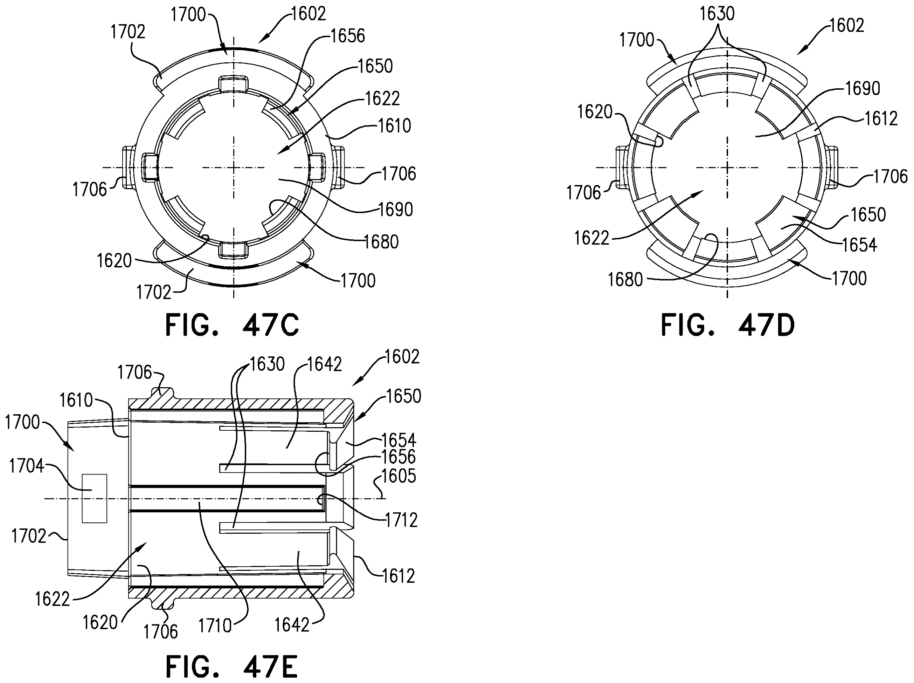

Reference is now made to Figs. 47A - 47E, which are simplified respective perspective view, side view, top view, bottom view and a sectional illustration taken along lines E - E in Fig. 47B of one of the connector body portions 1602 or 1603, forming part of the breakaway intra-medical tubing connector assembly 1600 of Figs. 45 - 46B.

The connector body portion 1602 is an integrally made cylindrical hollow element arranged along longitudinal axis 1605, and preferably made of plastic, such as Polypropylene, Polycarbonate or Polyethylene. The connector body 1602 has first open end 1610 and second open end 1612, as mentioned hereinabove. It is further seen in Figs. 47A - 47E that the connector body portion 1602 has an inner surface 1620 defining an interior volume 1622 between the first and second open ends 1610 and 1612.

A plurality of first cut-outs 1630 is formed around the circumference of the connector body portion 1602. First cut-outs 1630 extend longitudinally from the second open end 1612 and forwardly to a certain longitudinal extent of the connector body portion 1602.

A plurality of radially arranged spaced apart first arms 1642 are formed between each of the plurality of first cut-outs 1630 and each of the first arms 1642 includes a snap portion 1650 located at the second open end 1612 and extending generally perpendicularly with respect to longitudinal axis 1605. Each of the snap portions 1650 include an outwardly facing edge 1652, which extends radially inwardly and generally perpendicularly to first arms 1642, a generally inwardly tapered edge 1654 extending towards the center of the cylindrical connector body portion 1602 and an inwardly facing shoulder 1656 facing the inner volume 1622 of the connector body portion 1602.

An aperture 1680 is defined by the plurality of snap portions 1650 at the second open end 1612 of the connector body portion 1602. A through bore 1690 extends axially longitudinally along axis 1605, through connector body portion 1602 from the first aperture 1680 to the first open end 1610.

It is noted that the plurality of first cut-outs 1630 provides for relative resiliency of the second open end 1612 of the connector body portion 1602, thus allowing for radial deformation of the second open end 1612 of the connector body portion 1602 upon exertion of pressure thereon.

It is further seen in Figs. 47A - 47E that two forwardly extending arms 1700 are generally formed adjacent the first open end 1610 of the connector body portion 1602 and extend generally longitudinally from a location on a circumference of the connector body portion 1602 adjacent the first open end 1610 to a location forwardly located beyond the first open end 1610 and having a forwardly facing end 1702. The arms 1700 are preferably diametrically opposed to each other. Each of the arms has an aperture 1704 preferably formed adjacent the forwardly facing end 1702.

It is further seen in Figs. 47A - 47E that two generally diametrically opposed protrusions 1706 are formed on the circumference of the connector body portion 1602. Each protrusion 1706 is located adjacent the first open end 1610 and between two arms 1700.

A plurality of radially spaced apart grooves 1710 are formed on the inner surface 1620 of the connector body portion 1602. The grooves 1710 extend through the majority of the longitudinal extent thereof and preferably terminate at a shoulder surface 1712 adjacent the plurality of snap portions 1650.

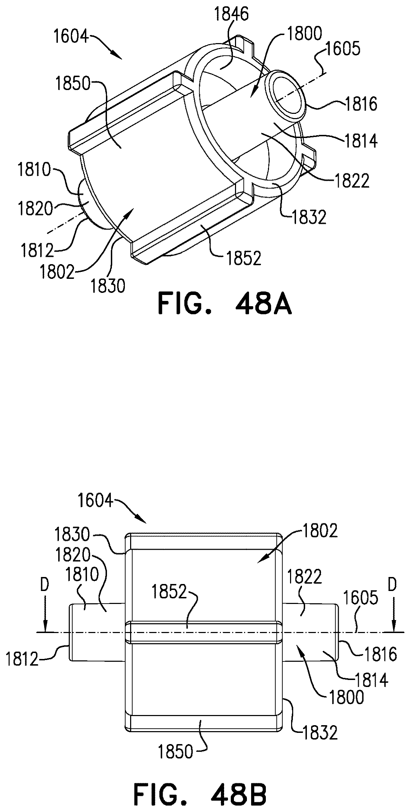

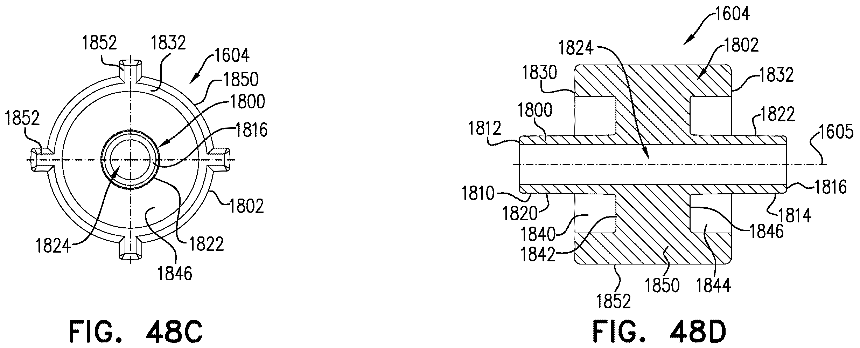

Reference is now made to Figs. 48A - 48D, which are simplified respective perspective view, side view, top view and a sectional illustration taken along lines D - D in Fig. 48B of the connector element 1604, forming part of the breakaway intra-medical tubing connector assembly 1600 of Figs. 45 - 46B.

The connector element 1604 is an integrally made element arranged along longitudinal axis 1605, and preferably made of plastic, such as Polypropylene, Polycarbonate, Polyethylene. The connector element 1604 includes a generally cylindrical hollow shuttle portion 1800 having a first diameter and an annular sleeve 1802 having a second diameter, which is substantially greater than the first diameter, surrounding a portion of the longitudinal extent of the shuttle portion 1800.

The shuttle portion 1800 has a first tubular end portion 1810 having a first open end 1812 and a second tubular end portion 1814 having a second open end 1816. The first tubular end portion 1810 defines an outer circumferential surface 1820 and the second tubular end portion 1814 defines an outer circumferential surface 1822. The shuttle portion 1800 defines a throuhgoing bore 1824.

The annular sleeve 1802 is located generally at an intermediate location of the shuttle portion 1800 and extends longitudinally, such that a first end 1830 of the annular sleeve 1802 is located generally adjacent the first open end 1812 of the shuttle portion 1800 and a second end 1832 of the annular sleeve 1802 is located generally adjacent the second open end 1816 of the shuttle portion 1800.

A first annular socket 1840 is formed at the first end 1830 of the annular sleeve 1802 and extends towards the middle thereof along longitudinal axis 1605. The first annular socket 1840 defines an outwardly facing annular edge surface 1842. A second annular socket 1844 is formed at the second end 1832 of the annular sleeve 1802 and extends towards the middle thereof along longitudinal axis 1605. The second annular socket 1844 defines an outwardly facing annular edge surface 1846. Annular edge surface 1842 and annular edge surface 1846 are facing opposite directions.

The annular sleeve 1802 has a cylindrical exterior surface 1850 and a plurality of radially spaced apart longitudinal ribs 1852 are formed on the exterior surface 1850 of the annular sleeve 1802 and preferably extend along the entire longitudinal extent thereof.

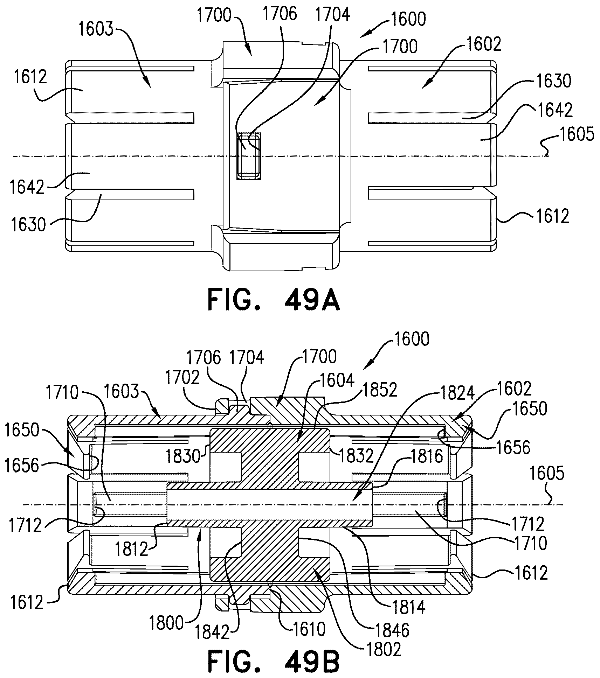

Reference is now made to Figs. 49A and 49B, which are simplified respective side view and a sectional illustration taken along lines B - B in Fig. 49A of the assembled breakaway intra-medical tubing connector assembly 1600 of Figs. 45 - 46B.

It is seen in Figs. 49A and 49B that the connector element 1604 is preferably fully inserted into the two connector body portions 1602 and 1603, which are thereafter lockingly connected to each other. It is seen that the first connector body portion 1602 is preferably identical to the second connector body portion 1603 and they are rotated by 90 degrees with respect to each other. Each of the two arms 1700 of connector body portion 1602 is located between two arms 1700 of the connector body portion 1603. The protrusions 1706 of the connector body portion 1602 are inserted into apertures 1704 of the connector body portion 1603. Similarly, the protrusions 1706 of the connector body portion 1603 are inserted into apertures 1704 of the connector body portion 1602, thereby locking the two connector body portions 1602 and 1603 to each other and enclosing the connector element 1604 therewithin.

It is noted that the connector element 1604 is shorter than the connector body formed of two connector body portions 1602 and 1603 in accordance with an embodiment of the present invention. The ribs 1852 formed on the connector element 1604 are slidably axially displaceable along the grooves 1710 formed on the two connector body portions 1602 and 1603, up to engagement of the ends of the ribs 1852 with either shoulder surface 1712 of the groove of connector body portion 1602 or the shoulder surface 1712 of the groove of the connector body portion 1603.

It is a particular feature of an embodiment of the present invention that the connector element 1604 is adapted to be axially slidable along longitudinal axis 1605 relative to the connector body portions 1602 and 1603.

It is noted that the breakaway intra-medical tubing connector assembly 1600 seen in Figs. 49A and 49B is operable similarly to the breakaway intra-medical tubing connector assembly 1300, which is shown and described with reference to Figs. 40A and 40B. It is appreciated that breakaway intra-medical tubing connector assembly 1600 is positionable in the same operative orientations as shown with respect to breakaway intra-medical tubing connector assembly 1300 in Figs. 41A - 44C and is operable in the same manner.

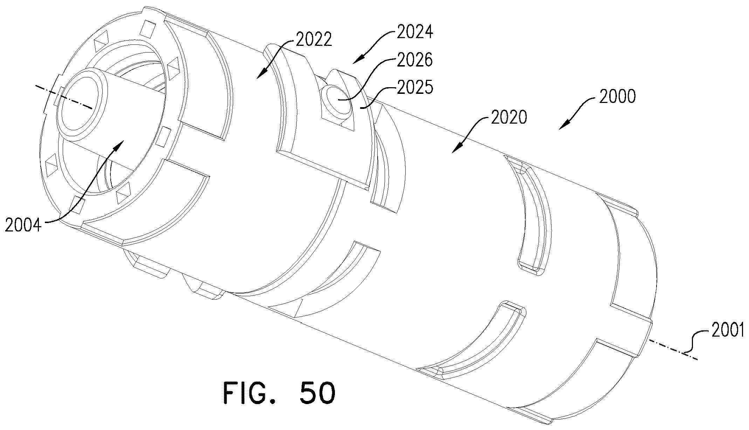

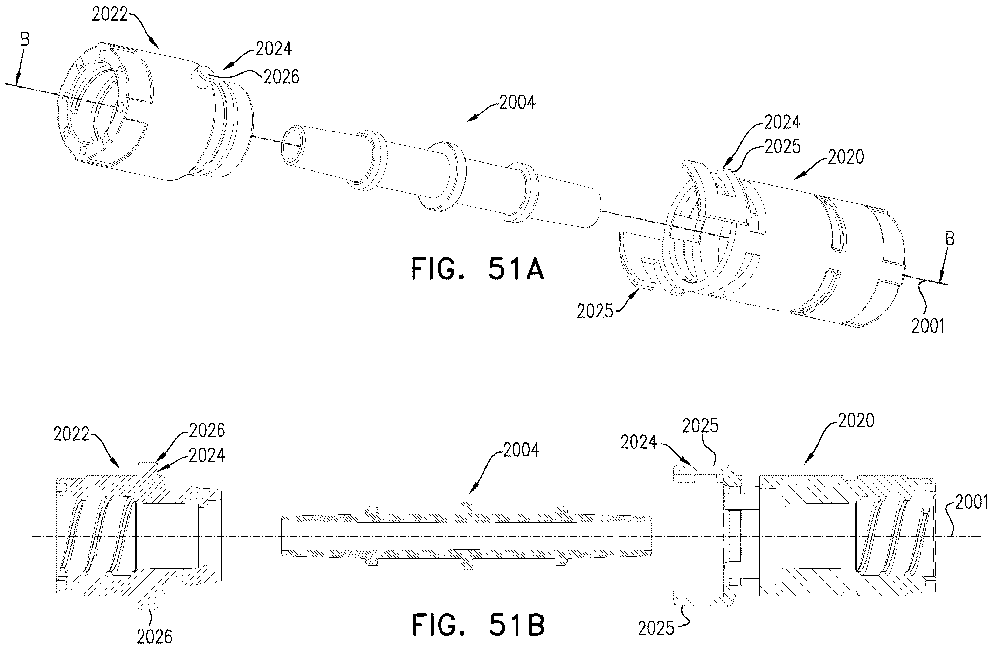

Reference is now made to Fig. 50, which is a simplified pictorial illustration of an assembled breakaway intra-medical tubing connector assembly, constructed and operative in accordance with still another embodiment of the present invention. Reference is additionally made to Figs. 51A and 51B, which are respectively a simplified exploded illustration and a sectional exploded illustration of the breakaway intra-medical tubing connector assembly of Fig. 50, Fig. 51B being taken along lines B - B in Fig. 51A.

A breakaway intra-medical tubing connector assembly 2000 arranged along a longitudinal axis 2001 is seen in Figs. 50 - 51B. The breakaway intra-medical tubing connector assembly 2000 preferably includes a generally cylindrical hollow elongate connector element 2004, a first snap-fit fitting 2020 and a second snap-fit fitting 2022. The connector element 2004 is adapted to be at least partially inserted through an inner volume of at least one of the first snap-fit fitting 2020 and the second snap-fit fitting 2022. The connector element 2004 and both the first snap-fit fitting 2020 and the second snap-fit fitting 2022 are mutually arranged along the longitudinal axis 2001.

It is a particular feature of an embodiment of the present invention, as seen in Figs. 51A & 51B, that the first snap-fit fitting 2020 and the second snap-fit fitting 2022 are adapted to be connected to each other in use in a tensile force responsive disconnectable snap fit connection manner, such that upon application of a tensile force on one of the snap-fit fittings 2020 and 2022 by the user, the two snap-fit fittings 2020 and 2022 are disconnected from each other.

It is a further particular feature of an embodiment of the present invention that during connection of the breakaway intra-medical tubing connector assembly 2000 with luer actuated valves (not shown), the two snap-fit fittings 2020 and 2022 are preferably non-removably locked to each other to provide an additional safety measure during connection of the luer-actuated valves. It is noted that in accordance with an embodiment of the present invention, the two snap-fit fittings 2020 and 2022 are locked to each other by means of a bayonet mechanism 2024, formed of an at least one locking member 2025 formed on the first snap-fit fitting 2020 and an at least one safety pin 2026 formed on the second snap-fit fitting 2022 and configured to cooperate with the locking member 2025.

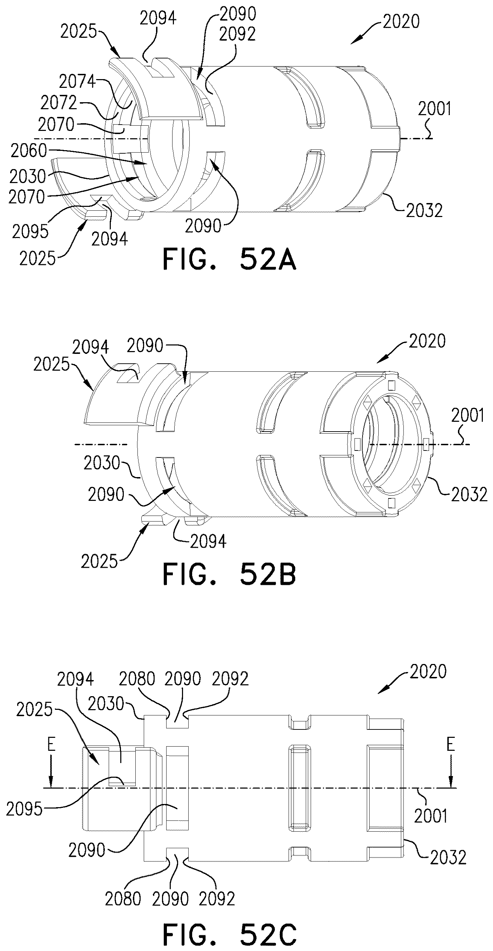

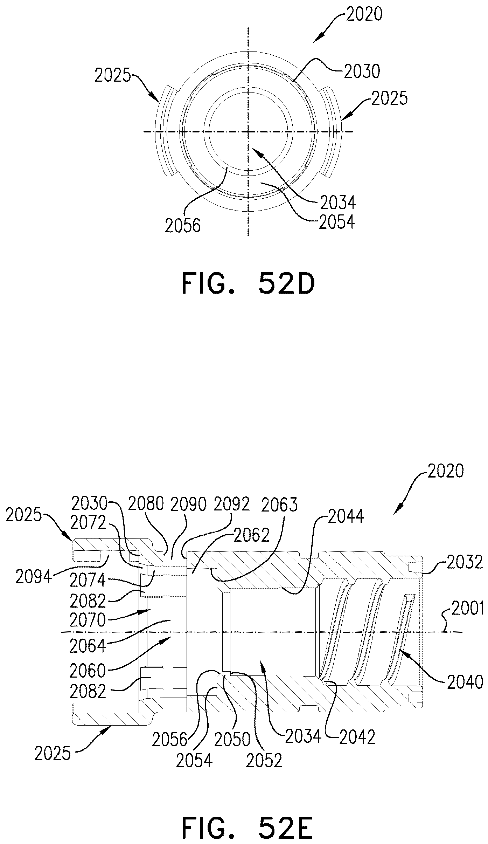

Reference is now made to Figs. 52A - 52D, which are simplified respective perspective, side view, top view and a sectional illustration taken along lines D - D in Fig. 52B of the first snap-fit fitting 2020, forming part of the breakaway intra-medical tubing connector assembly 2000 of Figs. 50 - 51B.

The snap-fit fitting 2020 is an integrally formed element which is generally symmetric about longitudinal axis 2001, and preferably made of plastic, such as Polypropylene, Polycarbonate and Polyethylene. The snap-fit fitting 2020 is a generally cylindrical hollow element having a first open end 2030 and a second open end 2032 and a thoroughgoing bore 2034 extending along longitudinal axis 2001.

An internally threaded portion 2040 forms part of the thoroughgoing bore 2034 of the snap-fit fitting 2020 and is disposed adjacent second open end 2032. The internally threaded portion 2040 extends longitudinally along axis 2001 and terminates at a rearwardly facing shoulder 2042, which extends slightly radially inwardly with respect to the internally threaded portion 2040. The threaded portion 2040 preferably meets ISO standard 80369-7. The rearwardly facing shoulder 2042 faces the second open end 2032 and preferably lies in a plane, which is generally perpendicular to axis 2001.

An intermediate bore portion 2044 extends longitudinally from the rearwardly facing shoulder 2042 and typically extends towards the first open end 2030 and terminates at a circumferential radially inwardly protruding flange 2050, which lies in a plane that is generally perpendicular to axis 2001. The radially inwardly protruding flange 2050 defines a rearwardly facing engagement surface 2052 and a forwardly facing surface 2054, which generally has a tapered portion 2056. It is noted, that the radially inwardly protruding flange 2050 may alternatively be formed of several discrete radially spaced portions instead of a continuous circumferential flange.

A snap-fit bore portion 2060 extends longitudinally from the intermediate bore portion 2044 up to the first open end 2030. The snap-fit bore portion 2060 includes an annular portion 2062 having a first inner diameter and extending forwardly from forwardly facing surface 2054. The annular portion 2062 defines an inner circumferential surface 2063. A tapered portion 2064 extends forwardly from the annular portion 2062 along longitudinal axis 2001. A generally circumferential snap connection 2070 is disposed between the tapered portion 2064 and between the first open end 2030. The snap connection 2070 has a tapered generally circumferential protrusion 2072 extending rearwardly from the first open end 2030 and inwardly into thoroughgoing bore 2034. The tapered generally circumferential protrusion 2072 continues with a generally annular protrusion 2074 extending further away from the first open end 2030 along axis 2001 and defining a rearwardly facing shoulder 2080, which lies generally in a plane perpendicular to the longitudinal axis 2001. Several typically radially spaced apart longitudinal grooves 2082 are formed in snap-fit bore portion 2060 and extend longitudinally along axis 2001, from the first open end 2030 preferably up to the annular portion 2062. Grooves 2082 are typically provided for increasing the resilience of the snap-fit fitting 2020 adjacent to the first open end 2030 thereof.

It is further seen in Figs. 52A - 52D that several radially spaced apart openings 2090 are formed through the snap-fit fitting 2020 adjacent the first open end 2030 thereof. Each of the openings 2090 extends between rearwardly facing shoulder 2080 and a forwardly facing shoulder 2092.

Typically, two diametrically opposed locking members 2025 extend forwardly from a location adjacent the first open end 2030. It is seen in Figs. 52A - 52E that each of the locking members typically includes a radially extending opening 2094 defining a stopping edge 2095. It is noted that alternatively, the locking member 2025 may be a circumferential annular member or any number of locking members 2025 located in a radially spaced manner from each other.

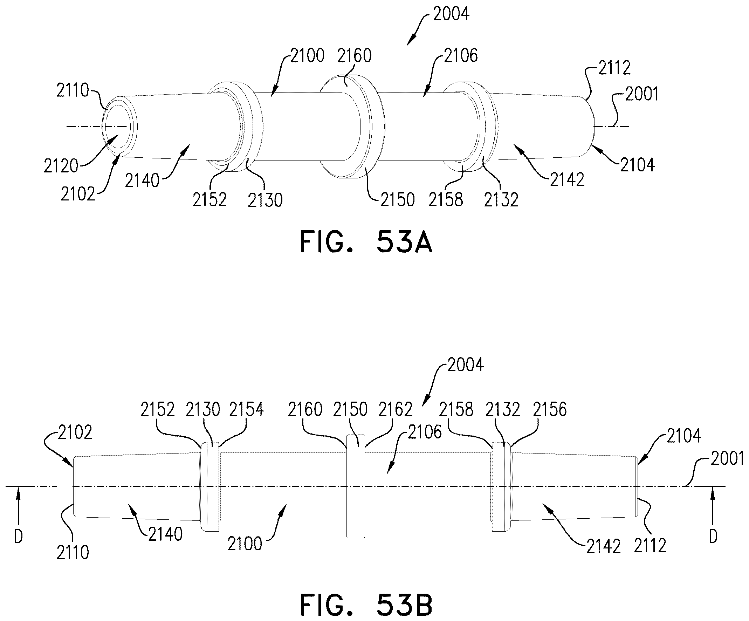

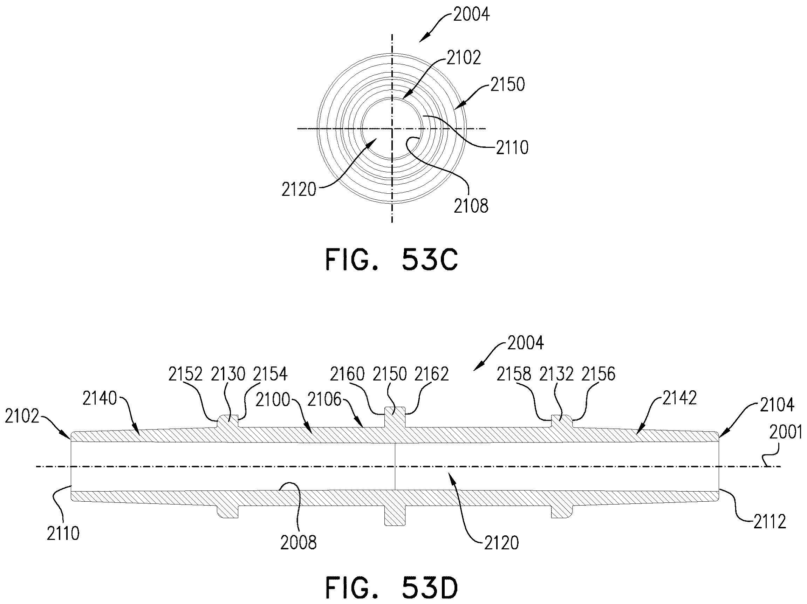

Reference is now made to Figs. 53A - 53D, which are simplified respective perspective, side view, top view and a sectional illustration taken along lines D - D in Fig. 4B of the connector element 2004, forming part of the breakaway intra-medical tubing connector assembly 2000 of Figs. 50 - 51B.

The connector element 2004 is an integrally made element arranged along longitudinal axis 2001, and preferably made of plastic, such as Polypropylene, Polycarbonate, Polyethylene. The connector element 2004 includes a generally cylindrical hollow portion 2100, defining a first end 2102 and a second end 2104. The cylindrical hollow portion 2100 also defines an outer surface 2106 and an inner surface 2108. A generally rounded edge 2110 is formed at the first end 2102 and a generally rounded edge 2112 is formed at the second end 2104. A thoroughgoing bore 2120 extends axially longitudinally along the connector element 2004.

A first annular end protrusion 2130 and a second annular end protrusion 2132 are formed on the cylindrical hollow portion 2100. Both annular protrusions 2130 and 2132 extend generally radially outwardly from the cylindrical hollow portion 2100. The first annular end protrusion 2130 is formed in a relative proximity to the first end 2102 and forming a first generally tubular portion 2140 therebetween. The second annular end protrusion 2132 is formed in a relative proximity to the second end 2104 and forming a second generally tubular portion 2142 therebetween. The first tubular portion 2140 and the second tubular portion 2142 are generally conical. Alternatively, the first tubular portion 2140 and the second tubular portion 2142 may also be cylindrical.

In accordance with an embodiment of the present invention, the first tubular portion 2140 and the second tubular portion 2142 are male luers, which preferably meet ISO standard 80369-7.

It is also seen in Figs. 53A - 53D that in accordance with an embodiment of the present invention, an intermediate annular protrusion 2150 is formed on the cylindrical hollow portion 2100. The intermediate annular protrusion 2150 extends generally radially outwardly from the cylindrical hollow portion 2100 and is formed between annular protrusions 2130 and 2132. It is noted that alternatively, the connector element 2004 may be formed without the intermediate annular protrusion 2150.

The first annular end protrusion 2130 defines a first generally rounded forwardly facing shoulder 2152, which faces the first end 2102 and a second rearwardly facing shoulder 2154 which faces the intermediate annular protrusion 2150. The second annular end protrusion 2132 defines a first generally rounded rearwardly facing shoulder 2156, which faces the second end 2104 and a second forwardly facing shoulder 2158 which faces the intermediate annular protrusion 2150.

The intermediate annular protrusion 2150 defines a forwardly facing shoulder 2160 facing the first annular end protrusion 2130 and a rearwardly facing shoulder 2162 facing the second annular end protrusion 2132.

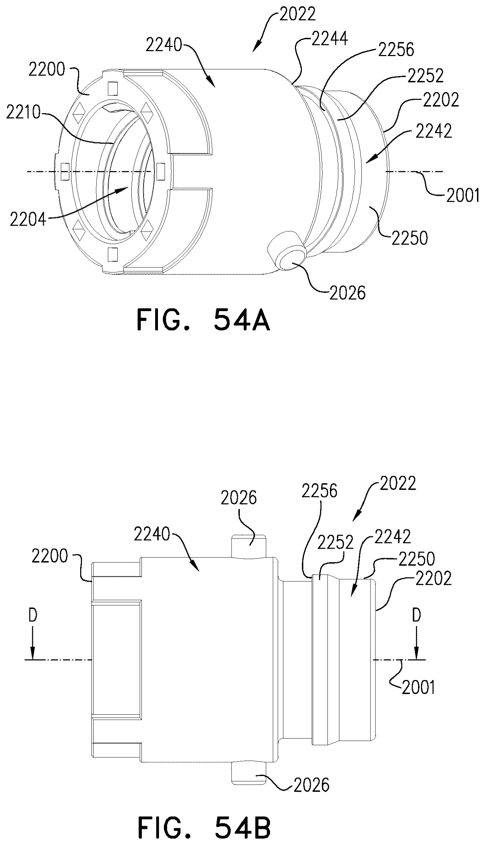

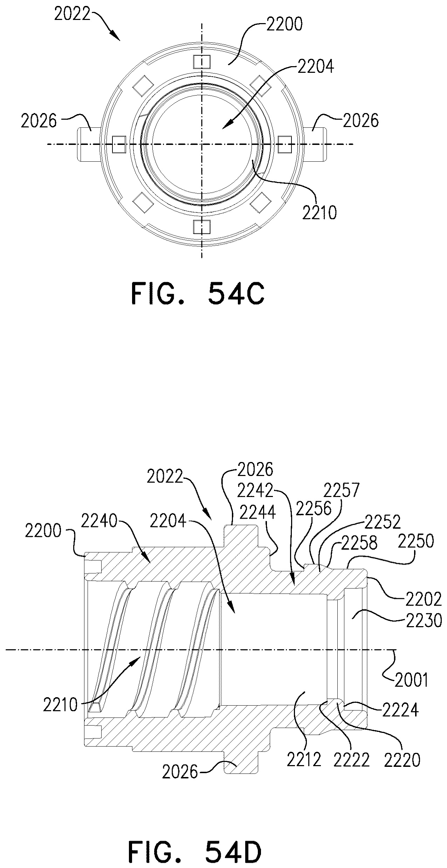

Reference is now made to Figs. 54A - 54D, which are simplified respective perspective, side view, top view and a sectional illustration taken along lines D - D in Fig. 54B of the second snap-fit fitting 2022, forming part of the breakaway intra-medical tubing connector assembly 2000 of Figs. 50 - 51B.

The second snap-fit fitting 2022 is an integrally made element arranged along longitudinal axis 2001, and preferably made of plastic, such as Polypropylene, Polycarbonate, Polyethylene. The second snap-fit fitting 2022 is a generally cylindrical hollow element having a first open end 2200 and a second open end 2202 and a thoroughgoing bore 2204 extending along longitudinal axis 2001.

An internally threaded portion 2210 forms part of the thoroughgoing bore 2204 of the second snap-fit fitting 2020 and is disposed adjacent first open end 2200. The internally threaded portion 2210 extends longitudinally along axis 2001 and preferably meets ISO standard 80369-7. The thoroughgoing bore 2204 further includes an intermediate bore portion 2212 having a first diameter and extending rearwardly of the internally threaded portion 2210 and terminating at a circumferential inwardly radially protruding annular flange 2220 having a forwardly facing shoulder 2222 and a rearwardly facing shoulder 2224. The forwardly facing shoulder 2222 is facing the first open end 2200 and preferably lies in a plane, which is generally perpendicular to axis 2001. It is noted, that the radially protruding annular flange 2220 may alternatively be formed of several discrete radially spaced portions instead of a continuous circumferential flange.

Extending rearwardly from the annular flange 2220 is an annular bore portion 2230 having a second diameter, generally greater than the first diameter and terminating at the second open end 2202.

The second snap-fit fitting 2022 includes a forward portion 2240 having a first outer diameter and extending rearwardly from the first open end 2200 and a rearward portion 2242 having a second outer diameter, which is generally smaller than the first outer diameter. The rearward portion 2242 extends from the second open end 2202 to a rearwardly facing shoulder 2244, which is formed between the forward portion 2240 and the rearward portion 2242.

The rearward portion 2242 defines an outer surface 2250, on which a generally circumferential snap protrusion 2252 is formed. The snap protrusion 2252 extends generally radially outwardly from outer surface 2250 and defines a forwardly facing shoulder 2256, which preferably lies in a plane that is perpendicular to axis 2001, a longitudinal outwardly facing surface 2257 and a rearwardly facing tapered surface 2258. It is noted, that the snap protrusion 2252 may alternatively be formed of several discrete radially spaced portions instead of a continuous circumferential protrusion.

It is further seen in Figs. 54A - 54D that typically two diametrically opposed safety pins 2026 extend radially outwardly from the forward portion 2240 of the second snap-fit fitting 2022.

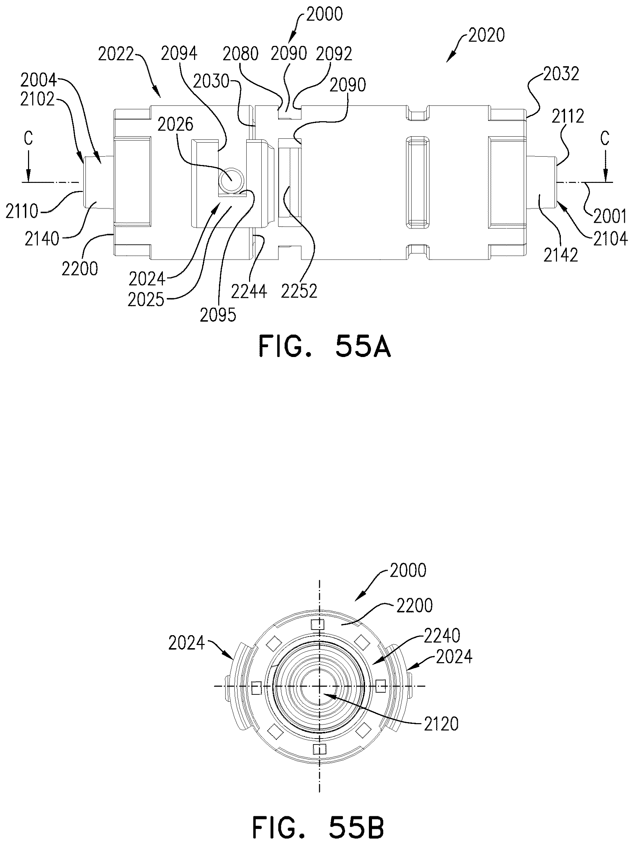

Reference is now made to Figs. 55A - 55C, which are simplified respective side view, top view and a sectional illustration taken along lines C - C in Fig. 55A of the assembled breakaway intra-medical tubing connector assembly 2000 of Figs. 50 - 52B, shown in a locked operative position.

It is seen in Figs. 55A - 55C that the connector element 2004 is at least partially inserted into the through bore 2034 of the first snap-fit fitting 2020 and into the through bore 2204 of the second snap-fit fitting 2022, such that the connector element 2004 and the first and second snap fit fittings 2020 and 2022 extend along mutual longitudinal axis 2001.

It is a particular feature of an embodiment of the present invention that the connector element 2004 is substantially fixedly mounted between the first snap fit fitting 2020 and the second snap fit fitting 2022 due to the intermediate annular protrusion 2150 that is fixedly held between the two snap fit fittings 2020 and 2022. Specifically, the forwardly facing shoulder 2160 of the intermediate annular protrusion 2150 generally abuts the rearwardly facing shoulder 2224 of the second snap fit fitting 2022 and the rearwardly facing shoulder 2162 of the intermediate annular protrusion 2150 generally abuts the forwardly facing surface 2054 of the first snap fit fitting 2020. It is appreciated that the fixed mounting of the connector element 2004 within the two snap-fit fittings 2020 and 2022 facilitates de-bubbling of the medical tube contents before connection of the breakaway intra-medical tubing connector assembly 2000 to the medical tube, specifically one of the tubular portions 2140 or 2142 of the connector element 2004 can be at least partially inserted into the medical tube for dispensing of a certain amount of fluid from the medical tube, for example in order to prime the IV line and remove any trapped air-bubbles therefrom.

It is noted that alternatively, in absence of the intermediate annular protrusion 2150, the connector element 2004 maybe freely slidable with respect to the first and second snap fit fittings 2020 and 2022 along longitudinal axis 2001 up to engagement of either the first annular end protrusion 2130 or the second annular end protrusion 2132 of the connector element 2004 with the respective annular flange 2220 of the second snap fit fitting 2022 and flange 2050 of the first snap fit fitting 2020.

It is a particular feature of an embodiment of the present invention that the first snap-fit fitting 2020 is lockingly connected to the second snap fit fitting 2022 prior to use by means of the bayonet mechanism 2024, and the connector element 2004 is inserted through the through bores 2034 and 2204 of the respective first and second snap fit fittings 2020 and 2022. It is noted that in this prior to use operative orientation, the snap-fit fittings 2020 and 2022 cannot be disconnected from each other in response to tensile force exerted on one of the snap-fit fittings 2020 and 2022 along longitudinal axis 2001, thus providing for enhanced secured connection between the first and second snap-fit fittings 2020 and 2022 during connection of the luer actuated valves 2300 and 2302 thereto.

It is specifically seen that the first snap-fit fitting 2020 and the second snap-fit fitting 2022 are locked to each other by means of the bayonet mechanism 2024, particularly by means of cooperation of the safety pins 2026 of second snap-fit fitting 2022 within the openings 2094 of the locking members 2025 of the first snap-fit fitting 2020, such that axial displacement between the first and second snap-fit fittings 2020 and 2022 along longitudinal axis 2001 is inhibited as long as the safety pins 2026 are located within the openings 2094.

It is noted that alternatively, any other locking mechanism may be used in order to safely lock the two snap-fit fittings 2020 and 2022 to each other. Exemplary locking mechanisms are a locking sleeve, safety pin, thread and a pivotable hinge.

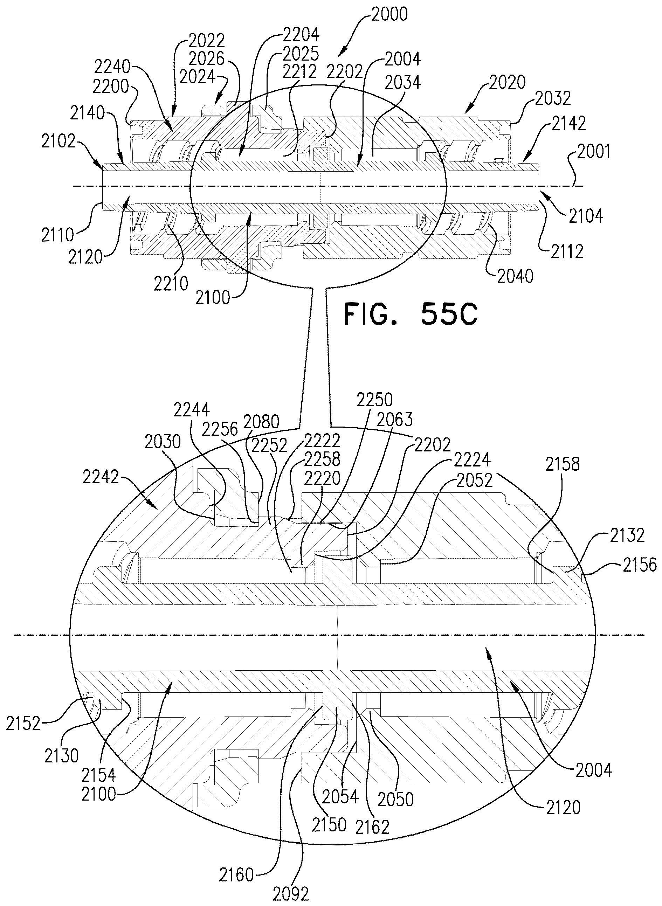

It is specifically seen in Fig. 55C that snap connection 2252 of the second snap-fit fitting 2022 is coupled with snap connection 2070 of the first snap fit fitting 2020, such that snap connection 2252 of the second snap fit fitting 2022 is disposed within each of the openings 2090 of the first snap fit fitting 2020. Specifically, the forwardly facing shoulder 2256 of snap connection 2252 is supported against rearwardly facing shoulders 2080, which defines the boundary of openings 2090.

It is noted that tapered surface 2258 is provided on snap connection 2252 of the second snap fit fitting 2022 and a corresponding tapered protrusion 2072 is provided on the first snap fit fitting 2020 for facilitating connection of the two snap fit fittings 2020 and 2022.

It is a further particular feature of an embodiment of the present invention that an optional sealing is provided between the two snap fit fittings 2020 and 2022 and between the connector element 2004 by means of engagement of the outer surface 2250 of the rearward portion 2242 of the second snap fit fitting 2022 and the inner circumferential surface 2063 of the annular portion 2062 of the first snap fit fitting 2020.

It is noted that in this assembled operative orientation of the breakaway intra-medical tubing connector assembly 2000, the snap fit fittings 2020 and 2022 are static with respect to each other. The connector element 2004 is mounted through the through bore 2034 of the first snap fit fitting 2020 and the through bore 2204 of the second snap fit fitting 2022, preferably such that the first annular end protrusion 2130 of the connector element 2004 is generally disposed within the internally threaded portion 2210 of the second snap fit fitting 2022 and is generally forwardly spaced from flange 2220. The second annular end protrusion 2132 of the connector element 2004 is generally disposed within the internally threaded portion 2040 of the first snap fit fitting 2020 and is generally rearwardly spaced from flange 2050.

It is particularly seen in Figs. 55A - 55C that the first tubular portion 2140 of the connector element 2004 is preferably partially surrounded by internally threaded portion 2210 of the second snap-fit fitting 2022 and partially protrudes outwardly from the first open end 2200 of the second snap-fit fitting 2022. Additionally, the second tubular portion 2142 of the connector element 2004 is partially surrounded by internally threaded portion 2040 of the first snap-fit fitting 2020 and partially protrudes outwardly from the second open end 2032 of the first snap-fit fitting 2020.

It is a particular feature of an embodiment of the present invention that the connector element 2004 is only axially slidable with respect to both snap fit fittings 2020 and 2022 in use, upon release of the locking mechanism 2024 and application of tensile force on one of the snap-fit fittings 2020 and 2022 and their respective disconnection from each other.

It is a particular feature of an embodiment of the present invention that flange 2050 of the first snap-fit fitting 2020 extends radially inwardly to a different extent than flange 2220 of the second snap-fit fitting 2022. Alternatively, both flanges 2050 and 2220 extend radially inwardly to the same extent.

It is a further particular feature of an embodiment of the present invention that there is a minimal radial overlap between flange 2050 of the first snap-fit fitting 2020 and annular end protrusion 2132 of the connector element 2004. Similarly, there is a minimal radial overlap between flange 2220 of the second snap-fit fitting 2022 and annular end protrusion 2130 of the connector element 2004. Thus, in use, the flanges 2050 and 2220 of the two snap-fit fittings 2020 and 2022 respectively are used to prevent axial displacement of the connector element 2004 upon engagement of one of the end protrusions 2130 and 2132 with the respective flanges 2220 and 2050 as long as a predetermined threshold of tensile force is not applied on one of the snap-fit fittings 2020 and 2022.

Insertion of the connector element 2004 into both of the snap-fit fittings 2020 and 2022 is enabled by the respective tapered edges of the respective flanges 2050 and 2220. Specifically, rounded edge of end protrusion 2132 of the connector element 2004 engages the forwardly facing tapered edge of flange 2050 of the first snap-fit fitting 2020 and thus the end protrusion 2132 is inserted into bore 2034 of the first snap-fit fitting 2020. Similarly, rounded edge of end protrusion 2130 of the connector element 2004 engages the rearwardly facing tapered edge of flange 2220 of the second snap-fit fitting 2022 and thus the end protrusion 2130 is inserted into bore 2204 of the second snap-fit fitting 2022.

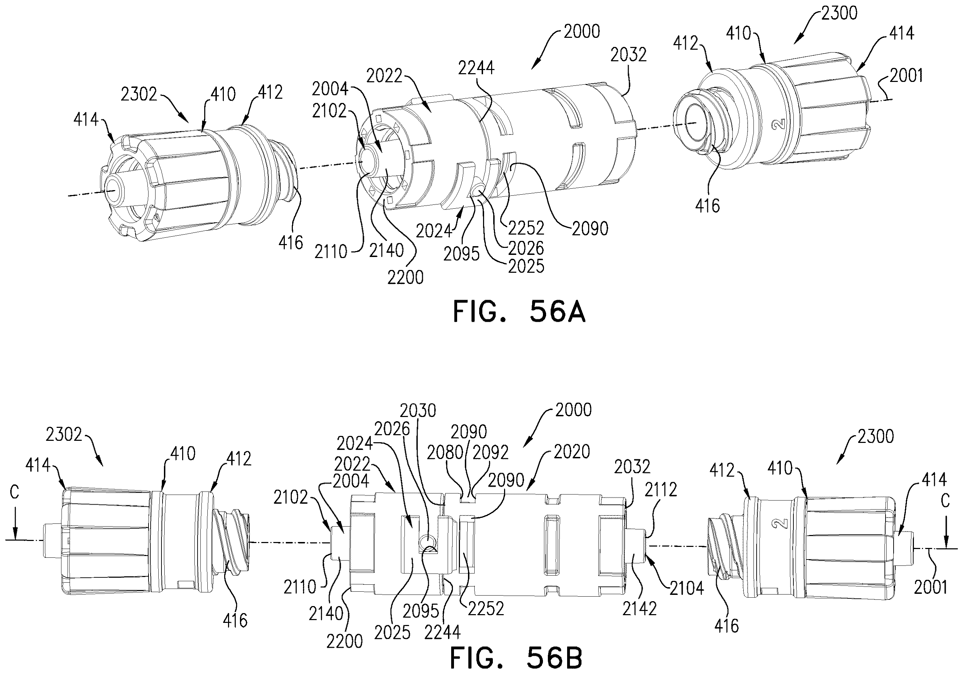

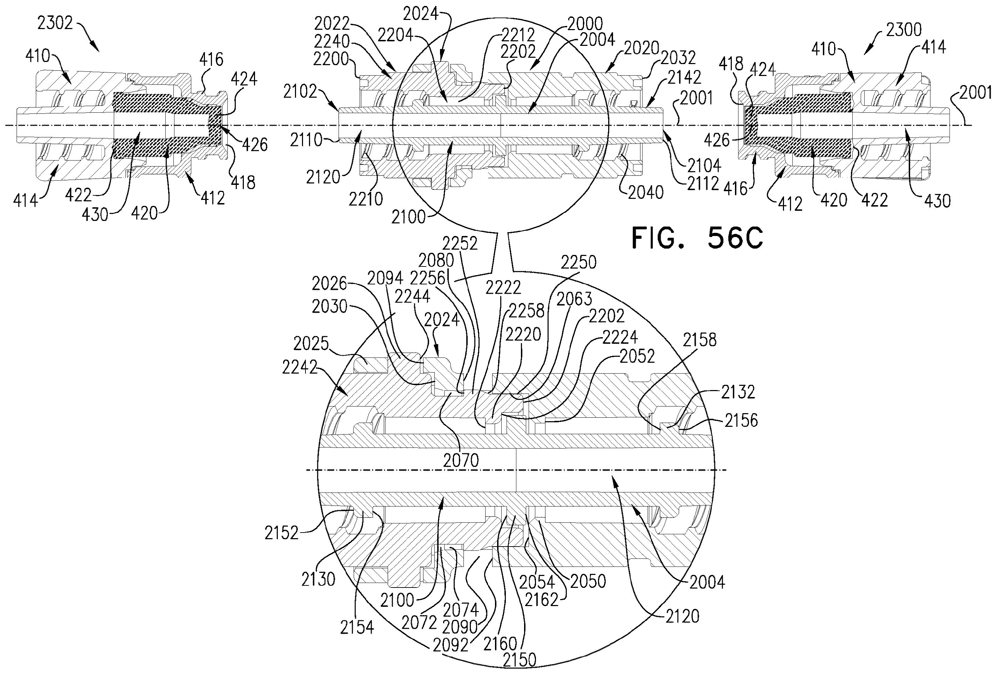

Reference is now made to Figs. 56A - 56C, which are simplified respective perspective view, side view and a sectional illustration taken along lines C - C in Fig. 56B of the breakaway intra-medical tubing connector assembly 2000 of Figs. 55A - 55C, shown in a first operative orientation, just prior to connection thereof with a first and a second luer-actuated valve assemblies constructed and operative in accordance with an embodiment of the present invention, the breakaway intra-medical tubing connector assembly 2000 of Figs. 50 - 52B is shown in a locked operative position.

It is seen in Figs. 56A - 56C that a first luer-actuated valve assembly 2300 and a second luer-actuated valve assembly 2302 are about to be threadably connected to the breakaway intra-medical tubing connector assembly 2100. The first luer-actuated valve assembly 2300 is adapted to be threadably connected to the first snap-fit fitting 2020 and the second luer-actuated valve assembly 2302 is adapted to be threadably connected to the second snap-fit fitting 2022 of the breakaway intra-medical tubing connector assembly 2000.

It is appreciated that the first and second luer-actuated valve assemblies 2300 and 2302 are preferably commercially available from various manufacturers, such as Haemopharm, Halkey-Roberts, Paolo Gobbi Frattini S.r.l, ICU, B.Braun. It is noted that both first and second luer-actuated valve assemblies 2300 and 2302 are preferably identical. Alternatively, two different first and second luer-actuated valve assemblies 2300 and 2302 can be used with the breakaway intra-medical tubing connector assembly 2000.

It is noted that first and second luer-actuated valve assemblies 2300 and 2302 are preferably substantially identical to first and second luer-actuated valve assemblies 400 and 402 which are described in detail hereinabove with reference to Figs. 7A - 7C.

It is noted that alternatively, any other type of luer-actuated valve assembly can be used in conjunction with the breakaway intra-medical tubing connector assembly 2000, constructed and operative in accordance with an embodiment of the present invention. It is particularly noted that luer-actuated valve assemblies having an internal fluid flow passing within the sealing element can be used in conjunction with the breakaway intra-medical tubing connector assembly 2000, as well as luer-actuated valve assemblies having an external fluid flow passing around the sealing element. Some examples of valve assemblies with internal fluid flow are: Smartsite^{®} of Alaris/Carefusion/Becton Dickinson; Microcline^{®} of ICU Medical; In-Vision Plus^{®} of RyMed; Clearlink^{®} of Baxter; One-Link^{®} of Baxter; Nexus TKO ^{®} of Nexus Medical; Caresite ^{®} of B.Braun Medical Inc.; Bionector^{®} of Vygon. Some examples of valve assemblies with external fluid flow are: MaxZero ^{™} of Becton Dickinson; MaxPlus^{™} of Carefusion/ Becton Dickinson; Posiflow^{™} of Becton Dickinson; Ultrasite^{®} of B.Braun Medical Inc.

It is a particular feature of an embodiment of the present invention that luer-actuated valve assemblies with an external fluid flow can be used in conjunction with the breakaway intra-medical tubing connector assembly 2000 due to the fact that the tubular portions 2140 and 2142 of the connector element 2004 are male luers, which preferably meet ISO standard 80369-7 and thus seal against the female luer portion of the luer-actuated valve assembly.

It is specifically seen in Figs. 56A - 56C that the luer-actuated valve assemblies 2300 and 2302 are not yet mounted onto the breakaway intra-medical tubing connector assembly 2000, thus both luer-actuated valve assemblies 2300 and 2302 are sealingly closed in this operative orientation.

It is further noted that the male luer portions 414 of each of the luer-actuated valve assemblies 2300 and 2302 are adapted to be connected to a medical tubing, such as for example, an IV line or a catheter.

It is a particular feature of an embodiment of the present invention that the connector element 2004 is preferably made of a relatively soft plastic material, such as Polyethylene for example, while the commercially available luer-actuated valve assemblies 2300 and 2302 are usually made of Polycarbonate. Due to this fact, the required tensile force for pulling the connector element 2004 out of the female portions of the luer-actuated valve assemblies 2300 and 2302 is minimized.

Spatial relationships between the various components of the breakaway intra-medical tubing connector assembly 2000 preferably remain the same as described with reference to Figs. 55A - 55C.

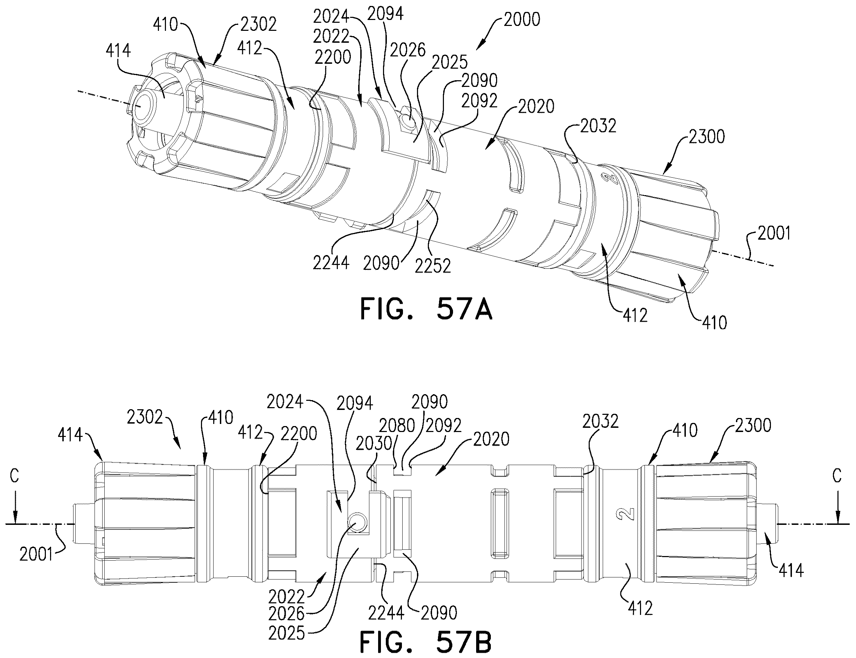

Reference is now made to Figs. 57A - 57C, which are simplified respective perspective view, side view and a sectional illustration taken along lines C - C in Fig. 57B of the breakaway intra-medical tubing connector assembly 2000 of Figs. 55A - 55C, shown in a second operative orientation, when the first and the second luer-actuated valve assemblies 2300 and 2302 of Figs. 56A - 56C are connected to the breakaway intra-medical tubing connector assembly 2000, which is shown in a locked operative position.

It is seen in Figs. 57A - 57C that both the first luer-actuated valve assembly 2300 and the second luer-actuated valve assembly 2302 are threadably connected to the breakaway intra-medical tubing connector assembly 2000. The first luer-actuated valve assembly 2300 is threadably connected to the first snap-fit fitting 2020 by means of engagement between the externally threaded portion 416 of the female luer portion 412 and between the internally threaded portion 2040 of the first snap-fit fitting 2020. The second luer-actuated valve assembly 2302 is threadably connected to the second snap-fit fitting 2022 by means of engagement between the externally threaded portion 416 of the female luer portion 412 and between the internally threaded portion 2210 of the second snap-fit fitting 2022. The first luer-actuated valve assembly 2300 can be threaded up to abutment of its female luer portion 412 with the second open end 2032 of the first snap-fit fitting 2020. The second luer-actuated valve assembly 2302 can be threaded up to abutment of its female luer portion 412 with the first open end 2200 of the second snap-fit fitting 2022.

It is a particular feature of an embodiment of the present invention that upon connection of both the first and the second luer-actuated valve assemblies 2300 and 2302 to the breakaway intra-medical tubing connector assembly 2000, a fluid flow passage is established between two medical tubes, each of which is adapted to be connected to the respective male luer portion 414 of the first and second luer-actuated valve assemblies 2300 and 2302.

It is a further particular feature of an embodiment of the present invention that the inner diameter of the fluid flow passage provided between the first and second luer-actuated valve assemblies 2300 and 2302 is substantially the same as the diameter of the thoroughgoing bore 2120 of the connector element 2004.

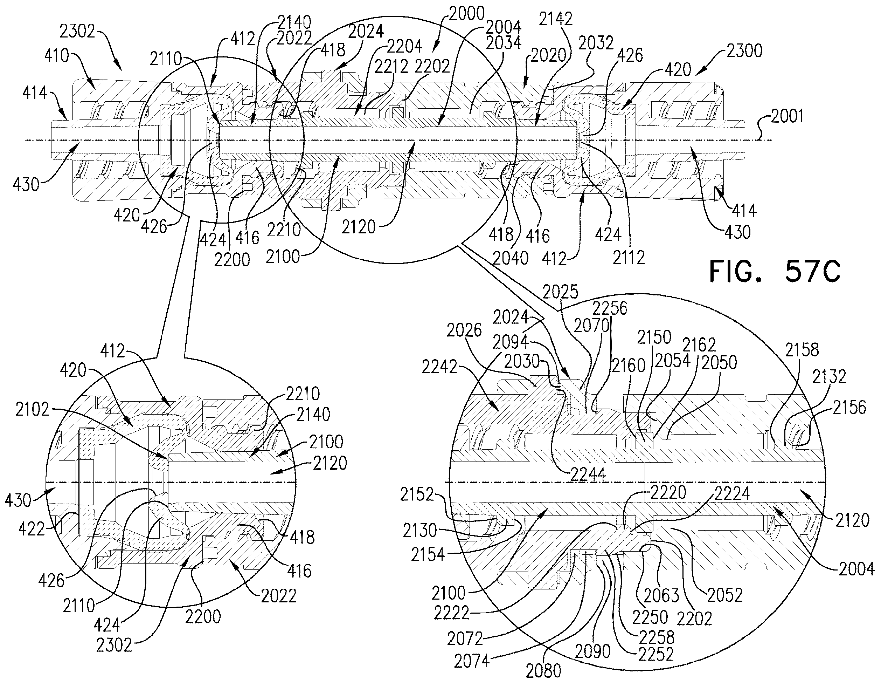

Specifically, it is seen in Fig. 57C that upon threaded engagement of both the first and second luer-actuated valve assemblies 2300 and 2302, the first tubular portion 2140 of the connector element 2004 compresses the sealing element 420 of the first luer-actuated valve assembly 2300 and thus urges opening of selectively openable slit 426 thereof. Similarly, upon threaded engagement of both the first and second luer-actuated valve assemblies 2300 and 2302, the second tubular portion 2142 of the connector element 2004 compresses the sealing element 420 of the second luer-actuated valve assembly 2302 and thus urges opening of selectively openable slit 426 thereof. The fluid flow passage is established from a first medical tube that is adapted to be connected to the male luer portion 414 of the first luer-actuated valve assembly 2300, through the fluid flow passage 430 thereof, via slit 426 of the sealing element 420 and through the thoroughgoing bore 2120 of the connector element 2004, further via slit 426 of the sealing element 420 of the second luer-actuated valve assembly 2302, through fluid flow passage 430 thereof and finally into a second medical tube that is adapted to be connected to the male luer portion 414 of the second luer-actuated valve assembly 2302, which leads to a desired treatment site within the body of the patient. It is noted that the fluid flow direction can be established in an opposite direction.

It is appreciated that the first and second medical tubes are adapted to be positioned in a certain treatment area within the patient's body, thus dis-location of one of the medical tubes from its desired position may require replacement of the entire medical set in absence of a connector such as the breakaway intra-medical tubing connector assembly 2000 constructed and operative in accordance with an embodiment of the present invention.

It is noted that compression forces exerted on both luer-actuated valve assemblies 2300 and 2302 by the connector element 2004 are enabled by the fact that both luer-actuated valve assemblies 2300 and 2302 are held in place relative to the breakaway intra-medical tubing connector assembly 2000 due to snap-fit connection between the two snap-fit fittings 2200 and 2202.

It is a particular feature of an embodiment of the present invention that during the connection of both luer actuated valve assemblies 2300 and 2302 to the breakaway intra-medical tubing connector assembly 2000, the two snap-fit fittings 2020 and 2022 are locked with respect to each other by means of the bayonet mechanism 2024, thus axial separation of the two snap-fit fittings 2020 and 2022 is prevented at this operative stage, irrespective of the magnitude of tensile force applied on the breakaway intra-medical tubing connector assembly 2000 along longitudinal axis 2001.

It is particularly seen in Fig.57C that the rearward portion 2242 of the second snap-fit fitting 2022 is at least partially inserted into snap-fit bore portion 2060 of the first snap-fit fitting 2020, such that snap protrusion 2252 of the second snap-fit fitting 2022 is supported forwardly by snap connection 2070 of the first snap-fit fitting 2020. It is specifically seen that the forwardly facing shoulder 2256 of snap protrusion 2252 is supported against rearwardly facing shoulder 2080 of the first snap-fit fitting 2020 and prevents disconnection of the two snap-fit fittings 2020 and 2022.

The breakaway intra-medical tubing connector assembly 2000 is disposable in case the snap protrusion 2252 of the second snap-fit fitting 2022 is deformable, such that additional engagement of snap protrusion 2252 of the second snap-fit fitting 2022 with snap connection 2070 of the first snap-fit fitting 2020 is not intended after first use.

Fluid tight and bacterial sealing is optionally provided between the snap-fit fittings 2020, 2022 and connector element 2004 in this operative orientation due to pressure engagement between inner circumferential surface 2063 of the first snap-fit fitting 2020 and outer surface 2250 of the second snap-fit fitting 2022.

It is noted that alternatively, any other type of luer-actuated valve assembly can be used in conjunction with the breakaway intra-medical tubing connector assembly 2000, constructed and operative in accordance with an embodiment of the present invention.

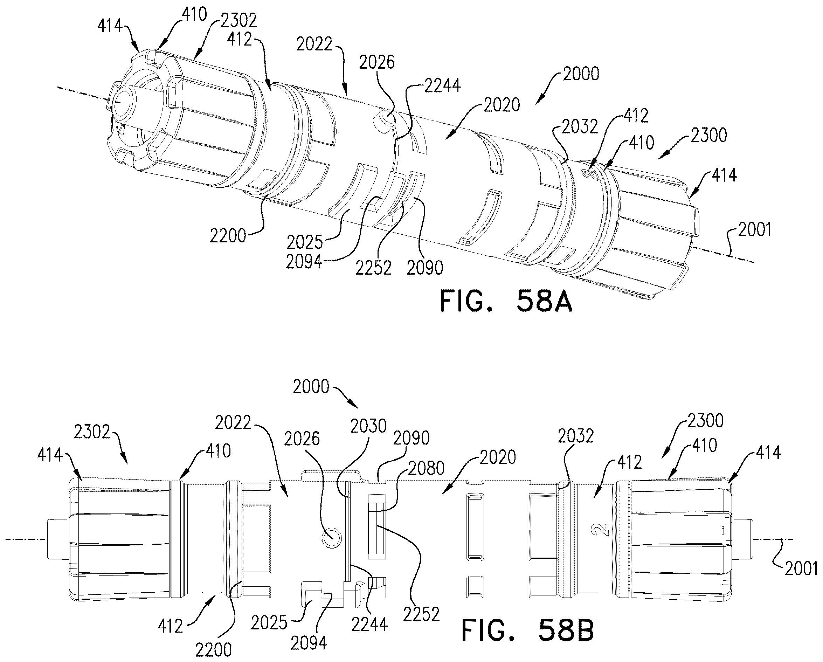

Reference is now made to Figs. 58A and 58B, which are simplified respective perspective view and side view of the breakaway intra-medical tubing connector assembly 2000 of Figs. 55A - 55C, shown in a third operative orientation, when the first and the second luer-actuated valve assemblies 2300 and 2302 of Figs. 56A - 56C are connected to the breakaway intra-medical tubing connector assembly 2000, which is shown in an unlocked operative position.

It is noted that all spatial relationships between the different components of the breakaway intra-medical tubing connector assembly 2000 in this third operative orientation are similar to that described with reference to Figs. 57A - 57C, other than the fact that the two snap-fit fittings 2020 and 2022 are now disposed in an unlocked operative orientation. Once the luer-actuated valve assemblies 2300 and 2302 are connected to the breakaway intra-medical tubing connector assembly 2000, the two snap-fit fittings 2020 and 2022 are rotated with respect to each other about axis 2001, thus removing the safety pins 2026 from the openings 2094 of the locking members 2025 and subsequently unlocking the snap-fit fittings 2020 and 2022 from each other.

It is a particular feature of an embodiment of the present invention that in this unlocked operative position, the first snap-fit fitting 2020 is disconnectably snap-fittingly connected to the second snap fit fitting 2022 and the connector element 2004 is inserted through the through bores 2034 and 2204 of the respective first and second snap fit fittings 2020 and 2022. It is noted that each of the snap-fit fittings 2020 and 2022 can be disconnected from each other in response to tensile force exerted on one of the snap-fit fittings 2020 and 2022 along longitudinal axis 2001. Specifically, as described with reference to Fig. 55C, the snap connection 2252 of the second snap-fit fitting 2022 is disconnectably coupled with snap connection 2070 of the first snap fit fitting 2020, such that snap connection 2252 of the second snap fit fitting 2022 is disposed within each of the openings 2090 of the first snap fit fitting 2020. Specifically, the forwardly facing shoulder 2256 of snap connection 2252 is supported against rearwardly facing shoulders 2080, which defines the boundary of openings 2090. The snap-fit fittings 2020 and 2022 are disconnectable coupled to each other in a snap-fit manner as long as a predetermined tensile force threshold is not applied on one of the snap-fit fittings 2020 and 2022. The tensile force threshold is preferably defined in the range of 0.2 - 5kgf.

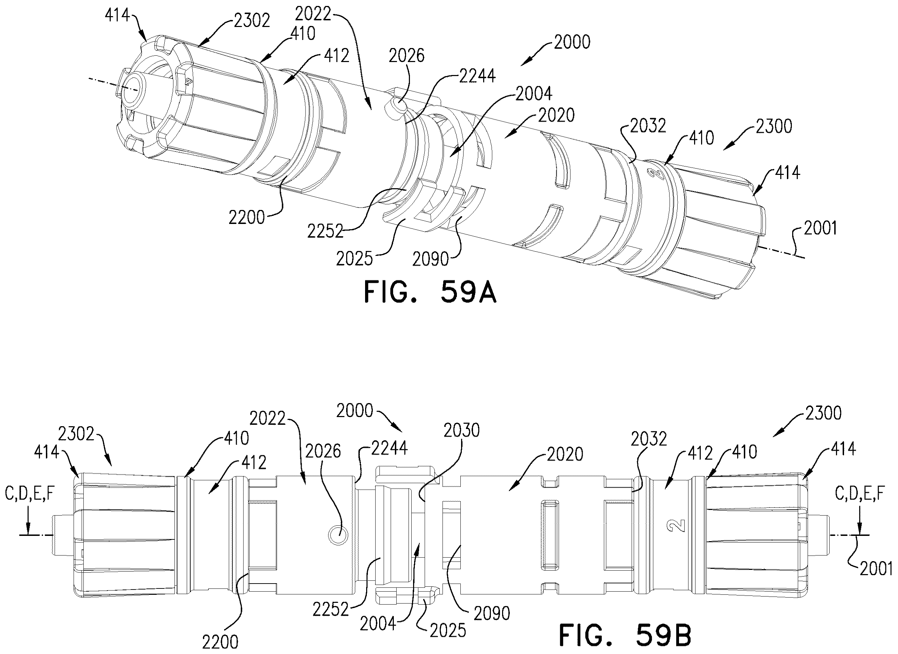

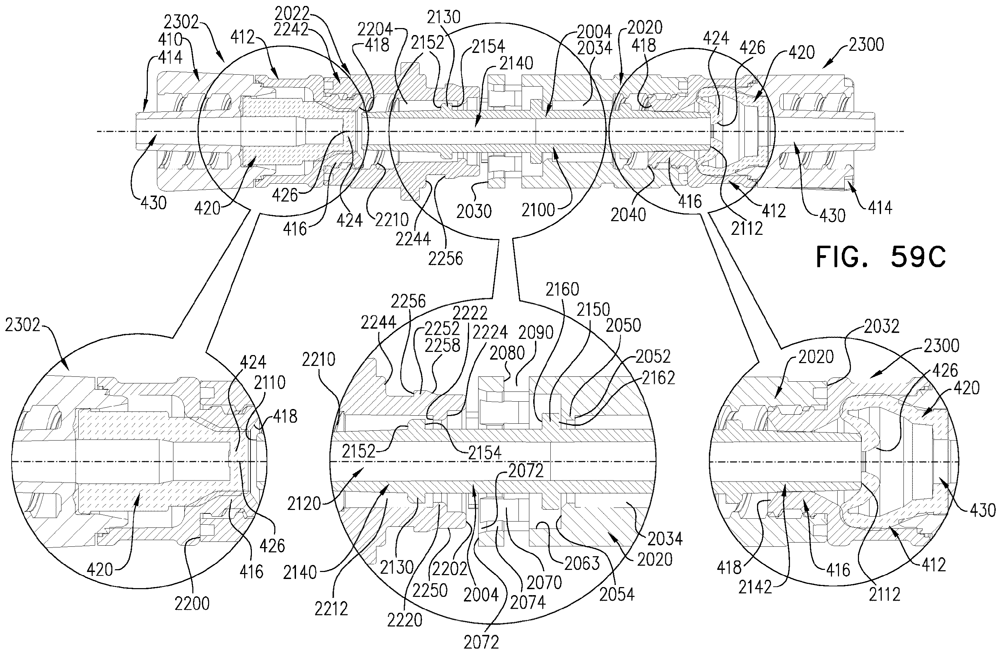

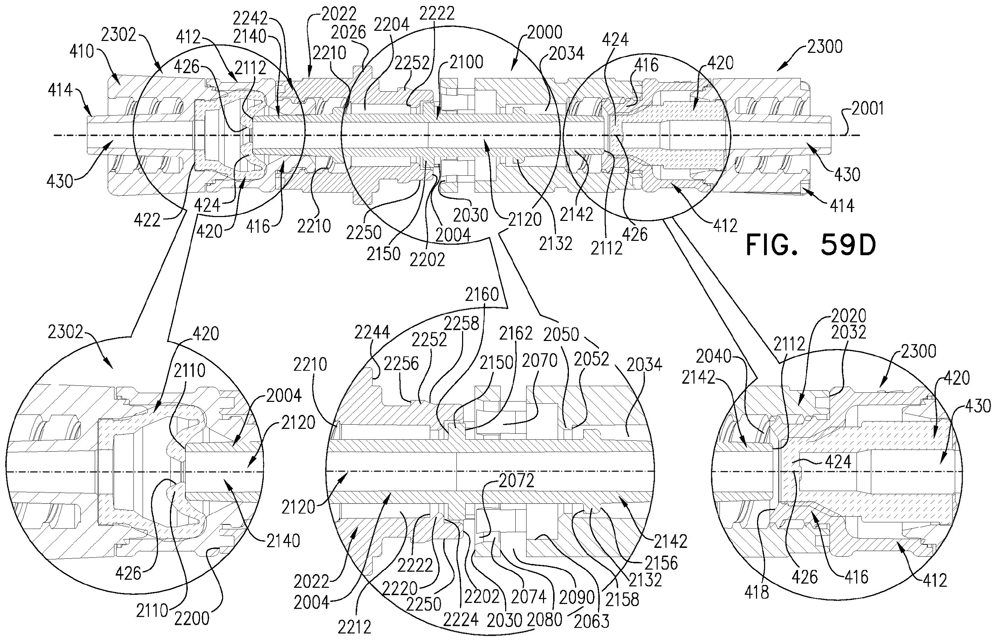

Reference is now made to Figs. 59A and 59B, which are simplified respective perspective view and side view of the breakaway intra-medical tubing connector assembly 2000 of Figs. 55A - 55C, shown in a fourth operative orientation, when the first and the second luer-actuated valve assemblies 2300 and 2302 of Figs. 56A - 56C are connected to the breakaway intra-medical tubing connector assembly 2000, which is shown in an intermediate stage of disconnection. Reference is additionally made to Figs. 59C and 59D, which are simplified sectional illustrations of the breakaway intra-medical tubing connector assembly 2000 of Figs. 59A and 59B, sections taken along respective lines C - C and D - D in Fig. 59B, when the breakaway intra-medical tubing connector assembly 2000 is shown in a first intermediate stage of disconnection.

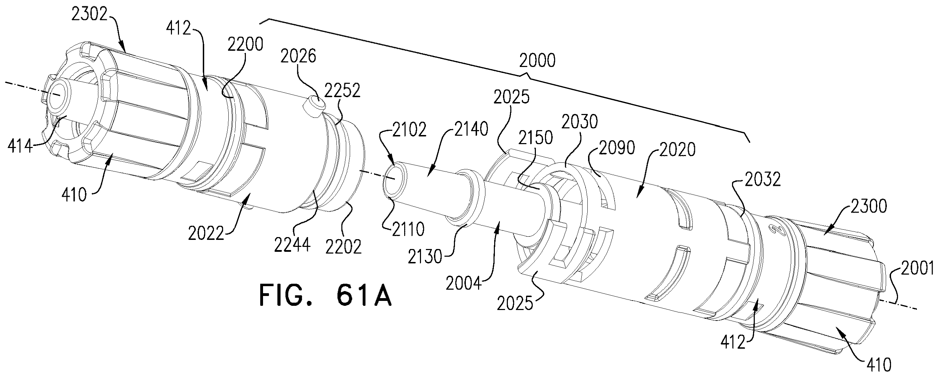

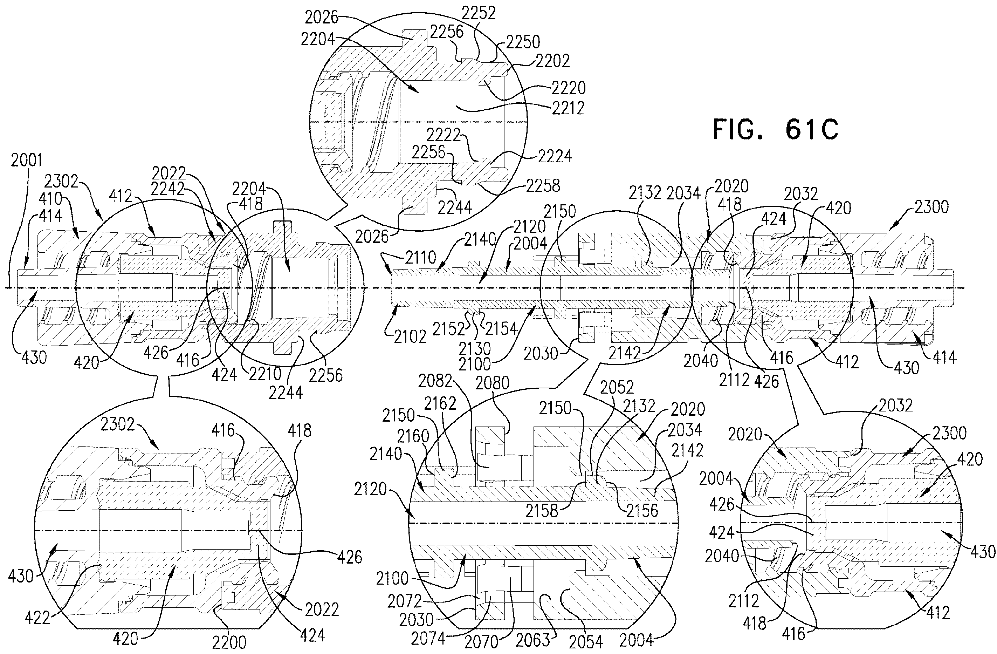

It is seen in this first intermediate stage of disconnection of the breakaway intra-medical tubing connector assembly 2000 that the snap connection between the two snap-fit fittings 2020 and 2022 is broken, but the connector element 2004 is not yet pulled out of both luer-actuated valve assemblies 2300 and 2302. It is noted that this first intermediate stage of disconnection is a momentary stage and upon further application of tensile force by the user along longitudinal axis 2001, the breakaway intra-medical tubing connector assembly 2000 subsequently assumes a disconnected orientation, as is described in detail hereinbelow with reference to Figs. 61A - 61C.

It is seen in Fig. 59C that the snap connection between the two snap-fit fittings 2020 and 2022 is broken and that the connector element 2004 is fully pulled out of the second luer actuated valve 2302, but is still engaged with and partially inserted into the first luer-actuated valve 2300.

It is seen in Fig. 59D that the snap connection between the two snap-fit fittings 2020 and 2022 is broken and that the connector element 2004 is fully pulled out of the first luer actuated valve 2300, but is still engaged with and partially inserted into the second luer-actuated valve 2302.

The first intermediate stage of disconnection might be any one of the illustrated in Fig. 59C or 59D, depends on the friction forces created between the tubular portions 2140 and 2142 of the connector element 2004 and the female luer portions 412 of the respective luer-actuated valves 2300 and 2302.

It is particularly seen in Figs. 59A - 59D that upon application of tensile force on either of the snap-fit fittings 2020, 2022, the luer-actuated valve assemblies 2300, 2302 or the medical tubes coupled to one of the luer actuated assemblies 2300, 2302, the snap-fit fittings 2020 and 2022 are disconnected from each other. It is seen that the luer-actuated valve assemblies 2300 and 2302 are threadably connected to the respective snap-fit fittings 2020 and 2022, thus cannot be disconnected therefrom upon application of tensile force, however the snap fit connection provided between the second snap-fit fitting 2022 and the first snap-fit fitting 2022 can be broken upon application of sufficient tensile force. According to an embodiment of the present invention, tensile force in the range of 0.2kgf - 5kgf is required in order to break the snap-fit connection between the snap-fit fittings 2022 and 2020.

It is specifically seen in Figs. 59C & 59D that upon application of tensile force upon one of the snap fit-fittings 2020 or 2022 along longitudinal axis 2001, the snap protrusion 2252 of the second snap-fit fitting 2022 is disengaged from the snap connection 2070 of the first snap-fit fitting 2020, such that shoulder 2256 of the snap protrusion 2252 disengages rearwardly facing shoulder 2080 of the snap connection 2070 due to the relative resiliency of the snap-fit fittings 2020 and 2022 provided by the material it is formed from and preferably also due to the existence of longitudinal grooves 2082 and by openings 2090, as described in detail hereinabove.

It is particularly seen in Figs.59A - 59D that the rearward portion 2242 of the second snap-fit fitting 2022 is now removed from the snap-fit bore portion 2060 of the first snap-fit fitting 2020, thus the two snap-fit fittings 2020 and 2022 are disconnected.

It is a particular feature of an embodiment of the present invention that upon application of sufficient tensile force on one of the snap-fit fittings 2020 or 2022, the connector element 2004 is pulled out of one of the luer-actuated valve assemblies 2300 and 2302 due to the friction force between the other one of the luer-actuated valve assemblies 2300 and 2302 and the connector element 2004, which holds the other end of the connector element 2004 at least partially inserted into the luer actuated valve assembly 2300 or 2302. Specifically, the friction force created between the opposite one of the tubular portions 2140 and 2142 of the connector element 2004 that is at least partially inserted into one of the female portions 412 of the luer actuated valve assemblies 2300 and 2302.

Specifically, there is a three-step disconnection process of the breakaway intra-medical tube connector assembly 2000 upon application of a sufficient tensile force by the user on one of the snap-fit fittings 2020 and 2022:
Firstly, the two snap-fit fittings 2020 and 2022 are disconnected by breaking the snap connection between snap connection 2070 and snap protrusion 2252, thereby providing a visual indication for the user that the medical tubes connected to the respective male luer portions 414 of the luer actuated valve assemblies 2300 and 2302 are now disconnected.
Secondly, further application of the tensile force by the user urges axial displacement of the connector element 2004 relative to one of the snap-fit fittings 2020 and 2022 along longitudinal axis 2001, such that one of the tubular portions 2140 and 2142 of the connector element 2004 is pulled out of the respective luer-actuated valve 2300 and 2302 due to the friction forces created between the opposite ones of tubular portions 2140 and 2142 and the luer -actuated valves 2300 and 2302. The connector element 2004 is relatively displaced axially along axis 2001 up to engagement of one of the end protrusions 2130 or 2132 of the connector element 2004 with one of the respective inner flanges 2220 or 2050 of the respective snap-fit fittings 2022 and 2020.
Thirdly, upon engagement of one of the end protrusions 2130 or 2132 of the connector element 2004 with one of the respective inner flanges 2220 or 2050 of the respective snap-fit fittings 2022 and 2020, the connector element 2004 is urged to be displaced axially along longitudinal axis 2001 relative to the other snap-fit fitting 2020 and 2022 up to engagement of another one of the end protrusions 2130 or 2132 of the connector element 2004 with another one of the respective inner flanges 2220 or 2050 of the respective snap-fit fittings 2022 and 2020, thus urging the connector element 2004 out of the other one of the luer-actuated valve assemblies 2300 and 2302.

It is noted that the first and second disconnection steps are illustrated and described with reference to Figs. 59A - 59F.

It is particularly seen in Fig. 59C that the first tubular portion 2140 of the connector element 2004 is pulled out first from the second luer-actuated valve assembly 2302 upon application of tensile force by the user and the second tubular portion 2142 of the connector element 2004 is still at least partially inserted into the female luer portion 412 of the first luer actuated valve assembly 2300 and engaged with the sealing element 420 of the first luer actuated valve assembly 2300. The first tubular portion 2140 of the connector element 2004 is pulled out of the second luer-actuated valve assembly 2302 upon axial displacement of the second snap-fit fitting 2022 relative to the connector element 2004, due to friction forces created between tubular portion 2142 of the connector element 2004 and female portion 412 of the first luer actuated valve assembly 2300, which hold the tubular portion 2142 within the first luer actuated valve assembly 2300.

It is noted that in this case, as illustrated in Fig. 59C, the friction forces between tubular portion 2142 of the connector element 2004 and between female portion 412 of the first luer actuated valve assembly 2300 are higher than the frictional forces between tubular portion 2140 of the connector element 2004 and between female portion 412 of the second luer actuated valve assembly 2302, thus the tubular portion 2140 is the first one to be pulled out from its respective luer actuated valve assembly 2302 once overcoming the friction forces between the tubular portion 2140 and luer actuated valve assembly 2302.

It is seen particularly in Fig. 59C that end protrusion 2130 of the connector element 2004 does not yet engage flange 2220 of the second snap-fit fitting 2022. Particularly, end protrusion 2130 is slightly forwardly spaced from flange 2220.

Alternatively, the second tubular portion 2142 of the connector element 2004 may be pulled out first from the first luer-actuated valve assembly 2300 upon application of tensile force by the user, as is particularly seen in Fig. 59D. In this case, the first tubular portion 2140 of the connector element 2004 is still at least partially inserted into the female luer portion 412 of the second luer actuated valve assembly 2302 and engaged with the sealing element 420 of the second luer actuated valve assembly 2302. The second tubular portion 2142 of the connector element 2004 is pulled out of the first luer-actuated valve assembly 2300 upon axial displacement of the first snap-fit fitting 2020 relative to the connector element 2004 due to friction forces created between tubular portion 2140 of the connector element 2004 and female portion 412 of the second luer actuated valve assembly 2302, which hold the tubular portion 2140 within the second luer actuated valve assembly 2302.

It is noted that in this case, as illustrated in Fig. 59D, the friction forces between tubular portion 2140 of the connector element 2004 and between female portion 412 of the second luer actuated valve assembly 2302 are higher than the frictional forces between tubular portion 2142 of the connector element 2004 and between female portion 412 of the first luer actuated valve assembly 2302, thus the tubular portion 2142 is the first one to be pulled out from its respective luer actuated valve assembly 2300 once overcoming the friction forces between the tubular portion 2142 and luer actuated valve assembly 2300.

It is seen particularly in Fig. 59D that end protrusion 2132 of the connector element 2004 does not yet engage flange 2050 of the first snap-fit fitting 2020. Particularly, end protrusion 2132 is slightly forwardly spaced from flange 2050.

It is noted that additionally, upon disconnection of the snap-fit fittings 2020 and 2022, the connector element 2004 is urged to be displaced axially along longitudinal axis 2001 and out of the luer-actuated valve assembly 2300 due to the biasing force of the sealing element 420 of the first luer-actuated valve assembly 2300, that is applied upon the connector element 2004. Similarly, the connector element 2004 is urged to be displaced axially along longitudinal axis 2001 and out of the second luer-actuated valve assembly 2302, due to the biasing force of the sealing element 420 of the second luer-actuated valve assembly 2302 that is applied upon the connector element 2004. This biasing force ensures that the sealing element 420 returns to its normally closed operative orientation, whereas the selectively closeable slit 426 is closed. It is noted that the biasing force is relatively low and the friction forces between the connector element 2004 and the female portion 412 of the luer actuated valve assembly provide a contra thereto.

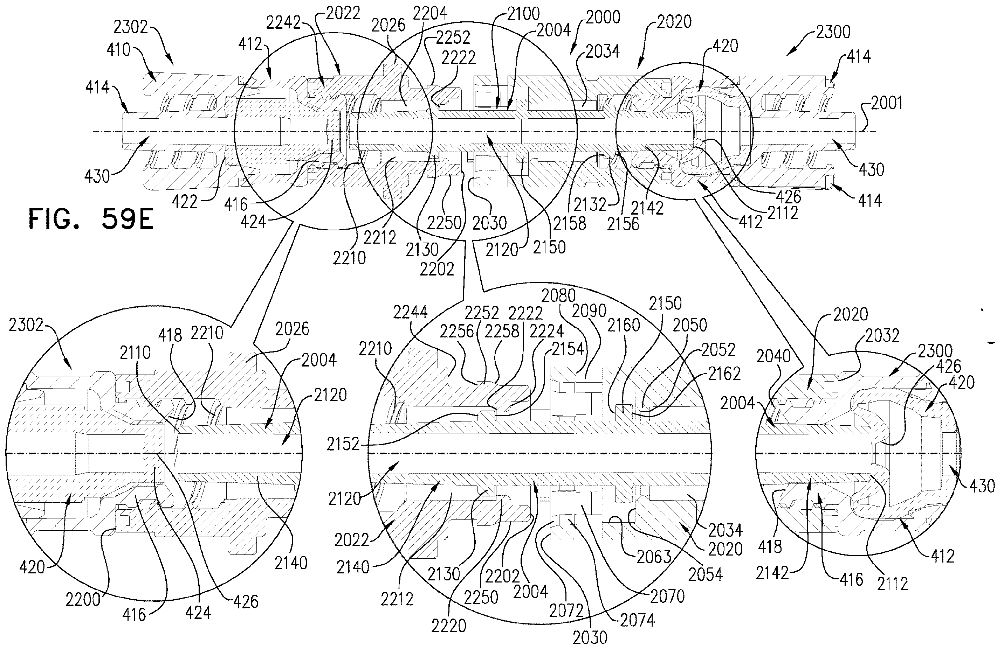

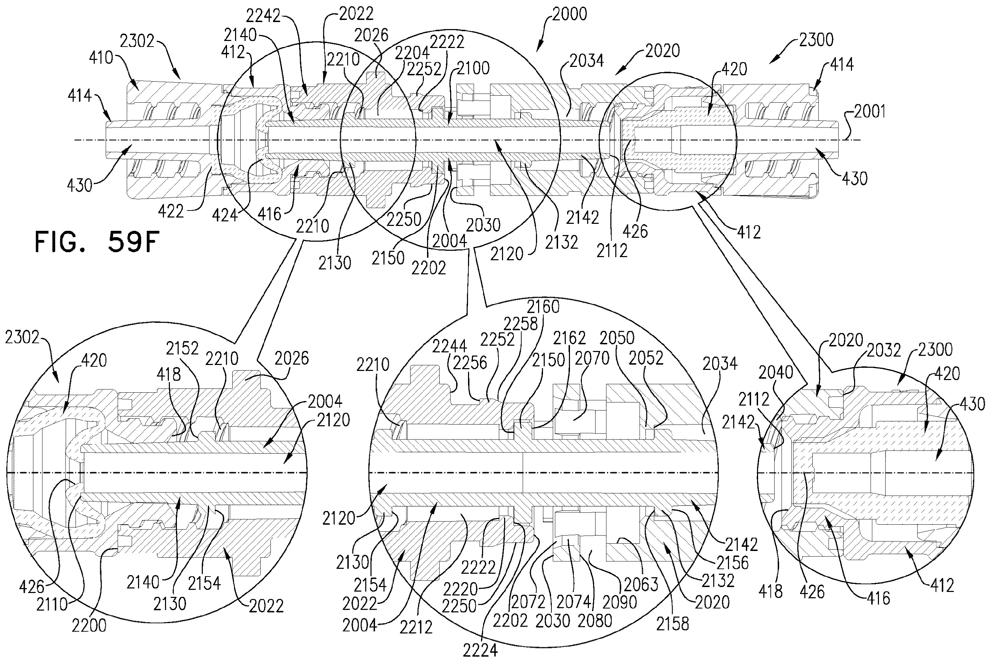

Reference is now made to Figs. 59E and 59F, which are simplified sectional illustrations of the breakaway intra-medical tubing connector assembly 2000 of Figs. 59A and 59B, sections taken along respective lines E - E and F - F in Fig. 59B, when the breakaway intra-medical tubing connector assembly 2000 is shown in a second intermediate stage of disconnection.

It is noted that all spatial relationships between the different components of the breakaway intra-medical tubing connector assembly 2000 and the luer actuated valve assemblies 2300 and 2302 remain generally identical to the relationships described with reference to Figs. 59C and 59D as shown in the first intermediate stage of disconnection, other than the following:
Upon further application of tensile force on one of the snap-fit fittings 2020 and 2022, there is a further relative axial displacement between one of the snap-fit fittings 2020 and 2022 and between the connector element 2004.

It is seen particularly in Fig. 59E that end protrusion 2130 of the connector element 2004 now engages flange 2220 of the second snap-fit fitting 2022 and is supported against it, such that further relative axial displacement between the connector element 2004 and the second snap-fit fitting 2022 is inhibited due to radial overlap between the flange 2220 and the end protrusion 2130, and upon further application of tensile force the connector element 2004 is urged to be axially displaced relative to the first snap-fit fitting 2020. Particularly, the rearwardly facing shoulder 2154 of end protrusion 2130 engages forwardly facing shoulder 2222 of flange 2220.

It is seen particularly in Fig. 59F that end protrusion 2132 of the connector element 2004 now engages flange 2050 of the first snap-fit fitting 2020 and is supported against it, such that further relative axial displacement between the connector element 2004 and the first snap-fit fitting 2022 is inhibited due to radial overlap between the flange 2050 and the end protrusion 2132, and upon further application of tensile force the connector element 2004 is urged to be axially displaced relative to the second snap-fit fitting 2022. Particularly, the forwardly facing shoulder 2158 of end protrusion 2132 engages rearwardly facing shoulder 2052 of flange 2050.

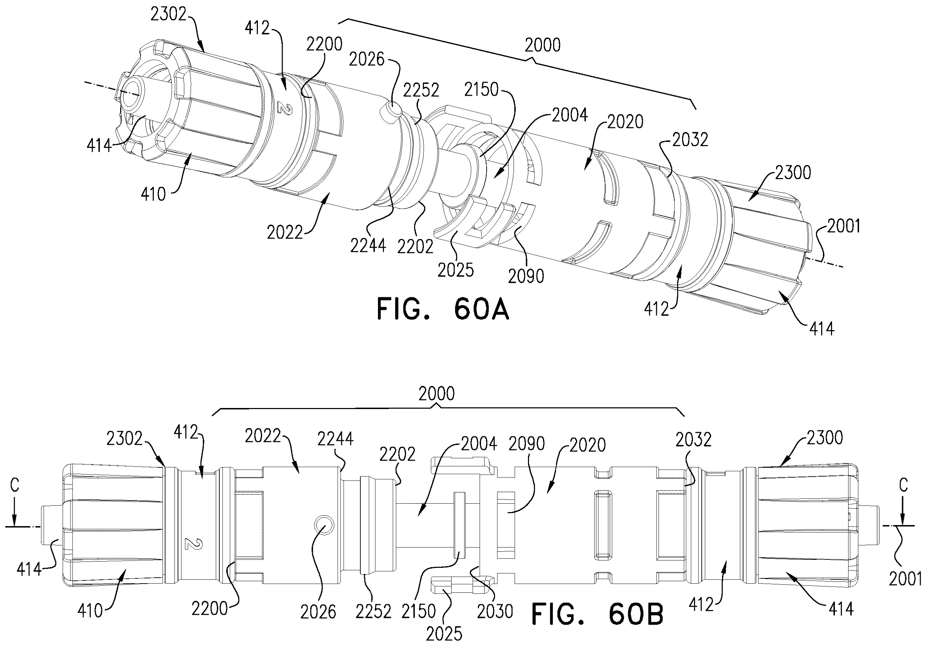

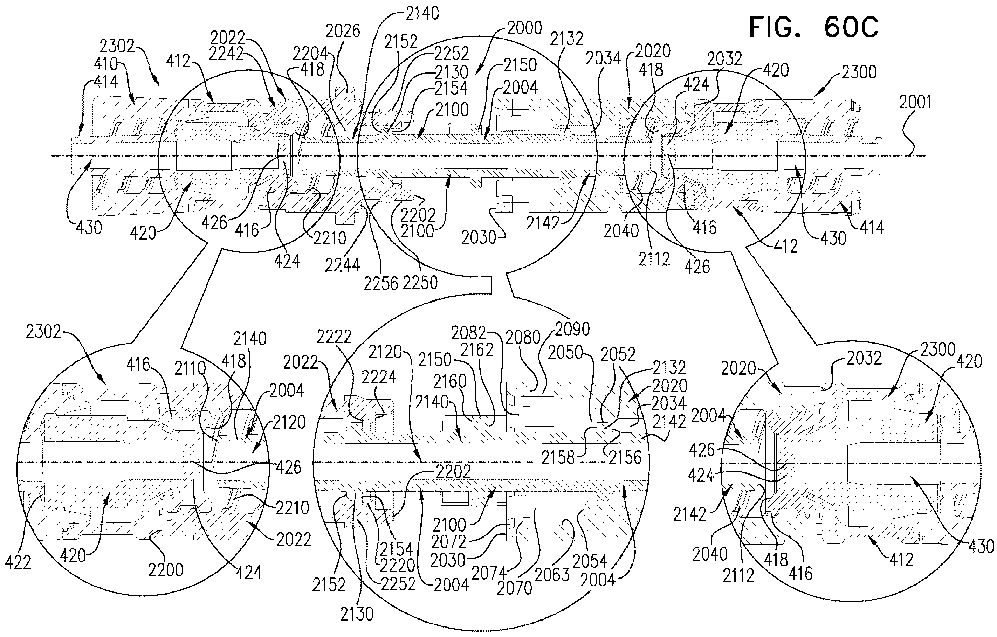

Reference is now made to Figs. 60A and 60B, which are simplified respective perspective view and side view of the breakaway intra-medical tubing connector assembly 2000 of Figs. 55A - 55C, shown in a fifth operative orientation, when the first and the second luer-actuated valve assemblies 2300 and 2302 of Figs. 56A - 56C are connected to the breakaway intra-medical tubing connector assembly 2000, which is shown in an intermediate stage just prior to disconnection. Reference is additionally made to Fig. 60C, which is a simplified sectional illustration of the breakaway intra-medical tubing connector assembly 2000 of Figs. 60A and 60B, section taken along lines C - C in Fig. 60B, when the breakaway intra-medical tubing connector assembly 2000 is shown in an operative orientation just prior to disconnection.

It is seen in this prior to disconnection orientation of the breakaway intra-medical tubing connector assembly 2000 that the connector element 2004 is now pulled out of both luer-actuated valve assemblies 2300 and 2302. It is noted that this is also a momentary stage and upon further application of tensile force by the user along longitudinal axis 2001, the breakaway intra-medical tubing connector assembly 2000 subsequently assumes a disconnected orientation, as is described in detail hereinbelow with reference to Figs. 61A - 61C.

It is noted that the third disconnection step is illustrated and described with reference to Figs. 60A - 60C.

It is a particular feature of an embodiment of the present invention that upon application of sufficient tensile force on one of the snap-fit fittings 2020 or 2022, the connector element 2004 is urged out of the other one of the luer-actuated valve assemblies 2300 and 2302 by means of engagement of one of the end protrusions 2130 and 2132 with one of the respective flanges 2050 and 2220. Upon further application of tensile force, relative axial displacement is provided between the connector element 2004 and one of the snap-fit fittings 2020 and 2022 up to engagement between the other one of end protrusions 2130 and 2132 with another one of the respective flanges 2050 and 2220. The engagement between the first one of the end protrusions 2130 and 2132 with the respective first one of the flanges 2050 and 2220 is described hereinabove with reference to Figs. 59E and 59F. Once both end protrusion 2132 engages flange 2050 and end protrusion 2130 engages flange 2220, the connector element 2004 is pulled out of both of the luer actuated valve assemblies 2300 and 2302.

It is particularly seen in Fig. 60C that in case the first and second intermediate stages are as illustrated and described with reference to Figs. 59C and 59E, upon further application of tensile force along axis 2001 by the user, the connector element 2004 is axially displaced relative to the first snap-fit fitting 2020 up to engagement between end protrusion 2132 of the connector element 2004 with flange 2050 of the first snap-fit fitting 2020, particularly engagement of forwardly facing shoulder 2158 of end protrusion 2132 with rearwardly facing shoulder 2052 of flange 2050, which in turn pulls the second tubular portion 2142 of the connector element 2004 out of the first luer-actuated valve assembly 2300.

It is particularly seen in Fig. 60C that in case the first and second intermediate stages are as alternatively illustrated and described with reference to Figs. 59D and 59F, upon further application of tensile force along axis 2001 by the user, the connector element 2004 is axially displaced relative to the second snap-fit fitting 2022 up to engagement between end protrusion 2130 of the connector element 2004 with flange 2220 of the second snap-fit fitting 2022, particularly engagement of rearwardly facing shoulder 2154 of end protrusion 2130 with forwardly facing shoulder 2222 of flange 2220, which in turn pulls the first tubular portion 2140 of the connector element 2004 out of the second luer-actuated valve assembly 2302.

It is a further particular feature of an embodiment of the present invention that the inner diameter of the flange 2220 is slightly smaller than the outer diameter of the first annular end protrusion 2130 and inner diameter of the flange 2050 is slightly smaller than the outer diameter of the second annular end protrusion 2132, thus upon engagement of respective end protrusions 2130, 2132 with the respective flanges 2220 and 2050, the tubular portion 2140 of the connector element 2004 is pulled out of the second luer actuated valve assembly 2302 and tubular portion 2142 of the connector element 2004 is pulled out of the first luer actuated valve assembly 2300.

It is noted that in this prior to disconnection orientation, the connector element 2004 remains partially within both snap-fit fittings 2020 and 2022, whereas end protrusion 2130 of the connector element 2004 engages flange 2220 of the second snap-fit fitting 2022 and end protrusion 2132 of the connector element 2004 engages flange 2050 of the first snap-fit fitting 2020, just prior to full disconnection of the breakaway intra-medical tubing connector assembly 2000.

It is noted that additionally, upon disconnection of the snap-fit fittings 2020 and 2022, the connector element 2004 is urged to be displaced axially along longitudinal axis 2001 and out of the luer-actuated valve assembly 2300 due to the biasing force of the sealing element 420 of the first luer-actuated valve assembly 2300, that is applied upon the connector element 2004. Similarly, the connector element 2004 is urged to be displaced axially along longitudinal axis 2001 and out of the second luer-actuated valve assembly 2302, due to the biasing force of the sealing element 420 of the second luer-actuated valve assembly 2302 that is applied upon the connector element 2004. This biasing force ensures that the sealing element 420 returns to its normally closed operative orientation, whereas the selectively closeable slit 426 is closed. It is noted that the biasing force is relatively low and the friction forces between the connector element 2004 and the female portion 412 of the luer actuated valve assembly provide a contra thereto.

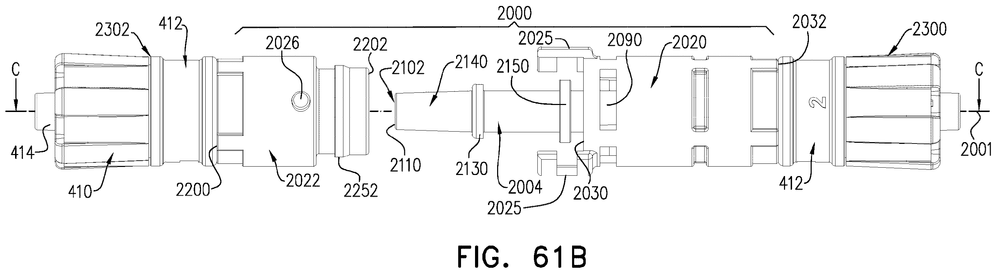

Reference is now made to Figs. 61A - 61C, which are simplified respective perspective view, side view and a sectional illustration taken along lines C - C in Fig. 61B of the breakaway intra-medical tubing connector assembly 2000 of Figs. 55A - 55C, shown in a sixth operative orientation, when the breakaway intra-medical tubing connector assembly 2000 is shown in a disconnected orientation.

It is seen in Figs. 61A - 61C that upon application of a sufficient tensile force by the user along axis 2001, the first tubular portion 2140 of the connector element 2004 is entirely removed from the second snap-fit fitting 2022, as end protrusion 2130 of the connector element 2004 is snapped over the flange 2220 of the second snap-fit fitting 2022.

It is noted that alternatively, the second tubular portion 2142 of the connector element 2004 can be removed entirely from the first snap-fit fitting 2020.

It is noted that there is a minimal radial overlap engagement between flange 2220 and first annular end protrusion 2130 and a minimal radial overlap engagement between flange 2050 and second annular end protrusion 2132, and application of sufficient tensile force overcomes one of these engagements, thus the connector element 2004 is completely removed from one of the snap-fit fittings 2020 and 2022. In this particular example, the connector element 2004 is removed from the second snap-fit fitting 2022 and the second annular end protrusion 2132 remains adjacent flange 2050 of the first snap-fit fitting 2020, thus preventing the connector element 2004 from falling out from the breakaway intra-medical tubing connector assembly 2000.

It is a particular feature of an embodiment of the present invention that preferably one of the inner flanges 2220 and 2050 has a smaller inner diameter than the other, such that it is pre-defined which side of the connector element 2004 is disconnected first from its respective luer-actuated valve assembly 2300 or 2302. If the inner diameter of flange 2220 is smaller than the inner diameter of flange 2050, then the connector element 2004 is removed entirely from the first snap-fit fitting 2020. Alternatively, if the inner diameter of flange 2050 is smaller than the diameter of inner flange 2220, then the connector element 2004 is removed entirely from the second snap-fit fitting 2022.

Alternatively, both inner flanges 2220 and 2050 may have the same diameter.

It is a particular feature of an embodiment of the present invention that upon disconnection of the two snap-fit fittings 2020 and 2022 from each other and subsequent removal of the connector element 2004 out of both luer-actuated valve assemblies 2300 and 2302, both of the luer-actuated valve assemblies 2300 and 2302 are automatically bidirectionally closed, thus preventing fluid flow passage from each of the two medical tubes. Upon discarding of the breakaway intra-medical tubing connector assembly 2000, two sealed and swabbale luer-actuated valve assemblies 2300 and 2302 are provided, which obviate the need to replace the entire medical set, and only requires replacement of the breakaway intra-medical tubing connector assembly 2000.

In this third operative orientation, as seen in this particular example, the first tubular portion 2140 of the connector element 2004 protrudes outwardly from the first snap-fit fitting 2020 and the second tubular portion 2142 of the connector element 2004 is fully surrounded by the first snap-fit fitting 2020.

The first snap-fit fitting 2020 can be threadably disengaged from the first luer-actuated valve assembly 2030 and the second snap-fit fitting 2022 can be threadably disengaged from the second luer-actuated valve assembly 2302, thus leaving the luer-actuated valve assemblies 2300 and 2302 along with the medical tubes associated therewith within the desired treatment location. Following disconnection of the snap-fit fittings 2020 and 2022 from the first and second luer-actuated valve assemblies 2300 and 2302, the valve assemblies are swabbable and can be cleaned by the user and subsequently ready to be used again with another breakaway intra-medical tubing connector assembly 2000.

It is noted that tensile force exerted on the medical set such as IV line may be unintentional, but due to the presence of the breakaway intra-medical tubing connector assembly 2000, the catheter or other medical tube associated with one of the luer-actuated valve assemblies 2300 and 2302 remains in its position within the treatment site and the luer-actuated valve assemblies 2300 and 2302 are safely sealed once the snap-fit fittings 2020 and 2022 are disconnected from each other. Therefore, no fluid can unintentionally flow out of the IV line and risk of contamination of the treatment site is prevented due to sealing of the luer-actuated valve assemblies 2300 and 2302 and the ability to clean the exterior surface of the sealing element 420 remains.

It is a particular feature of an embodiment of the present invention that the breakaway intra-medical tubing connector assembly 2000 associated with medical connectors, such as luer-actuated valve assemblies 2300 and 2302 enables safe unintentional disconnection of one of the luer-actuated valve assemblies.

It will be appreciated by persons skilled in the art that the present invention is not limited by what has been particularly shown and described hereinabove. Rather the scope of the present invention includes both combinations and subcombinations of various features described hereinabove as well as variations and modifications thereof which are not in the prior art.
Aspects of the present invention include:
Inventive concept 1. An automatically bidirectionally-sealable breakaway intra-medical tubing connector assembly configured for use with first and second luer-actuated valve assemblies connected to opposite intermediate ends of a medical tube, said connector assembly comprising:
   a connector body, having first and second ends and arranged for tensile force responsive disconnectable snap fit connection with at least one of said first and second luer actuated valve assemblies, which connection provides opening of said first and second luer actuated valve assemblies; and
   a connector element, having first and second ends and defining a fluid flow path therethrough, slidably located within said connector body and being configured to permit automatic closing of both said first and second luer actuated valve assemblies upon disconnection of at least one of said first and second luer actuated valve assemblies from said connector body.
Inventive concept 2. An automatically bidirectionally-sealable breakaway intra-medical tubing connector assembly according to inventive concept 1 and also comprising at least one snap-fit fitting adapted to be connected to said at least one of first and second luer-actuated valve assembly and being arranged for tensile force responsive disconnectable snap fit connection with one of first or second ends of said connector body.
Inventive concept 3. An automatically bidirectionally-sealable breakaway intra-medical tubing connector assembly according to inventive concept 1 and also comprising two snap-fit fittings, each being adapted to be connected to said at least one of first and second luer-actuated valve assemblies and being arranged for tensile force responsive disconnectable snap fit connection with one of first or second ends of said connector body.
Inventive concept 4. An automatically bidirectionally-sealable breakaway intra-medical tubing connector assembly according to any of inventive concepts 1 - 3 and wherein said connector element is configured to have first and second operative orientations with respect to said first and second luer-actuated valve assemblies:
   said first operative orientation in which said first and second ends of said connector element both openingly engage said respective first and second luer-actuated valve assemblies, said first operative orientation existing when both of said first and second luer-actuated valve assemblies are in snap fit connection with said connector body;
   said second operative orientation in which neither of said first and second ends of said connector element openingly engage said respective first and second luer-actuated valve assemblies, said second operative orientation existing when at least one of said first and second luer-actuated valve assemblies is disconnected from said connector body.
Inventive concept 5. An automatically bidirectionally-sealable breakaway intra-medical tubing connector assembly according to any of inventive concepts 2 - 4 and wherein said connector element is configured to have first and second operative orientations with respect to said first and second luer-actuated valve assemblies:
   said first operative orientation in which said first and second ends of said connector element both openingly engage said respective first and second luer-actuated valve assemblies, said first operative orientation existing when both of said first and second snap-fit fittings are in snap fit connection with said connector body;
   said second operative orientation in which neither of said first and second ends of said connector element openingly engage said respective first and second luer-actuated valve assemblies, said second operative orientation existing when at least one of said first and second snap-fit fittings is disconnected from said connector body.
Inventive concept 6. An automatically bidirectionally-sealable breakaway intra-medical tubing connector assembly according to inventive concept 4 or inventive concept 5 and wherein in said second operative orientation, both of said first and second luer-actuated valve assemblies are in a closed and sealed operative orientation.
Inventive concept 7. An automatically bidirectionally-sealable breakaway intra-medical tubing connector assembly according to any of inventive concepts 1 - 6 and wherein said connector element is a double-sided male connector, having first and second conical connector ends.
Inventive concept 8. An automatically bidirectionally-sealable breakaway intra-medical tubing connector assembly according to any of inventive concepts 1 - 7 and wherein said medical tube is selected from the group consisting of a catheter, an IV line and a drain.
Inventive concept 9. An automatically bidirectionally-sealable breakaway intra-medical tubing connector assembly according to any of inventive concepts 1 - 8 and wherein a radially inwardly extending protrusion is formed on an inner surface of said connector body and a radially outwardly extending protrusion is formed on an outer surface of the connector element, and said connector element is freely slidable within said connector body up to engagement of said radially outwardly extending protrusion with said radially inwardly extending protrusion.
Inventive concept 10. An automatically bidirectionally-sealable breakaway intra-medical tubing connector assembly according to inventive concept 1 and wherein a first snap connection is formed on an outer surface of said connector body, adjacent said first end thereof and a second snap connection is formed on the outer surface of said connector body, adjacent said second end thereof.
Inventive concept 11. An automatically bidirectionally-sealable breakaway intra-medical tubing connector assembly according to inventive concept 10 and wherein said snap-fit fitting is adapted to be connected to said first or second snap connection and arranged for tensile force responsive disconnection from said first or second snap connection.
Inventive concept 12. An automatically bidirectionally-sealable breakaway intra-medical tubing connector assembly according to inventive concept 2 and wherein said connector element is axially slidable with respect to both said connector body and said at least one snap-fit fitting.
Inventive concept 13. An automatically bidirectionally-sealable breakaway intra-medical tubing connector assembly according to inventive concept 2 and wherein at least one of said first or second luer-actuated valve assembly is configured for threadable connection to said at least one snap-fit fitting.
Inventive concept 14. An automatically bidirectionally-sealable breakaway intra-medical tubing connector assembly according to any of inventive concepts 1 - 13 and wherein the inner diameter of a fluid flow passage provided between said first and second luer-actuated valve assemblies is substantially the same as the diameter of a thoroughgoing bore formed within said connector element.
Inventive concept 15. An automatically bidirectionally-sealable breakaway intra-medical tubing connector assembly according to any of inventive concepts 1 - 14 and wherein each of said first and second luer-actuated valve assemblies includes a compressible sealing element having a first open end and a second selectably openable end, including a selectively openable slit and wherein said sealing element is biased to its normally closed position when no compression force is exerted thereon.
Inventive concept 16. An automatically bidirectionally-sealable breakaway intra-medical tubing connector assembly according to inventive concept 15 and wherein upon engagement of said first and second luer-actuated valve assemblies with said breakaway intra-medical tubing connector assembly, said first end of said connector element compresses said sealing element of said first luer-actuated valve assembly and said second end of said connector element compresses said sealing element of said second luer-actuated valve assembly, thereby opening said respective selectively openable slits and establishing fluid flow passage between said first and second luer-actuated valve assemblies.
Inventive concept 17. An automatically bidirectionally-sealable breakaway intra-medical tubing connector assembly according to any of inventive concepts 2 - 16 and wherein said at least one snap-fit fitting is freely rotatable about said connector body.
Inventive concept 18. An automatically bidirectionally-sealable breakaway intra-medical tubing connector assembly according to any of inventive concepts 1 - 17 and wherein tensile force in the range of 0.5kgf - 3kgf is required in order to disconnect said luer-actuated valve assembly from said connector body.
Inventive concept 19. An automatically bidirectionally-sealable breakaway intra-medical tubing connector assembly according to any of inventive concepts 2 - 18 and wherein tensile force in the range of 0.5kgf - 3kgf is required in order to disconnect said at least one snap-fit fitting from said connector body.
Inventive concept 20. An automatically bidirectionally-sealable breakaway intra-medical tubing connector assembly according to any of inventive concepts 1 - 19 and wherein the magnitude of the required tensile force to disconnect the first luer-actuated valve assembly from said connector body is greater than the tensile force required to disconnect the second luer-actuated valve assembly from said connector body.
Inventive concept 21. An automatically bidirectionally-sealable breakaway intra-medical tubing connector assembly according to any of inventive concepts 3 - 20 and wherein the magnitude of the required tensile force to disconnect the first snap-fit fitting from said connector body is greater than the tensile force required to disconnect the second snap-fit fitting from said connector body.
Inventive concept 22. An automatically bidirectionally-sealable breakaway intra-medical tubing connector assembly according to any of inventive concepts 4 - 21 and wherein in said second operative orientation said first and second luer-actuated valve assemblies are swabbable.
Inventive concept 23. An automatically bidirectionally-sealable breakaway intra-medical tubing connector assembly according to any of inventive concepts 5 - 22 and wherein in said second operative orientation said first and second luer-actuated valve assemblies are swabbable once said first and second snap-fit fittings are detached therefrom.
Inventive concept 24. An automatically bidirectionally-sealable breakaway intra-medical tubing connector assembly according to any of inventive concepts 1 - 23 and wherein said medical tube remains static upon disconnection of at least one of said first and second luer actuated valve assemblies from said connector body.
Inventive concept 25. An automatically bidirectionally-sealable breakaway intra-medical tubing connector assembly according to any of inventive concepts 2 - 24 and wherein said medical tube remains static upon disconnection of at least one of said snap-fit fittings from said connector body.
Inventive concept 26. An automatically bidirectionally-sealable breakaway intra-medical tubing connector assembly according to any of inventive concepts 1 - 25 and wherein at least one of said first and second luer-actuated valve assemblies is integrally formed with or fixedly connected to said connector body.
Inventive concept 27. An automatically bidirectionally-sealable breakaway intra-medical tubing connector assembly according to any of inventive concepts 1 - 26 and wherein each of said first and second ends of the connector body includes a plurality of radially arranged spaced apart arms and a plurality of cut-outs formed therebetween, wherein at least one of said plurality of arms includes a snap portion extending radially inwardly and configured for engagement with at least one of said first and second luer-actuated valve assemblies.
Inventive concept 28. An automatically bidirectionally-sealable breakaway intra-medical tubing connector assembly according to inventive concept 27 and wherein said connector body is composed of two parts attachable to each other.
Inventive concept 29. An automatically bidirectionally-sealable breakaway intra-medical tubing connector body configured for use with a male luer-actuated valve assembly and a female luer-actuated valve assembly connected to opposite intermediate ends of a medical tube, comprising:
   said connector body arranged for tensile force responsive disconnectable snap fit connection with at least one of said male luer actuated valve assembly and said female luer-actuated valve assembly, which connection provides for opening of both said male luer actuated valve assembly and said female luer-actuated valve assembly, said connector body is configured such that disconnection of at least one of said male luer actuated valve assembly and said female luer-actuated valve assembly from said connector body permits automatic closing of both said male luer actuated valve assembly and said female luer-actuated valve assembly.
Inventive concept 30. An automatically bidirectionally-sealable breakaway intra-medical tubing connector body according to inventive concept 29 and wherein said connection provides a fluid flow path between said male luer actuated valve assembly and said female luer-actuated valve assembly.
Inventive concept 31. An automatically bidirectionally-sealable breakaway intra-medical tubing connector assembly according to inventive concept 29 or inventive concept 30 and wherein said male luer-actuated valve assembly and said female luer-actuated valve assembly assume a closed and sealed operative orientation upon disconnection thereof from said connector body. Inventive concept 32. An automatically bidirectionally-sealable breakaway intra-medical tubing connector assembly according to any of inventive concepts 29 - 31 and wherein said medical tube is selected from the group consisting of a catheter, an IV line and a drain.
Inventive concept 33. An automatically bidirectionally-sealable breakaway intra-medical tubing connector assembly according to any of inventive concepts 29 - 32 and wherein a first snap connection is formed on an inner surface of said connector body, adjacent said first end thereof and a second snap connection is formed on the inner surface of said connector body, adjacent said second end thereof, said first and second snap portions are configured for attachment with said male luer actuated valve assembly and said female luer-actuated valve assembly respectively.
Inventive concept 34. An automatically bidirectionally-sealable breakaway intra-medical tubing connector assembly according to any of inventive concepts 29 - 33 and wherein each of said male luer actuated valve assembly and said female luer-actuated valve assembly includes a compressible sealing element having a first open end and a second selectably openable end, including a selectively openable slit and wherein said sealing element is biased to its normally closed position when no compression force is exerted thereon.
Inventive concept 35. An automatically bidirectionally-sealable breakaway intra-medical tubing connector assembly according to inventive concept 34 and wherein upon engagement of said male luer actuated valve assembly and said female luer-actuated valve assembly with said connector body, said female luer-actuated valve assembly actuates said male luer-actuated valve assembly , both sealing elements of said male luer-actuated valve assembly and of said female luer-actuated valve assembly are compressed, thereby opening said respective selectively openable slits and establishing fluid flow passage between said male luer-actuated valve assembly and said female luer-actuated valve assembly.
Inventive concept 36. An automatically bidirectionally-sealable breakaway intra-medical tubing connector assembly according to any of inventive concepts 29 -35 and wherein tensile force in the range of 0.5kgf - 3kgf is required in order to disconnect one of said male luer actuated valve assembly and said female luer-actuated valve assembly from said connector body.
Inventive concept 37. An automatically bidirectionally-sealable breakaway intra-medical tubing connector assembly according to any of inventive concepts 29 - 36 and wherein the magnitude of the required tensile force to disconnect one of said male luer-actuated valve assembly and said female luer-actuated valve assembly from said connector body is greater than the tensile force required to disconnect the other of said male luer-actuated valve assembly and said female luer-actuated valve assembly from said connector body.
Inventive concept 38. An automatically bidirectionally-sealable breakaway intra-medical tubing connector assembly according to any of inventive concepts 29 - 37 and wherein upon disconnection of said male luer-actuated valve assembly and said female luer-actuated valve assembly from said connector body, both said said male luer-actuated valve assembly and said female luer-actuated valve assembly are swabbable.
Inventive concept 39. An automatically bidirectionally-sealable breakaway intra-medical tubing connector assembly according to any of inventive concepts 29 - 38 and wherein said medical tube remains static upon disconnection of said male luer-actuated valve assembly and said female luer-actuated valve assembly from said connector body.

## Claims

1. An automatically bidirectionally-sealable breakaway intra-medical tubing connector assembly comprising:
a male luer actuated valve assembly and a female luer-actuated valve assembly, which are configured for connection to opposite intermediate ends of a medical tube;
a connector body arranged for tensile force responsive disconnectable snap fit connection with at least one of said male luer actuated valve assembly and said female luer-actuated valve assembly, which connection provides for opening of both said male luer actuated valve assembly and said female luer-actuated valve assembly, said connector body is configured such that disconnection of at least one of said male luer actuated valve assembly and said female luer-actuated valve assembly from said connector body permits automatic closing of both said male luer actuated valve assembly and said female luer-actuated valve assembly.

2. The automatically bidirectionally-sealable breakaway intra-medical tubing connector assembly according to claim 1 and wherein said connection provides a fluid flow path between said male luer actuated valve assembly and said female luer-actuated valve assembly.

3. The automatically bidirectionally-sealable breakaway intra-medical tubing connector assembly according to claim 1 or claim 2 and wherein said male luer-actuated valve assembly and said female luer-actuated valve assembly assume a closed and sealed operative orientation upon disconnection thereof from said connector body.

4. The automatically bidirectionally-sealable breakaway intra-medical tubing connector assembly according to any of claims 1-3 and wherein said medical tube is selected from the group consisting of a catheter, an IV line and a drain.

5. The automatically bidirectionally-sealable breakaway intra-medical tubing connector assembly according to any of claims 1-4 and wherein a first snap connection is formed on an inner surface of said connector body, adjacent said first end thereof and a second snap connection is formed on the inner surface of said connector body, adjacent said second end thereof, said first and second snap portions are configured for attachment with said male luer actuated valve assembly and said female luer-actuated valve assembly respectively.

6. The automatically bidirectionally-sealable breakaway intra-medical tubing connector assembly according to any of claims 1-6 and wherein each of said male luer actuated valve assembly and said female luer-actuated valve assembly includes a compressible sealing element having a first open end and a second selectably openable end, including a selectively openable slit and wherein said sealing element is biased to its normally closed position when no compression force is exerted thereon.

7. The automatically bidirectionally-sealable breakaway intra-medical tubing connector assembly according to claim 6 and wherein upon engagement of said male luer actuated valve assembly and said female luer-actuated valve assembly with said connector body, said female luer-actuated valve assembly actuates said male luer-actuated valve assembly , both sealing elements of said male luer-actuated valve assembly and of said female luer-actuated valve assembly are compressed, thereby opening said respective selectively openable slits and establishing fluid flow passage between said male luer-actuated valve assembly and said female luer-actuated valve assembly.

8. The automatically bidirectionally-sealable breakaway intra-medical tubing connector assembly according to any of claims 1-7 and wherein tensile force in the range of 0.5kgf - 3kgf is required in order to disconnect one of said male luer actuated valve assembly and said female luer-actuated valve assembly from said connector body.

9. The automatically bidirectionally-sealable breakaway intra-medical tubing connector assembly according to any of claims 1-8 and wherein the magnitude of the required tensile force to disconnect one of said male luer-actuated valve assembly and said female luer-actuated valve assembly from said connector body is greater than the tensile force required to disconnect the other of said male luer-actuated valve assembly and said female luer-actuated valve assembly from said connector body.

10. The automatically bidirectionally-sealable breakaway intra-medical tubing connector assembly according to any of claims 1-9 and wherein upon disconnection of said male luer-actuated valve assembly and said female luer-actuated valve assembly from said connector body, both said said male luer-actuated valve assembly and said female luer-actuated valve assembly are swabbable.

11. The automatically bidirectionally-sealable breakaway intra-medical tubing connector assembly according to any of claims 1-10 and wherein said medical tube remains static upon disconnection of said male luer-actuated valve assembly and said female luer-actuated valve assembly from said connector body.
